# EUROPEAN PATENT SPECIFICATION

(11) **EP 1 929 303 B1**
(45) Date of publication and mention of the grant of the patent: **24.11.2010**
(21) Application number: 05748354.7
(22) Date of filing: 20.05.2005
(51) Int. Cl.: G01N 33/569, C07K 14/00, A61K 39/00

(54) **ASSAY AND KITS THEREFOR**
TEST UND KITS DAFÜR
DOSAGE ET NECESSAIRES CORRESPONDANTS

(30) Priority: 23.06.2004 GB 0414024
(43) Date of publication of application: 11.06.2008
(73) Proprietor: Institute for Animal Health, Newbury, Berkshire RG20 7NN (GB)
(72) Inventor: FERRIS, Nigel, Pirbright, Surrey GU24 0NF (GB); KING, Donald, Guildford, Surrey GU2 9UN (GB); JACKSON, Terry, Farnham, Surrey GU9 8TQ (GB); PATON, David, Leatherhead, Surrey KT24 5HF (GB)
(74) Representative: Smith, Stephen Edward
(86) International application number: PCT/GB2005/001952
(87) International publication number: WO 2006/000740

(56) References cited:
- JACKSON TERRY ET AL: "The epithelial integrin alphavbeta6 is a receptor for foot-and-mouth disease virus" JOURNAL OF VIROLOGY, vol. 74, no. 11, June 2000 (2000-06), pages 4949-4956, XP002354450 ISSN: 0022-538X
- JACKSON TERRY ET AL: "Integrin alphavbeta8 functions as a receptor for foot-and-mouth disease virus: Role of the beta-chain cytodomain in integrin-mediated infection" JOURNAL OF VIROLOGY, vol. 78, no. 9, May 2004 (2004-05), pages 4533-4540, XP002354449 ISSN: 0022-538X
- FERRIS N P ET AL: "Utility of recombinant integrin alphavbeta6 as a capture reagent in immunoassays for the diagnosis of foot-and-mouth disease" JOURNAL OF VIROLOGICAL METHODS, AMSTERDAM, NL, vol. 127, no. 1, July 2005 (2005-07), pages 69-79, XP004893939 ISSN: 0166-0934
- MACKAY DAVID K J ET AL: "A solid-phase competition ELISA for measuring antibody to foot-and-mouth disease virus" JOURNAL OF VIROLOGICAL METHODS, vol. 97, no. 1-2, September 2001 (2001-09), pages 33-48, XP002354451 ISSN: 0166-0934
- SHEPPARD D ET AL: "COMPLETE AMINO ACID SEQUENCE OF A NOVEL INTEGRIN BETA SUBUNIT (BETA6) IDENTIFIED IN EPTIHELIAL CELLS USING THE POLYMERASE CHAIN REACTION" JOURNAL OF BIOLOGICAL CHEMISTRY, AMERICAN SOCIETY OF BIOLOCHEMICAL BIOLOGISTS, BIRMINGHAM,, US, vol. 265, no. 20, 15 July 1990 (1990-07-15), pages 11502-11507, XP001089800 ISSN: 0021-9258

## Description

The present invention relates to the use of an αvβ6 integrin in the capture and/or detection of target microbial agents. In particular, the invention provides methods for the detection of target microbial agents, such as foot-and-mouth disease virus, and assay kits for use in such methods.

Foot-and-Mouth Disease (FMD) is a highly contagious viral infection of domestic and wild cloven-hoofed animals, such as pigs and wild and domesticated ruminants, *e.g*. cattle, buffalo, sheep, goats, camel and deer.

The FMD virus (FMDV) is a member of the *Aphthovirus* genus in the *Picornaviridae* family of positive-sense, single-stranded RNA viruses (Belsham, 1993, Prog Biophys Mol Biol 60(3): 241-260). There are seven FMDV serotypes, A, O, C, Asial, SAT1, SAT2 and SAT3, of which over 65 subtypes are known (Martinez et al., 1991, Virology, 184: 695-706). The virus has a high mutation rate (Haydon et al., 1998, Virus Genes, 16(3): 253-266), changing on a random basis between one and eight nucleotides per replication (Knowles et al., 3 March 2001, The Veterinary Record: 258-259).

In economic terms, FMD is the arguably the most important disease of domestic livestock. It can cause high mortality in young animals, as well as production losses in adults, and is considered the single most important constraint to trade in live animals and animal products. The disease is endemic in many countries in the Middle and Far East, Africa, Asia and South America, areas from which the virus periodically escapes to cause disease in other countries normally free of disease. Such spread of FMD can occur by a variety of mechanisms including animal movement, contaminated animal products (meat, milk, semen), mechanically by people and fomites, and by the wind (Knowles et al, 3 March 2001, The Veterinary Record: 258-259).

FMD-free countries have adopted strict measures to minimise the risk of an unwarranted ingress of the FMD virus onto its territory. However, an essential component in attaining and maintaining FMD-free status is the need to recognise the presence of the disease as soon as possible after its introduction, to impose animal movement restrictions to prevent its spread and thence to rapidly control disease (either through the disposal of infected stock or by vaccination) should preventative measures be breached. The severe consequence of failing to fulfil these criteria was graphically illustrated by the extensive outbreak which occurred in the UK in 2001, which ultimately caused 2030 separate confirmed cases over a seven-month duration and was estimated to have cost the UK government £3 billion.

Diagnosis of FMD is dependent upon early clinical precognition of the disease in the field, followed by confirmation of the presence of the FMD virus by objective tests most commonly performed in specialised laboratories. The laboratory diagnostic tests most commonly used for FMD virus antigen detection currently are the ELISA combined with virus isolation in cell culture (Ferris & M. Dawson, 1988, Vet. Microbiol. 16:201-209; Roeder & Le Blanc Smith, 1987, Res. Vet. Sci. 43:226-232) and fluorogenic reverse transcription polymerase chain reaction (RT-PCR; Reid et al., 2002, J Virol. Methods 105:67-80; Reid et al., 2003, J. Virol. Methods 107:129-139). The ELISA and RT-PCR test procedures can both be performed on suspensions of vesicular epithelia (the RT-PCR being more sensitive than the ELISA) but whereas the RT-PCR can function directly on blood or other fluids, such samples must first be inoculated onto sensitive cell cultures for isolation of virus before confirmation of virus specificity can be made by the ELISA. The type of ELISA which has been found to be the most sensitive format is an indirect sandwich assay employing type-specific antibodies of polyclonal and/or monoclonal origin.

However, despite developments in the field of FMD diagnosis, there remains a need for improved methods of detecting and diagnosing the disease.

Hence, the present invention seeks to provide means and methods for the detection of microbial agents such as FMDV.

### Summary of the Invention

According to a first aspect of the invention, there is provided a method for determining whether a sample contains a target microbial agent comprising:
(a) contacting the sample with an αvβ6 integrin under conditions that allow the target microbial agent, if present in the sample, to bind to the integrin; and
(b) determining whether the integrin has any target microbial agent bound thereto.

In a preferred embodiment, the method comprises a first step of contacting the sample with a capture ligand having affinity for the target microbial agent and a subsequent second step of determining whether the capture ligand has any target microbial agent bound thereto using a detection ligand having affinity for the target microbial agent, wherein either the capture ligand or the detection ligand is an αvβ6 integrin.

By 'capture ligand' we mean a moiety having affinity for the target microbial agent which may be used to isolate or capture the target microbial agent from the sample. Thus, the capture ligand may provide a means of concentrating the target microbial agent from the sample.

By 'detection ligand' we mean a moiety having affinity for the target microbial agent which provides a means of detecting the presence of the target microbial agent. The detection ligand may comprise a directly or indirectly detectable label. By "detectable label" is included any convenient radioactive label which can readily be incorporated into polypeptide using well known methods, as well as any convenient fluorescent or chemiluminescent label which can readily be incorporated into a polypeptide. In addition, the term "detectable label" also includes a moiety which can be detected by virtue of binding to another moiety (such as biotin which can be detected by binding to streptavidin) and a moiety, such as an enzyme, which can be detected by virtue of its ability to convert a colourless compound into a coloured compound, or *vice versa* (for example, alkaline phosphatase can convert colourless o-nitrophenylphosphate into coloured *o*-nitrophenol).

Preferably, the capture ligand comprises an antibody having specificity for the target microbial agent or antigen-binding fragment thereof.

Alternatively, the detection ligand may comprise an antibody having specificity for the target microbial agent or antigen-binding fragment thereof.

Antigen-binding antibody fragments include Fab-like molecules (Better et al., 1988, Science 240, 1041): Fv molecules (Skerra et a/., 1988, Science 240, 1038); disulphide-linked Fv molecules (Young et al., 1995. FEBS Lett. 377:135-139); single-chain Fv (ScFv) molecules where the V_{H} and V_{L} partner domains are linked via a flexible oligopeptide (Bird et al., 1988, Science 242, 423; Huston et al., 1988, Proc. Natl. Acad. Sci. USA 85, 5879) and single domain antibodies (dAbs) comprising isolated V domains (Ward et al. ,1959, Nature 341, 544). A general review of the techniques involved in the synthesis of antibody fragments which retain their specific binding sites is to be found in Winter & Milstein (1991) Nature 349, 293-299.

A second aspect of the invention provides a method for determining whether a sample contains antibodies to a target microbial agent comprising:
(a) contacting the sample with a specimen of the target microbial agent under conditions that allow anti-target microbial agent antibodies, if present in the sample, to bind to the target microbial agent;
(b) contacting the target microbial agent specimen with an immobilised capture ligand having affinity for the target microbial agent;
(c) contacting the immobilised target microbial agent specimen with a competitor ligand having affinity for the target microbial agent; and
(d) determining whether the immobilised target microbial agent specimen has any competitor ligand bound thereto
wherein step (b) may be performed either before or after step (a)
wherein either the capture ligand or the competitor ligand is an αvβ6 integrin
and wherein the amount of the competitor ligand bound to the target microbial agent specimen provides an indication of the presence of antibodies to a target microbial agent in the sample.

By a 'competitor ligand' we mean a moiety having affinity for the target microbial agent which competes with any target microbial agent antibodies present in the test sample for binding to the target microbial agent.

Preferably, the capture ligand is immobilised or capable of being immobilised.

Conveniently, the capture ligand or the competitor ligand is or comprises an antibody or antigen-binding fragment thereof.

It will be appreciated by persons skilled in the art that in the above methods, step (a) must be performed under conditions which allow the target microbial agents or antibodies thereto, as appropriate, to bind to the αvβ6 integrin. Suitable conditions may be determined using methods well known in the art. For example, when the method is performed using an ELISA (see below), step (a) is preferably carried out in the presence of cations, such as a buffer composed of 0.85% saline with 0.02 M Tris, 0.002M CaCl₂ and 0.001 M MgCl₂ and 2% bovine serum albumen.

In a preferred embodiment of the methods of the invention, the target microbial agent is a virus or bacterium.

Preferably, the target microbial agent comprises an RGD motif.

More preferably, the target microbial agent is a virus selected from the group consisting of picornaviruses, adenoviruses, noroviruses, coxsackie virus, echovirus 9 and human herpesvirus 8 (gB protein).

Most preferably, the target microbial agent is foot-and-mouth disease virus (FMDV).

It will also be appreciated by persons skilled in the art that the methods of the invention may be used to detect a target microbial agent or antibodies thereto in any sample which is suspected of, or is capable of, harbouring such agents and antibodies. Typically, samples for use in the methods of the invention are *ex vivo* samples taken from the body of an animal to be tested. Such samples maybe tested either on site or taken remotely and transferred to a testing facility for assay.

In a preferred embodiment, the sample is taken from a mammal, such as a pig or a wild or domesticated ruminant (*e*.*g*. cattle, buffalo, sheep, goat, camel and deer). The skilled person will appreciate that any suitable type of sample from such animals may be used. For example, suitable samples include vesicular epithelium, vesicular fluid, blood samples, probang samples (collection of fluid from the throat), cardiac muscle (such as whole heart from young pigs or lambs), semen, saliva and milk. In some circumstances, it may be beneficial to culture cells from such a sample, and to perform the diagnostic methods of the invention on the cultured cells. This latter approach can be particularly advantageous if optimal assay sensitivity is desired.

The above methods of the invention exploit the ability of an αvβ6 integrin to bind to a target microbial agent. Thus, in one embodiment, the methods of the invention make use of an αvβ6 integrin as a capture or detection ligand to 'trap' FMDV particles present in the sample to be tested. The αvβ6 integrin is able to bind FMDVs of all known serotypes and, hence, is able to provide a universal method for detecting FMDV infection.

Integrins are a family of cell-surface α-β3 heterodimeric glycoproteins composed of at least 15 α subunits and 8 β subunits which associate to form at least 24 different α-β combinations. Each subunit is composed of large extracellular domains, a transmembrane region and, in most cases, a short cytoplasmic domain. Integrins contribute to a variety of processes, including adhesion between cells and between cells and the extracellular matrix, and induction of signal transduction pathways that modulate various processes, including cell proliferation, morphology, migration and apoptosis.

The integrin β6 subunit forms only a single heterodimer, αvβ6, which has been shown to be a receptor for the extracellular matrix proteins fibronectin, tenascin and vibronectin, as well as for latency-associated protein-1 (LAP-1), a protein involved in modulation of the activity of transforming growth factor β1. Expression of αvβ6 is restricted to epithelial cells and has been observed at a number of sites, including the epithelia of the uterus, bladder, respiratory tract and salivary gland. Expression levels are moderate or low in normal healthy volunteers but are rapidly upregulated at sites of tissue injury and inflammation.

The integrin αvβ6, which is one of a number of integrins which bind their ligands by recognition of an RGD motif, has been implicated as one of several integrins which may act as receptors for FMDV (see Duque & Baxt, 2003, J. Virol. 77:2500-2511 and Jackson et al., 2000, J. Virol. 74:4949-4956). However, the potential utility of αvβ6 integrin as a capture ligand in the diagnosis of FMD has not been recognised previously.

The αvβ6 integrin for use in the methods of the invention may be produced using techniques well known in the art. Advantageously, the αvβ6 integrin comprises a naturally occurring αv subunit and/or a naturally occurring β6 subunit. In a preferred embodiment of the methods of the first and second aspects of the invention, the αvβ6 integrin is of mammalian origin. For example, the αvβ6 integrin may be selected from the group consisting of porcine, ovine, bovine, murine, guinea pig and human αvβ6 integrins. Most preferably, the integrin is porcine αvβ6 integrin.

Advantageously, the integrin is derived from the same species as the sample to be tested.

It will be appreciated that the integrin may be prepared by any known method. For example, the integrin may be extracted from mammalian tissue. However, more preferably, the αvβ6 integrin is obtained by recombinant means, *e.g*. from a culture of cells which are transformed to express one or both of the αv and β6 subunits. The sequences of nucleic acid molecules encoding αv and β6 subunits are known in the art and have been deposited in the GenBank database (see Table 1).

**TABLE 1**

| Integrin subunit | Database accession no. |
|---|---|
| bovine αv | AF239958 |
| human αv | NM002210 |
| porcine αv (partial) | AB048865 |
| ovine αv (partial) | AF349464 |
| bovine β6 | AF468060 |
| ovine β6 | AJ439062 |
| human β6 | M35198 |
| porcine β6 | SEQ ID NOS 1 & 2* |

| | |
|---|---|
| * These sequences have not been published previously (see Example 1) | |

Nucleic acid molecules encoding αv and β6 integrin subunits may be used in accordance with known techniques, appropriately modified in view of the teachings contained herein, to construct an expression vector, which is then used to transform an appropriate host cell for the expression and production of the polypeptide of the invention. Such methods of expressing proteins in recombinant cells lines are widely known in the art (for example, see Sambrook & Russell, 2000, Molecular Cloning, A Laboratory Manual, Third Edition, Cold Spring Harbor, New York).

In a particularly preferred embodiment, the αvβ6 integrin is produced by expression in cell culture as a secreted protein, such that the integrin may readily be harvested from the culture maintenance medium.

In addition to using naturally occurring αvβ6 integrins, it will be appreciated that variants of such integrins may be used. By "a variant" we include a polypeptide comprising the amino acid sequence of a naturally occurring αv and/or β6 subunit wherein there have been amino acid insertions, deletions or substitutions, either conservative or non-conservative, such that the changes do not substantially reduce the ability of the variant to bind to the target microbial agent compared to the binding ability of the naturally occurring integrin. For example, the variant may have increased affinity for the target microbial agent compared to that of the naturally occurring 'parent' integrin. Alternatively, the variant may have increased stability, *e.g*. tolerance to proteases, pH and/or thermal stability. Such variant integrins may be made using methods of protein engineering and site-directed mutagenesis commonly known in the art (for example, see Sambrook & Russell, 2000, *supra*).

In a preferred embodiment, the αvβ6 integrin is a soluble variant of a naturally occurring αvβ6 integrin. Such soluble αvβ6 integrins may be prepared as described in Weinacker et al., 1994, J. Biol. Chem. 269(9):6940-8.

Conveniently, the αvβ6 integrin may be immobilised. For example, the αvβ6 integrin may be immobilised on the surface of a container for the sample, such as on the surface of a well of a multiwell plate. Advantageously, the integrin is immobilised on the surface of an ELISA plate (such as Nunc Maxisorp immunoplates).

Alternatively, the αvβ6 integrin may be immobilised on microparticles of metal, synthetic polymers or nitrocellulose. Substrates of this type are suitable for use in chromatographic strip tests, such as the *Cleaoview*® technology introduced by Unipath Limited (see EP 0 291 194 B).

In a preferred embodiment of the methods of the first aspect of the invention, step (b) is performed by reverse transcription-polymerase chain reaction (RT-PCR), radioimmunoassay (RIA) or enzyme-linked immunosorbent assay (ELISA).

The use of reverse transcription-polymerase chain reaction methods for the detection and characterisation of microbial agents is known in the art. For example, exemplary RT-PCR based assays for FMDV are described in Reid et al., 1999, J Virol. Methods 83: 113-123 and Reid et al., 2000, J. Virol. Methods 89: 167-176. Alternative methods include proximity ligation assays (PLA) and DNA circularisation amplification systems.

Radioimmunoassays (RIA) and enzyme-linked immunosorbent assays (ELISA) are direct binding assays for antibody (or antigen) and both work on the same principle, but the means of detecting specific binding is different. In RIA for an antigen, pure antibody against that target molecule (*e.g*. the FMDV or anti-FMDV antibody) is radioactively labelled, usually with ¹²⁵I, whereas in ELISA, an enzyme is linked chemically to the antibody.

The labelled antibody is allowed to bind to the unlabelled antigen, under conditions where non-specific adsorption is blocked, and any unbound antibody and other proteins are washed away. Antibody binding in RIA is measured directly in terms of the amount of radioactivity retained by the coated wells, whereas in ELISA, binding is detected by a reaction that converts a colourless substrate into a coloured reaction product. The colour change can be read directly in the reaction tray, making data collection very easy, and ELISA also avoids the hazards of radioactivity. In the present invention, a modification of ELISA known as a capture or sandwich ELISA (or more generally as an antigen-capture assay) is employed. Rather than the antigen being directly attached to a plastic plate, the integrin is used to capture the target microbial agent and thus concentrate it on the surface of the plate.

Thus, in a preferred embodiment of the first aspect of the invention, step (b) comprises the following sub-steps:
(i) removing from the integrin any excess (*i.e*. unbound) sample;
(ii) adding to the integrin (together with any target microbial agents from the sample bound thereto) a primary antibody having specificity for the target microbial agent; and
(iii) adding an immunoconjugate comprising the antigen-binding domain of an antibody with specificity for the primary antibody fused (or otherwise conjugated) to a directly or indirectly detectable label.

It will be appreciated that the primary antibody may be either a polyclonal or monoclonal antibody. Advantageously, however, the primary antibody is a monoclonal antibody. Methods of making such antibodies with affinity for a target microbial agent are well known in the art. For example, suitable monoclonal antibodies to selected antigens may be prepared by known techniques, such as those disclosed in "Monoclonal Antibodies: A manual of techniques", H Zola (CRC Press, 1988) and in *"*Monoclonal Hybridoma Antibodies: Techniques and Applications", J G R Hurrell (CRC Press, 1982).

For example, monoclonal antibodies to FMDV may be produced against inactivated 146S FMDV antigens (purified whole virus) as follows.

Inactivation and purification of FMDV can be performed by methods similar to those described (Ferris et al., 1984, Rev. Sci. Tech. Off Int. Epi. 3:339-3s0). Selected strains of the seven immunological types of FMDV are grown in monolayer cell cultures of (typically) baby hamster kidney (BHK-21) cells, the supernatant medium is harvested when cultures show maximal cytopathic effect and then clarified by centrifugation. Inactivation of the FMDV is carried out by preparing 0.1 M bromoethyleneimine (BEI), which is added to the virus harvest to a final concentration of 0.001 M and stirred at 37°C for 24 hours. Virus inactivation can be verified by the method of Ferris & Donaldson, 1984, Report of the Session of the Research Group of the Standing Technical Committee of the European Commission for the Control of Foot-and-Mouth Disease, Brescia, Italy 1984, Appendix 1, 7-9. Concentration of virus is carried out through the precipitation of protein following the addition of 7.5% (w/v) polyethylene glycol 6000 (stirring at temperatures between +1 and 8°C for 6-16 hours), centrifugation to pellet the precipitate and resuspension in, for example, a Tris/NaCl buffer (0.05 M Tris, 1 M NaCl, pH 7.6). Following resuspension, insoluble material is removed by low speed centrifugation. The supernatant fluid is then centrifuged at high speed to pellet the virus protein, which is subsequently resuspended in 1-2 ml of Tris/NaCl buffer. A detergent (*e.g*. Igepal, Sigma) is added to a final concentration of 1% and samples layered onto 15-45% linear sucrose gradients and centrifuged at high speed for 3-4 hours. Samples of the gradient are then run through a flow cell of a UV spectrophotometer and 146S identified and quantified by measurement at a wavelength of 259 nm.

Polyclonal antisera may be raised in rabbits and guinea pigs according to methods described (Ferris & Donaldson, 1984, Rev. Sci. Tech. Off. Int. Epiz. 3:563-574; Have et al., 1984, Acta Vet. Scand. 25:280-296). Rabbits can be inoculated sub-cutaneously and intra-muscularly with an emulsion of 15 µg of inactivated antigen of FMDV plus adjuvant at each of several sites (1 ml per rabbit) and boosted 28 days later as before. The animals are killed and exsanguinated 10-14 days later and serum separated. Guinea pigs are inoculated sub-cutaneously either with an emulsion of 10 µg of inactivated 146S antigen of FMDV in Tris buffer mixed with an adjuvant (1 ml per guinea pig), animals killed and exsanguinated after 28 days and serum separated or with an emulsion of 5 µg of inactivated 146S antigen of FMDV in Tris buffer mixed with adjuvant, animals boosted at 28 days as before, animals killed and exsanguinated 10-14 days later and serum separated.

For the production of monoclonal antibodies, Balb/C mice are immunised with 2-3 doses of 20-100 µg of inactivated and purified FMDV 146S antigen at intervals of one month (first inoculation subcutaneously with antigen in Freund's adjuvant and boosts intraperitoneally with antigen in phosphate buffered saline). Three days after the last boost, 10⁸ spleen cells are collected and fused with 10⁷ NSO myeloma cells using polyethylene glycol 4000. Fused cells, suspended in Dulbecco's modified Eagle's medium, supplemented with hypoxanthine/aminopterin/thymidine and 20% foetal calf serum are distributed over wells of microplates. Hybridoma supernatants are screened for antibody secretion by ELISA and positive hybridomas cloned by limiting dilution on feeder layers of normal mouse spleen cells, in 96-well microplates (Brocchi et al., 1993, Rev. Sci. Tech. Off. Int. Epizoot. 12: 559-570). Monoclonal antibodies may be used either as cell culture supernant fluids or as ascites fluids following induction of the hybridoma in mice.

Immunoconjugates suitable for use in RIA and ELISA methods are well known in the art. Such immunoconjugates typically comprise a peptide portion having horseradish peroxidase activity, however it will be appreciated that any directly or indirectly detectable label may be utilised. Immunoconjugates suitable for use in step (iii) are commercially available. For example, when guinea pig polyclonal antibodies against a target microbial agent are used in step (ii), a rabbit immunoglobulin to guinea pig immunoglobulin conjugated to horseradish peroxidase may be used in step (iii) (such as cat no P0141 from Dako Cytomation, Ely, UK). An alternative strategy is to use monoclonal antibodies against the target microbial agent in step (ii), instead of guinea pig polyclonal antibodies, and thence to use a rabbit anti-mouse/HRP conjugate in step (iii) (such as cat no P0260, also from Dako).

ELISA methodology may also be used in step (d) of the methods of the second aspect of the invention.

Prior art methods for FMDV serology by ELISA include liquid phase blocking ELISA (Hamblin et al., 1986, J. Immumol. Methods 93: 123-129.) and solid phase competition ELISA (Mackay et al., 2001, J Viral. Methods 97: 33-48). Such methods typically use rabbit antiserum as a capture ligand for FMDV particles and known anti-FMDV antibodies (either polyclonal or monoclonal) as a competitor ligand. The present invention allows these prior art methods to be adapted by using an αvβ6 integrin as a capture ligand or competitor ligand, *i.e*. in place of the rabbit antiserum or known anti-FMDV antibodies.

In a preferred embodiment of the first aspect of the invention, the method further comprises step (c), after step (b), of characterising the virus strain detected in step (b). Methods for characterising FMDV strain are well known in the art, for example RT-PCR (see Reid et al, 1999, Journal of Virological Methods, 83: 113-123) or ELISA. The ELISA is the most commonly and simplest method for serotypic characterisation of virus strains (Ferris & Dawson, 1988, Vet. Microbiol. 16:201-209). Antigenic characterisation (matching of field FMDVs to vaccine strains as a means of selection of appropriate vaccines for control) may also be done by ELISA (Kitching et al., 1988, Vaccine 6:403-408)

A third aspect of the invention provides a kit of parts for performing a method according to the first or second aspect of the invention comprising an αvβ6 integrin or cells capable of expressing the integrin when grown in culture or vectors comprising a nucleotide sequence which encodes the integrin. Such cells and vectors may be produced using standard techniques (for example, see Sambrook & Russell, 2000, *supra*).

In a preferred embodiment, the kit comprises a substrate upon which the αvβ6 integrin is or can be immobilised. Conveniently, the substrate is capable of containing a sample to be tested, such as a multiwell plate. Advantageously, the substrate is suitable for use in a chromatographic strip test, for example microparticles or nitrocellulose (see EP 0 291 194 B and Reid et al., 2001, J. Virol. Methods. 96:189-202).

Preferably, the kit further comprises means for detecting a target microbial agent, such as an anti-target microbial agent antibody (which may be polyclonal or monoclonal). More preferably, the kit further comprises an immunoconjugate comprising the antigen-binding domain of an antibody with specificity for the anti-target microbial agent antibody fused to a directly or indirectly detectable label.

In an alternative embodiment, the kit further comprises means for detecting anti-target microbial agent antibodies, such as an immunoconjugate comprising the antigen-binding domain of an antibody with specificity for (*i.e*. capable of binding to) anti-FMDV antibodies fused to a directly or indirectly detectable label.

Conveniently, the directly or indirectly detectable label is a polypeptide having an enzyme activity, such as horseradish peroxidase activity, alkaline phosphatase, *etc*., and the kit of the invention further comprises a suitable enzyme substrate capable of giving a detectable, *e*.*g*. coloured, product upon action by the peroxidase.

It will be appreciated that the kit may further comprise one or more reagents for performing an ELISA, such as blocking buffers, dilution buffers and wash solutions.

The kit may additionally comprise instructions for performing a method according to the invention

Preferably, the kit is provided in sterilised form.

Further aspects of the invention include the use of an αvβ6 integrin to detect a target microbial agent in a sample and/or to purify a target microbial agent from a sample (wherein the target microbial agent may be any of those described above). Preferably, the αvβ6 integrin is used as a capture ligand and/or detection ligand for FMDV.

Conveniently, the αvβ6 integrin is used in an ELISA. For example, then integrin may be used as the detector in an indirect sandwich ELISA for antigen detection or as a competitor with test serum for captured antigen in a competition ELISA for antibody detection, etc., as described earlier (page 4).

In a preferred embodiment, the αvβ6 integrin comprises a naturally occurring αv subunit and/or a naturally occurring β6 subunit. Preferably, the integrin is mammalian, for example a porcine, ovine, bovine, murine, guinea pig or human αvβ6 integrin. More preferably, αvβ6 integrin may be of mammalian origin for example the αvβ6 integrin may be selected from the group consisting of porcine, ovine, bovine, murine, guinea pig and human αvβ6 integrins. Most preferably, the αvβ6 integrin is porcine αvβ6 integrin.

Conveniently, the αvβ6 integrin is derived from the same species as the sample to be tested.

Advantageously, the αvβ6 integrin is a recombinant integrin.

It will be appreciated by persons skilled in the art that the integrin may also be immobilised.

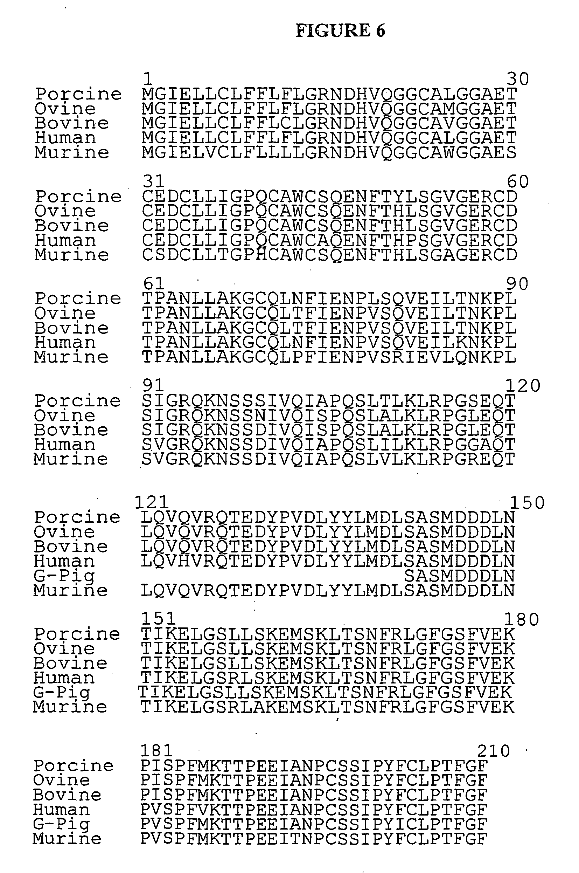

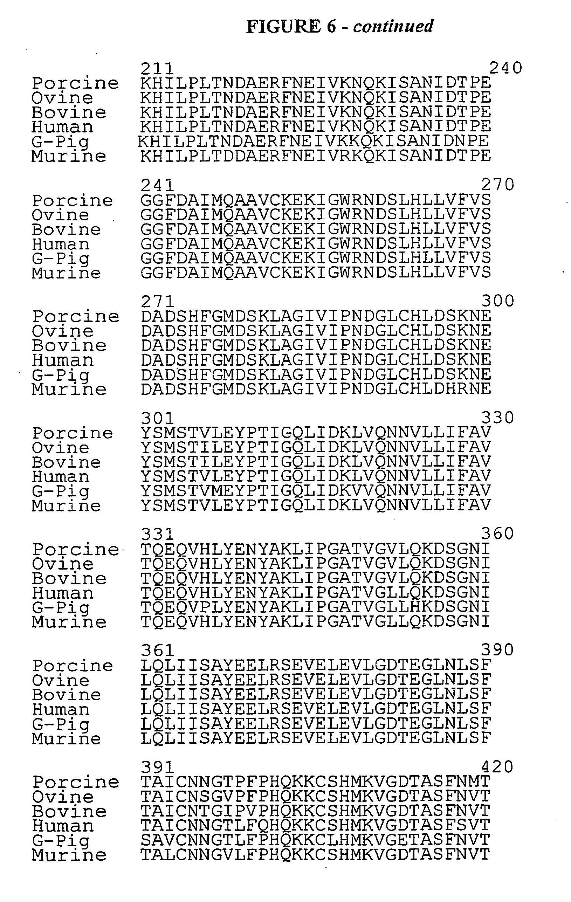

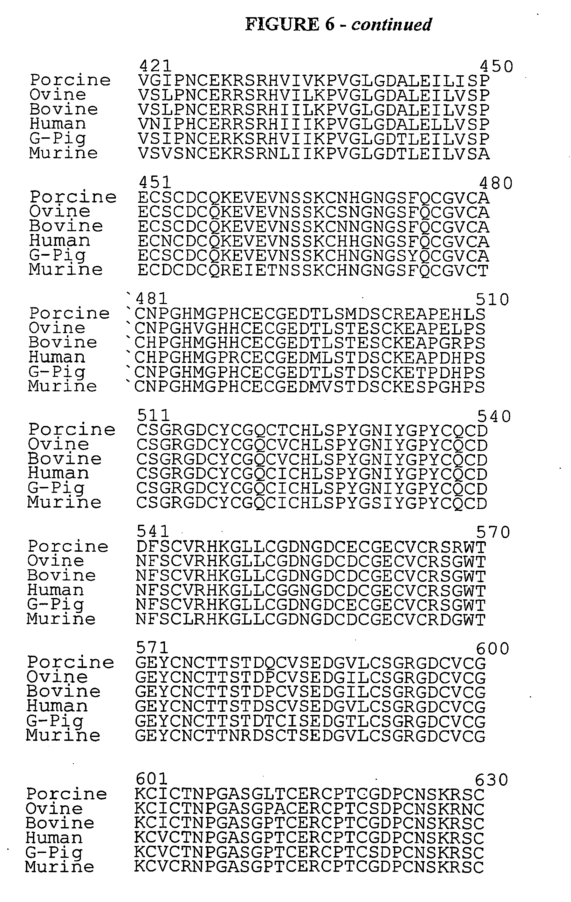

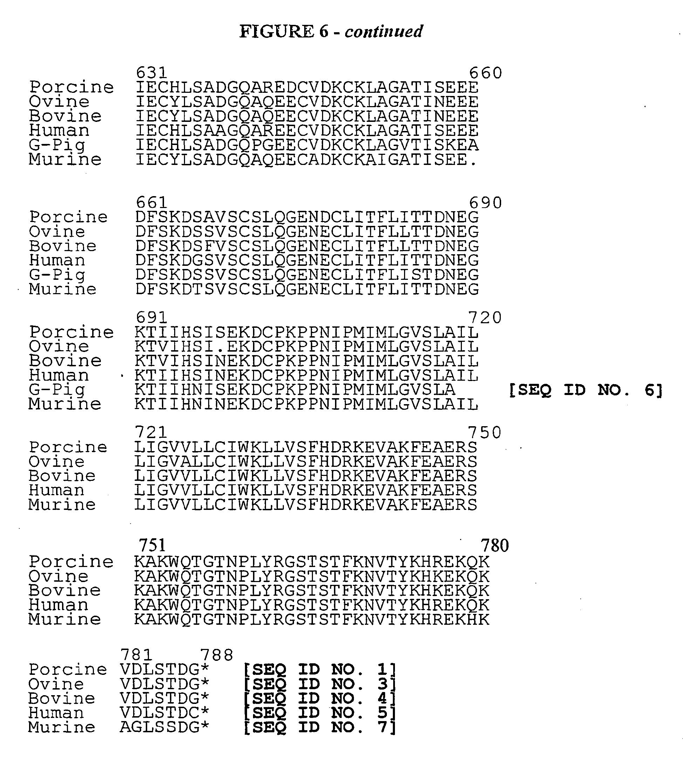

A further aspect of the invention provides an isolated polypeptide comprising or consisting of the amino acid sequence of SEQ 1D NO. I (porcine β6 subunit; see Figure 6) :

MGIELLCLFFLFLGRNDHVQGGCALGGAETCEDCLLIGPQCAWCSQENFT
YLSGVGERCDTPANLLAKGCQLNFIENPLSQVEILTNKPLSIGRQKNSSSIV
QIAPQSLTLKLRPGSEQTLQVQVRQTEDYPVDLYYLMDLSASMDDDLNTI
KELGSLLSKEMSKLTSNFRLGFGSFVEKPISPFMKTTPEEIANPCSSIPYFCL
PTFGFKHILPLTNDAERFNEIVKNQKISANIDTPEGGFDAIMQAAVCKEKIG
WRNDSLHLLVFVSDADSHFGMDSKLAGIVIPNDGLCHLDSKNEYSMSTVL
EYPTIGQLIDKLVQNNVLLIFAVTQEQVHLYENYAKLIPGATVGVLQKDSG
NILQLIISAYEELRSEVELEVLGDTEGLNLSFTAICNNGTPFPHQKKCSHMK

VGDTASFNMTVGIPNCEKRSRHVIVKPVGLGDALEILISPECSCDCQKEVE
VNSSKCNHGNGSFQCGVCACNPGHMGPHCECGEDTLSMDSCREAPEHLS
CSGRGDCYCGQCTCHLSPYGNIYGPYCQCDDFSCVRHKGLLCGDNGDCE
CGECVCRSGWTGEYCNCTTSTDQCVSEDGVLCSGRGDCVCGKCICTNPG
ASGLTCERCPTCGDPCNSKRSCIECHLSADGQAREDCVDKCKLAGATISEE
EDFSKDSAVSCSLQGENDCLITFLITTDNEGKTIIHSISEKDCPKPPNIPMIML
GVSLAILLIGVVLLCIWKLLVSFHDRKEVAKFEAERSKAKWQTGTNPLYR
GSTSTFKNVTYKHREKQKVDLSTDG
[SEQ ID NO. 1]

By 'variant', we include a polypeptide wherein at one or more positions there have been amino acid insertions, deletions, or substitutions, either conservative or non-conservative, provided that such changes result in a protein capable of binding a target microbial agent, for example FMDV, when complexed to an αv integrin subunit. Such variants may be made using the methods of protein engineering and site-directed mutagenesis.

Methods of detecting binding of the polypeptide of the invention or fragment, variant, derivative or fusion thereof, or fusion of a variant, fragment or derivative to an αv integrin subunit include a surface plasmon resonance assay, for example as described in Plant et al (1995) Analyt Biochem 226(2), 342-348. Methods may make use of a polypeptide of the invention or fragment, variant, derivative or fusion thereof, or fusion of a variant, fragment or derivative that is labelled, for example with a radioactive or fluorescent label.

A yet further aspect of the invention provides an isolated complex comprising a polypeptide according to the above aspect of the invention and an αv integrin subunit. Preferably, the complex is immobilised, *e.g*. on the surface of a multi-well plate.

Another aspect of the invention provides an isolated polynucleotide encoding a polypeptide according to the invention. Preferably, the polynucleotide comprises or consists of the nucleotide sequence of SEQ ID No. 2:

ATGGGGATTGAACTGCTTTGCCTGTTCTTTCTATTTCTAGGAAGGAATG
ATCACGTACAAGGTGGCTGTGCCCTGGGAGGTGCAGAGACCTGTGAAG
ACTGCTTACTCATTGGACCGCAGTGTGCCTGGTGTTCTCAGGAGAATTT
TACTTATCTCTCTGGAGTTGGAGAAAGGTGTGACACCCCAGCAAATCT
TTTAGCTAAAGGATGTCAACTAAACTTCATCGAAAACCCTCTCTCCCAA
GTAGAAATACTTACAAATAAGCCTCTCAGCATAGGCAGACAGAAAAAT
AGTTCCAGCATCGTTCAGATTGCACCTCAAAGCTTGACTCTTAAATTGA
GACCAGGCAGTGAACAGACTCTGCAAGTTCAAGTTCGCCAGACCGAG
GACTACCCAGTGGACTTGTATTACCTCATGGACCTCTCGGCCTCCATGG
ATGACGACCTCAACACGATCAAAGAGCTGGGCTCCCTGCTTTCCAAGG
AGATGTCTAAATTAACGAGCAACTTTAGACTGGGCTTTGGCTCTTTTGT
GGAAAAACCCATCTCCCCATTTATGAAAACAACGCCAGAAGAAATCGC
CAACCCTTGCAGTAGTATTCCATACTTCTGCTTGCCTACCTTTGGATTC
AAGCACATTTTGCCGTTGACAAATGATGCTGAAAGATTCAATGAAATT
GTGAAGAATCAGAAAATTTCTGCTAATATTGATACTCCGGAAGGTGGA
TTTGATGCAATCATGCAAGCCGCTGTGTGTAAGGAAAAAATTGGCTGG
CGGAATGACTCCCTCCATCTCCTGGTCTTTGTGAGTGATGCCGATTCAC
ATTTTGGAATGGACAGCAAACTGGCGGGCATTGTCATTCCTAACGATG
GGCTCTGTCACTTGGACAGCAAGAATGAATACTCCATGTCAACAGTCT
TGGAATATCCAACCATTGGACAACTCATTGATAAACTGGTACAAAATA
ACGTGTTACTGATCTTTGCTGTAACTCAAGAACAAGTTCATCTGTATGA
GAATTATGCAAAACTCATTCCTGGAGCTACAGTAGGGGTACTTCAGAA
GGACTCTGGGAACATTCTCCAGCTGATCATCTCAGCTTATGAAGAACT
GCGGTCTGAGGTGGAGCTGGAAGTGTTAGGAGACACGGAAGGACTCA
ACCTGTCATTTACAGCCATCTGTAACAATGGGACTCCCTTCCCACACCA
AAAGAAATGCTCTCACATGAAGGTGGGAGACACAGCTTCGTTCAACAT
GACTGTGGGGATACCAAACTGTGAGAAAAGAAGCAGGCATGTTATTGT

AAAGCCCGTGGGGCTGGGGGACGCCCTAGAAATCCTGATCAGTCCAG
AATGCAGCTGCGACTGTCAGAAAGAAGTGGAGGTGAACAGCTCCAAA
TGCAACCACGGCAACGGCTCCTTCCAGTGTGGGGTGTGTGCCTGCAAC
CCTGGCCACATGGGTCCTCACTGCGAGTGCGGGGAGGACACGCTGAGC
ATGGACTCCTGCAGGGAGGCCCCGAACACCTCTCATGCAGCGGGAGA
GGCGATTGCTACTGTGGGCAGTGCACCTGCCACTTGTCTCCTTATGGAA
ACATTTATGGGCCTTACTGCCAATGCGATGATTTCTCCTGCGTGAGACA
CAAGGGGCTGCTCTGCGGAGATAATGGTGACTGTGAGTGCGGCGAGTG
CGTGTGCAGGAGCGGCTGGACAGGCGAGTACTGTAACTGCACCACCA
GCACGGACCAGTGCGTCTCAGAGGATGGGGTGCTCTGCAGCGGTCGAG
GGGACTGTGTGTGCGGCAAGTGCATTTGCACGAACCCTGGAGCCTCAG
GACTCACCTGTGAACGCTGTCCCACCTGTGGCGATCCCTGTAACTCTAA
ACGGAGCTGCATTGAATGCCACTTGTCTGCTGATGGCCAGGCCCGAGA
AGACTGTGTTGACAAATGCAAACTAGCTGGTGCGACCATCAGTGAAGA
AGAAGATTTCTCAAAGGATAGTGCTGTTTCCTGTTCTCTGCAAGGAGA
AAATGACTGTCTTATTACATTCCTAATAACTACAGATAACGAAGGAA
AACCATCATTCACAGCATCAGCGAGAAAGACTGCCCAAAACCTCCCAA
TATTCCCATGATCATGTTGGGGGTTTCACTGGCTATTCTTCTCATTGGG
GTTGTCCTACTGTGTATTTGGAAGCTGCTGGTGTCATTTCACGATCGGA
AAGAAGTTGCCAAATTTGAAGCAGAACGGTCAAAGGCCAAGTGGCAA
ACGGGAACCAATCCACTGTACAGAGGCTCTACCAGCACCTTTAAAAAT
GTAACCTACAAACACAGGGAAAAACAAAAGGTGGACCTTTCCACGGA
TGGATAG

[SEQ ID NO:2]

By 'isolated' we mean that the polypeptide, complex or polynucleotide is not located in a cell, *i.e. in situ,* but is suitable for *in vitro* use in the methods of the invention.

The present invention also provides vectors (such as expression vectors), host cells (such as a mammalian host cells) for expressing the polypeptides of the invention and methods for making such polypeptides comprising culturing a host cell or vector according to the invention.

The skilled person will appreciate that there are many applications for the methods of the invention. Also described herein potential uses of the methods of the invention.
(a) A method of obtaining/purifying a target microbial agent from a sample comprising contacting the sample with an αvβ6 integrin under conditions that allow any target microbial agent present in the sample to bind to the integrin. It will be appreciated that such a methods may be used to detect in a sample new merging pathogens that comprise an RGD motif and are able to bind to αvβ6 integrin.
(b) A method for determining whether an organism is infected with a target microbial agent comprising determining whether a sample from the organism contains a target microbial agent by a method according to the first our second aspect of the invention. Thus, the invention provides an *in vitro* method for diagnosing infection with a microbial agent by testing a sample for the presence of the agent or an antibody thereto.
(c) A method of vaccinating an organism against a target microbial agent comprising determining whether a sample from an organism contains a target microbial agent by a method according to the first or second aspect of the invention, and, if a target microbial agent is not detected in the sample, administering a vaccine against the target microbial agent to the organism. For example, vaccines for FMDV are known in the art and include inactivated whole viruses, and are available commercially from companies such as Merial, Bayer and Intervet, as well as vaccines described in US 6,107.021 1 and US 4,732,971. Generally, primary administration of an FMDV vaccine is followed with a booster, for example, after 1 month, which may be followed by further regular boosters, for example, every 6 months.
(d) A method of treating an organism infected with a target microbial agent comprising determining whether a sample from an organism contains a target microbial agent by a method according to the first or second aspect of the invention, and, if a target microbial agent is detected in the sample, administering a therapeutic agent to the organism, which agent is effective in combating the target microbial agent. By "treating" is included the meaning that one or more symptoms of infection with the target microbial agent are ameliorated. Preferably treatment includes providing relief from pain. For example, when the target microbial agent is FMDV, treatment may include substantially reversing the effects of FMD on production losses. Any therapeutic agent, such as interferon-γ, may be used: The form, amount and administrative regime used for treatment of FMDV with a therapeutic agent will be apparent to the skilled person and will be typically determined based on the identity, age, weight and/or condition of the recipient. A therapeutic agent or a formulation thereof may be administered by any conventional method including oral and parenteral (*e.g*. subcutaneous or intramuscular) injection. The treatment may consist of a single dose or a plurality of doses over a period of time. Whilst it is possible for a therapeutic agent to be administered alone, it is preferable to present it as a pharmaceutical formulation, together with one or more acceptable carriers. The carrier(s) must be "acceptable" in the sense of being compatible with the therapeutic agent and not deleterious to the recipients thereof. Typically, the carriers will be water or saline which will be sterile and pyrogen free.
(e) A method for combating the spread of a target microbial agent between organisms comprising determining whether a sample from an organism contains a target microbial agent by a method according to the first or second aspect of the invention, and, if the organism is infected with a target microbial agent, sacrificing the organism. By "combating the spread of a target microbial agent between organisms" we mean that the rate of spread of the target microbial agent is reduced in comparison to an analogous situation where no action is taken. After the organism is sacrificed, typically the corpse will be disposed of, for example by cremation or burial, to prevent or reduce the spread of the target microbial agent from the corpse.
(f) A method for determining whether a test vaccine is capable of preventing infection with a target microbial agent comprising administering the test vaccine to an organism, inoculating the organism with the target microbial agent and determining whether a sample from the organism contains the target microbial agent by a method according to the first or second aspect of the invention. Any molecule or combination thereof can be used as a test vaccine. Typically, the test vaccine may comprise a polypeptide or polynucleotide derived from the target microbial agent, or a synthetically modified version thereof. The vaccine can be administered to the recipient in any suitable form. Typically, the recipient is maintained for a period of time, such as one week, one months or six months, before inoculation, in order to allow the test vaccine to mediate an immune response in the recipient, and thus immunise the recipient against infection. Further administrations of the test vaccine may be made during the maintenance period. Inoculation with the target microbial agent by techniques known in the art typically follows (Burrows, J Hyg (Lond). 1966, 64:419-429; Burrows, J Hyg (Lond), 1968, 66:633-640; Henderson, J Hyg (Lond), 1952, 50:182-194; Sellers et al, Vet Rec, 1969, 85:198-199). After a brief period of time to allow the establishment of disease, such as FMD, samples may then be taken from the recipient organism, typically at regular intervals, such as daily during the first and typically also the second week post inoculation and, thereafter, weekly. The presence (or absence) of, and levels of, the target microbial agent in the samples can then be determined using a method according to the first aspect of the invention in order to trace the progress of the disease, thereby to determine whether the test vaccine has been able to render the recipient organism immune to infection with the target microbial agent.
(g) A method for determining whether a test agent is capable of combating a target microbial agent comprising administering the test agent to an organism infected with a target microbial agent and determining whether the test agent is capable of combating the target microbial agent by a method according to the first or second aspect of the invention. Inoculation with the target microbial agent can be achieved by techniques known in the art (Burrows, J Hyg (Lond), 1966, 64:419-429; Burrows, J Hyg (Lond), 1968, 66:633-640; Henderson, J Hyg (Lond), 1952, 50:182-194; Sellers et al, Vet Rec, 1969, 85:198-199). Typically the recipient is maintained for a period of time, such as up to one day, one week, one month or six months before administration of the test agent begins. Any molecule or combination thereof can be used as a test agent. The test agent can be administered to the recipient in any suitable form, any suitable dosage and using any suitable regimen. This can be determined by the skilled person on the basis of identity, age, weight and condition of the recipient. However, with experimental test agents, initial empirical testing may additionally be required to determine toxicity levels and the like prior to attempts to treat infection with a target microbial agent. A sample, typically a plurality of samples, is taken from the recipient organism. At least one sample may be taken after inoculation of the recipient but before onset of administration of the test agent to provide a 'pre-treatment' reading. The presence of, and levels of, the target microbial agent in the samples can then be determined using a method according to the fourth aspect of the invention in order to trace the progress of the disease, thereby to determine whether the test agent has been able to combat the proliferation of the target microbial agent in the recipient organism.

Preferably, the target microbial agent is FMDV.

In a preferred embodiment, the methods of the invention are automated. By 'automated' we mean that one or more of the steps of the method are performed by a machine.

The invention will now be described in more detail by reference to the following Figures and Examples wherein:

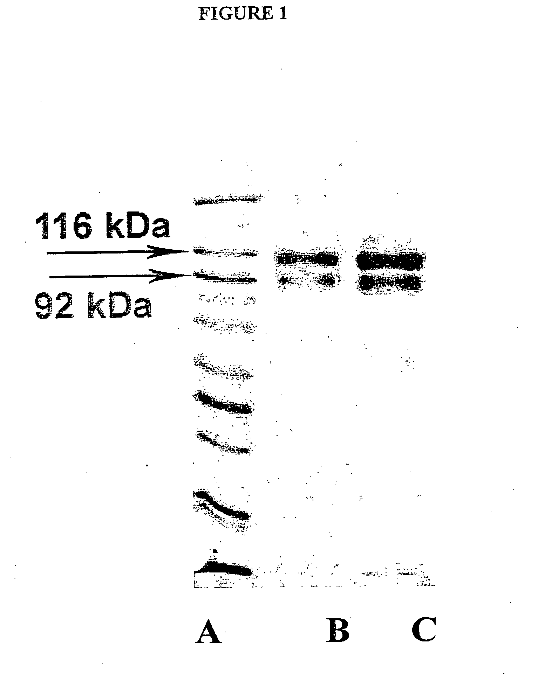

FIG.1. Polyacrylamide gel electrophoresis analysis on two different preparations (lanes B and C) of recombinant soluble αvβ6 purified according to the protocol in the Examples below. Lane A, protein molecular weight markers (SigmaMarkers Wide Range). The bands closest in weight to αv and β6 chains are indicated.

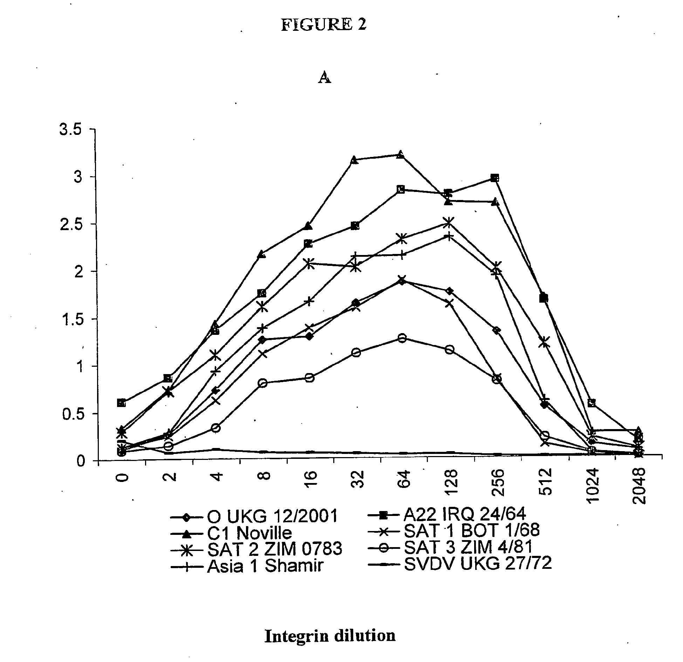

FIG.2 A. Titration of CHO cell culture supernatant fluid containing soluble recombinant integrin against purified inactivated antigens of each of the seven serotypes of FMD virus plus swine vesicular disease (SVD) virus (at 1 µg/ml). B. Reaction of purified inactivated antigens of each of the seven serotypes of FMD virus plus SVD virus (at 1 µg/ml) added directly onto plates previously blocked with blocking buffer 2.

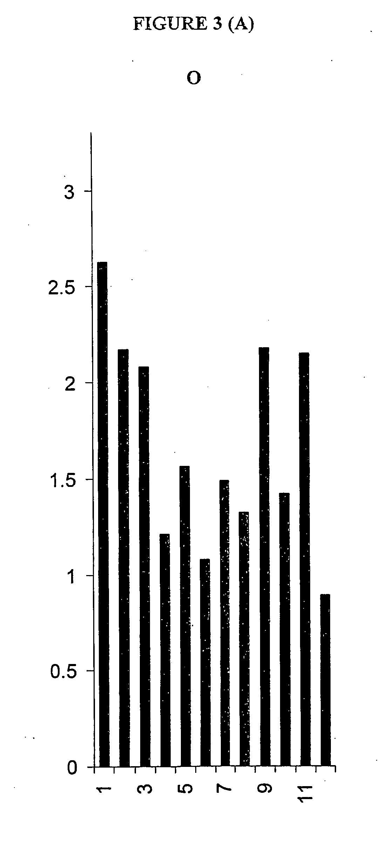

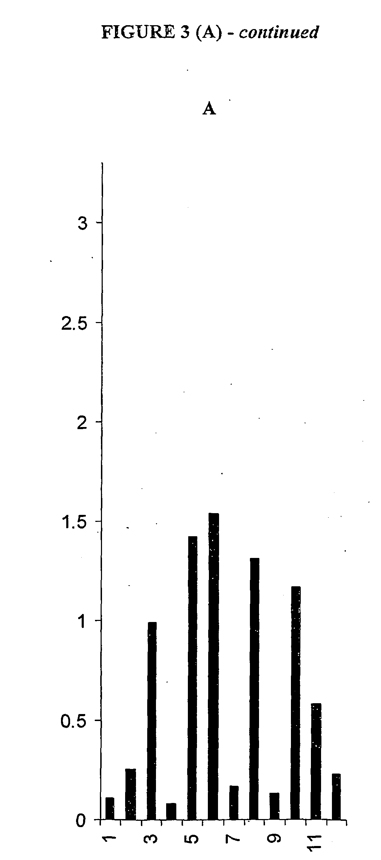

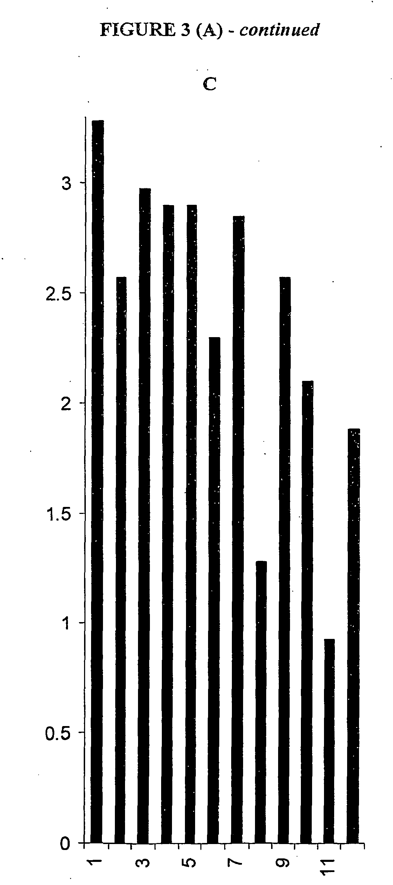

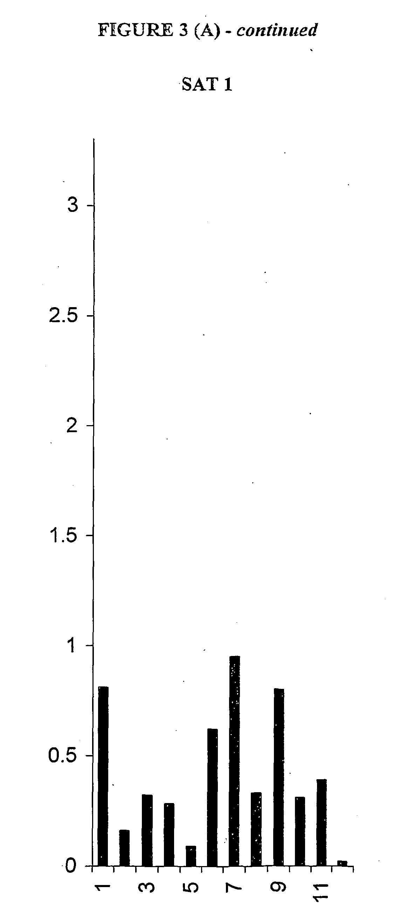

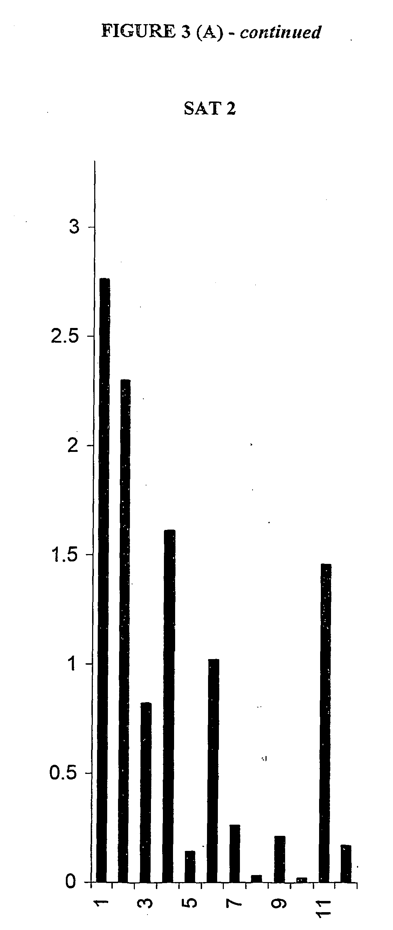

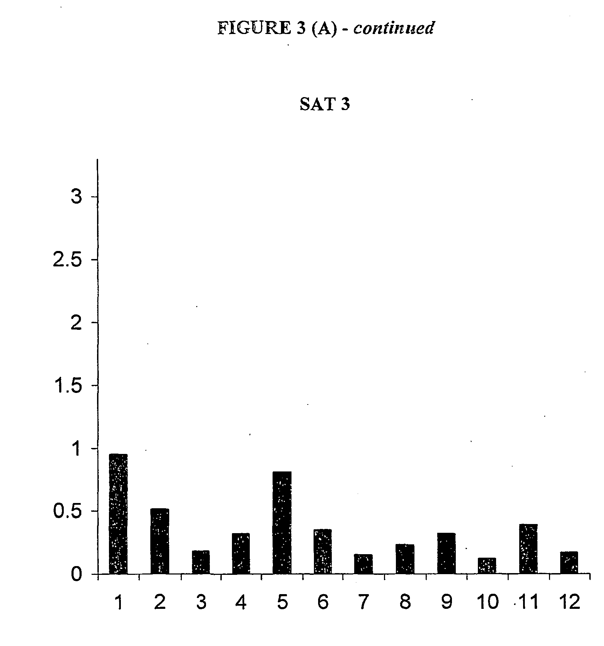

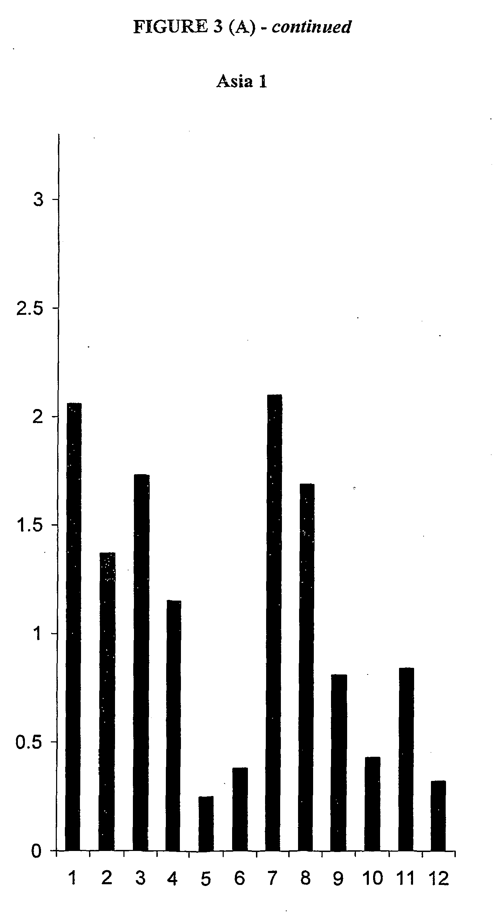

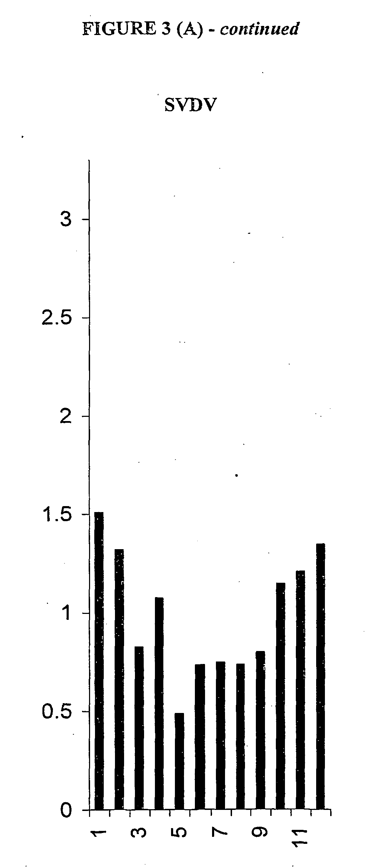

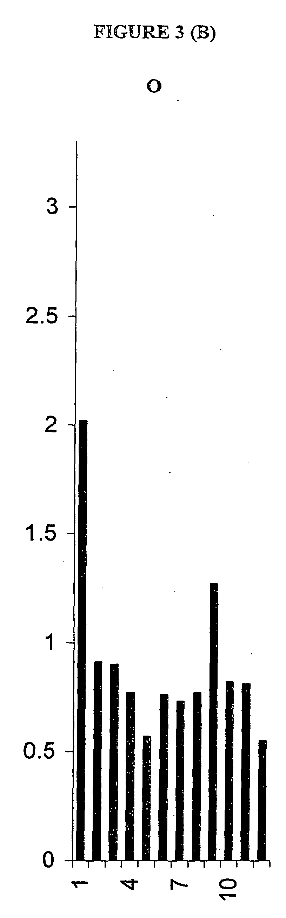

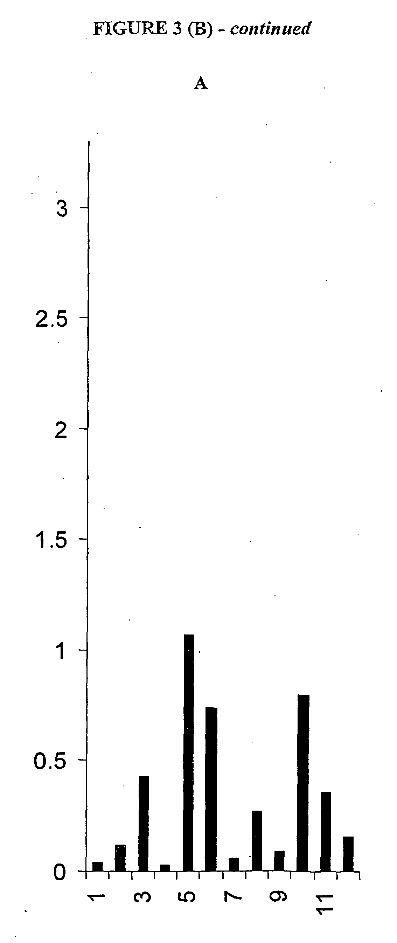

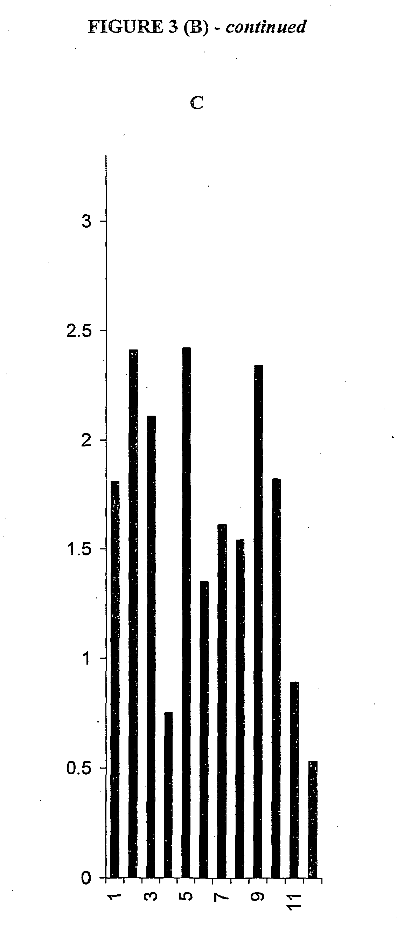

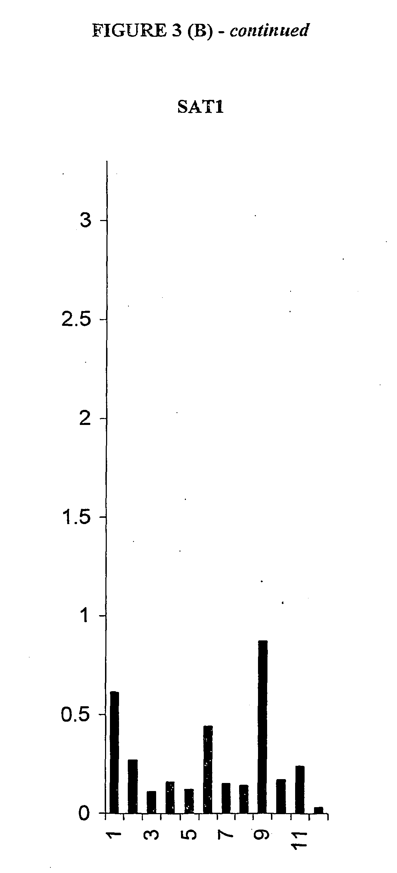

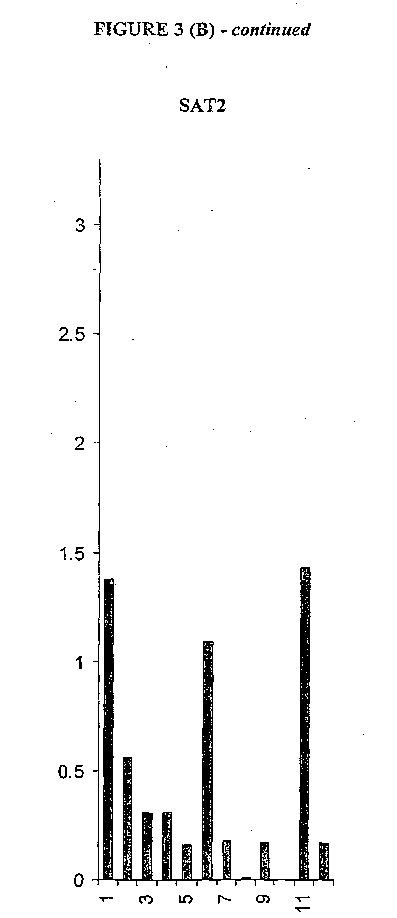

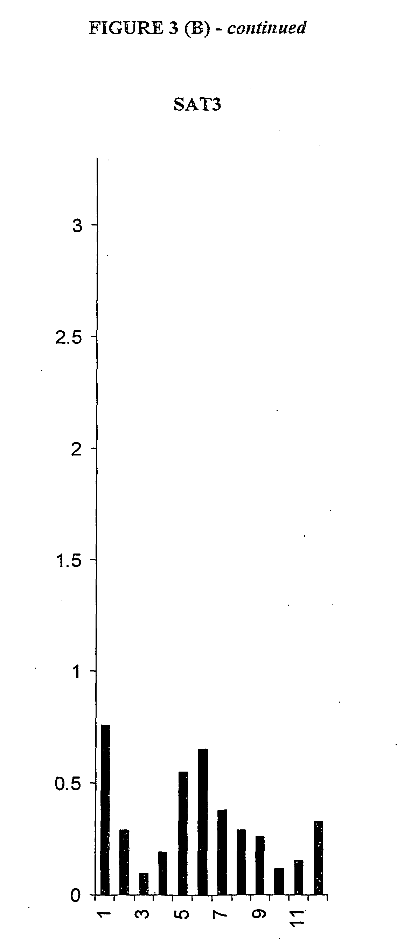

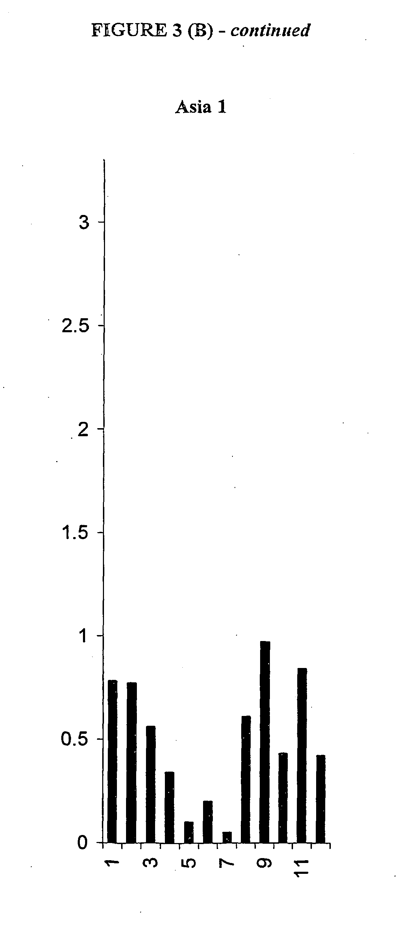

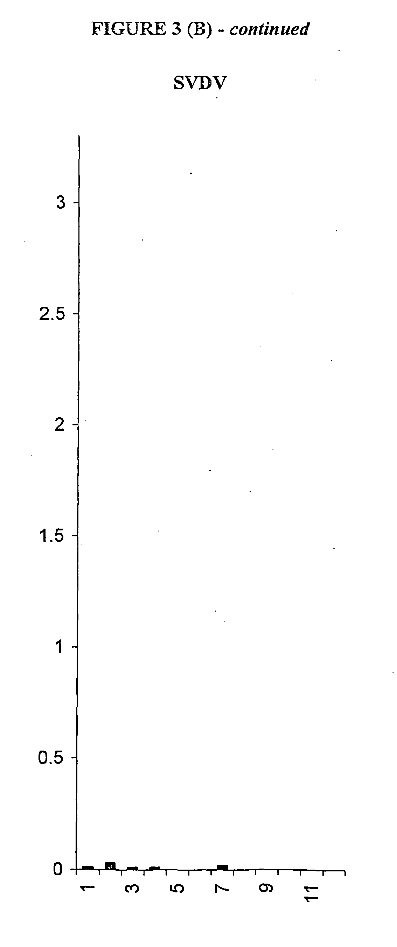

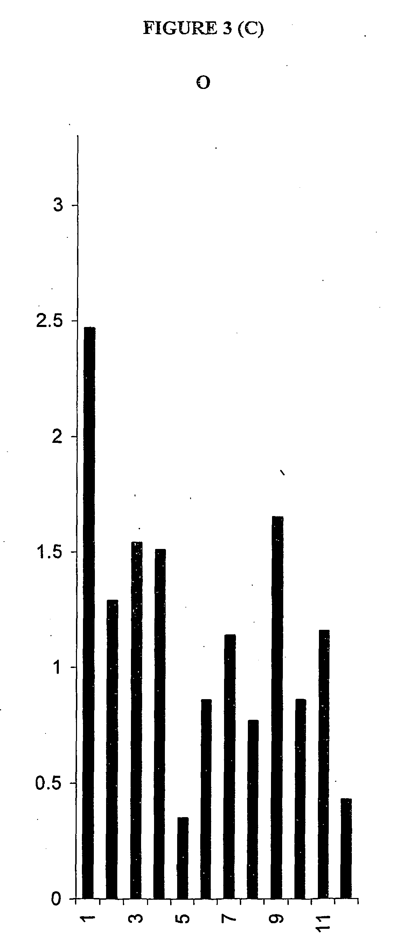

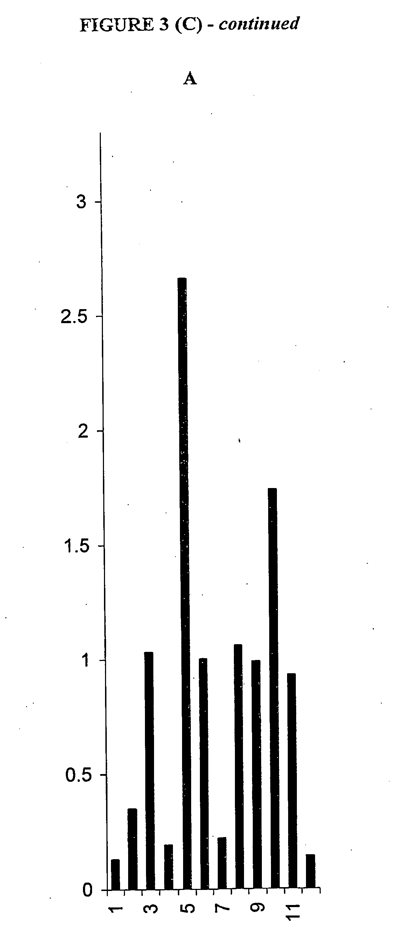

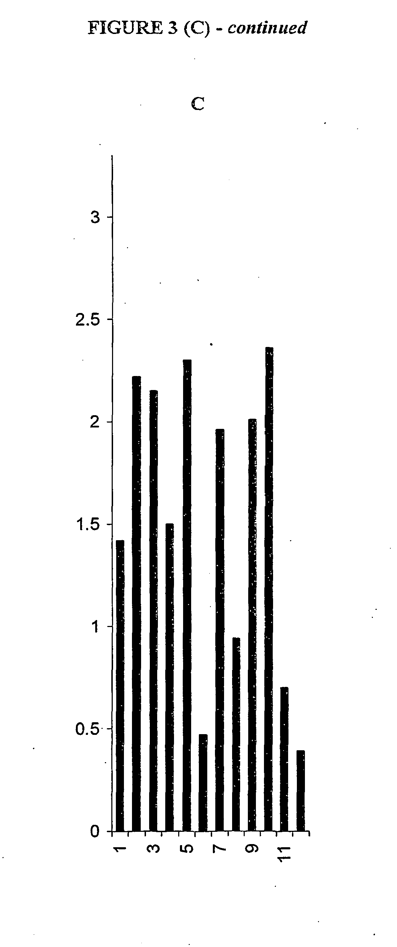

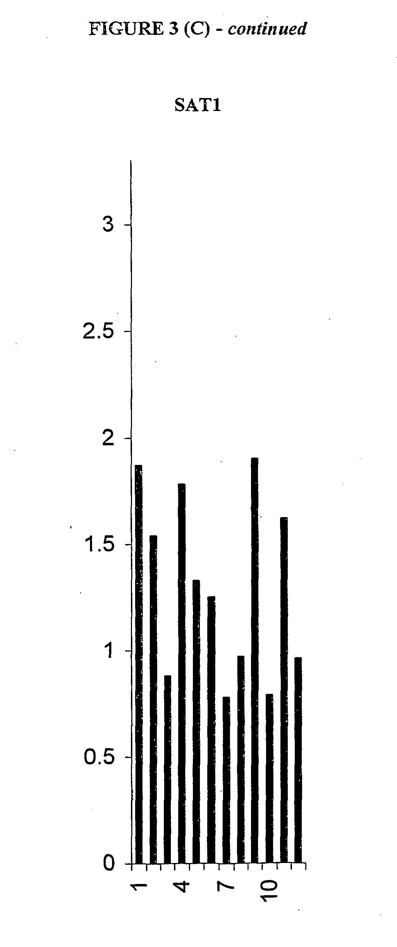

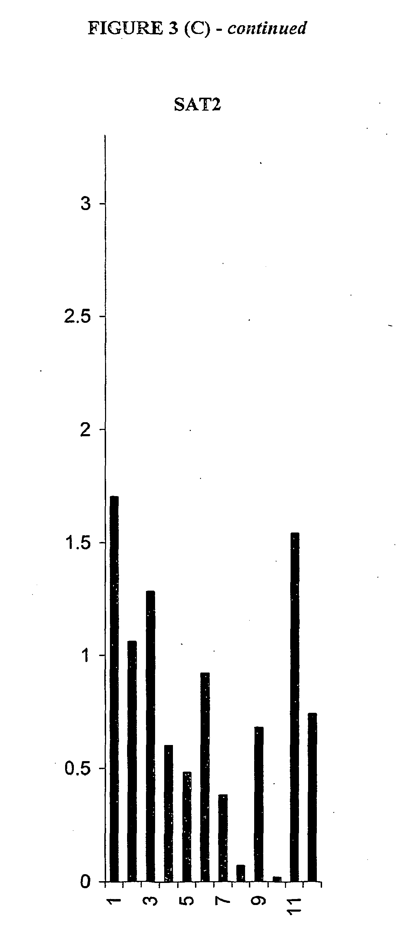

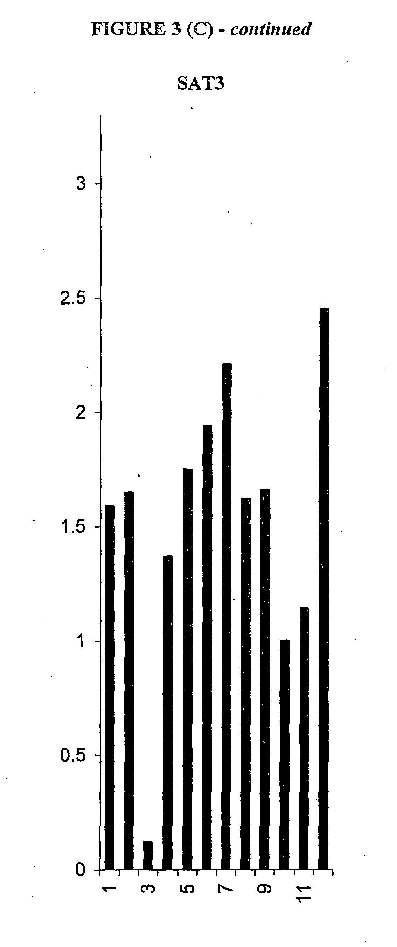

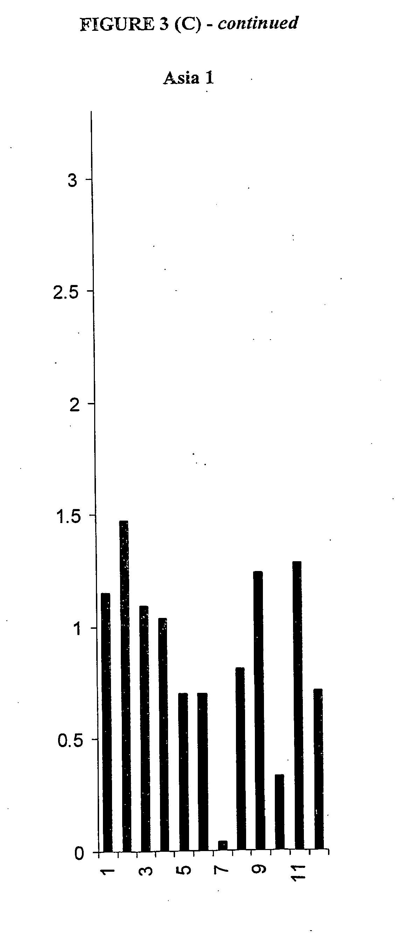

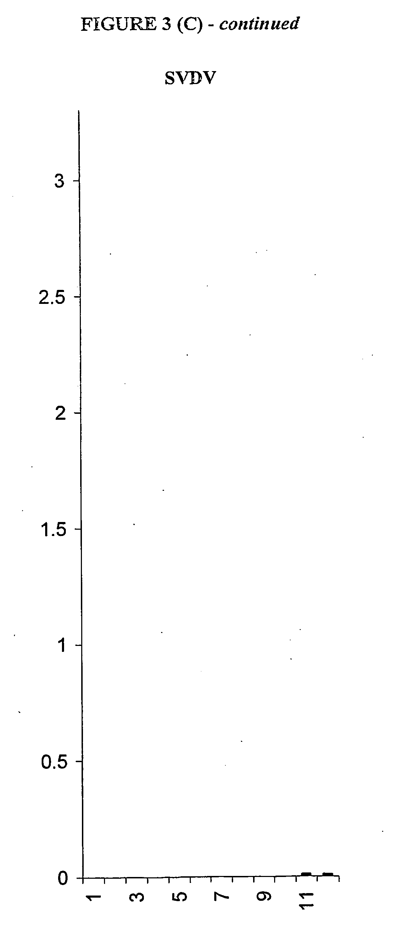

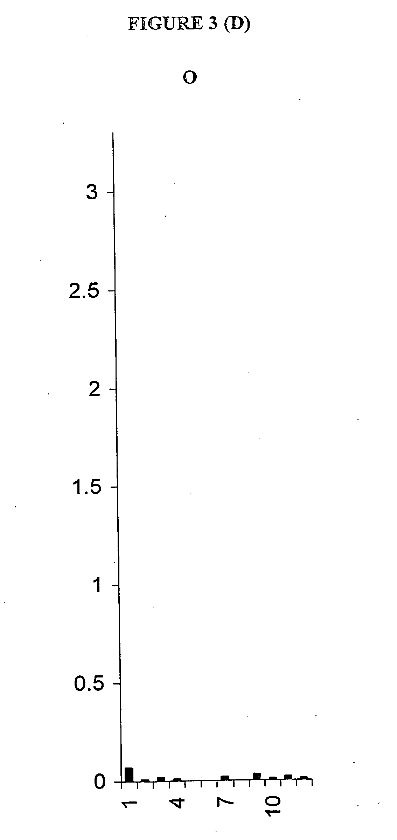

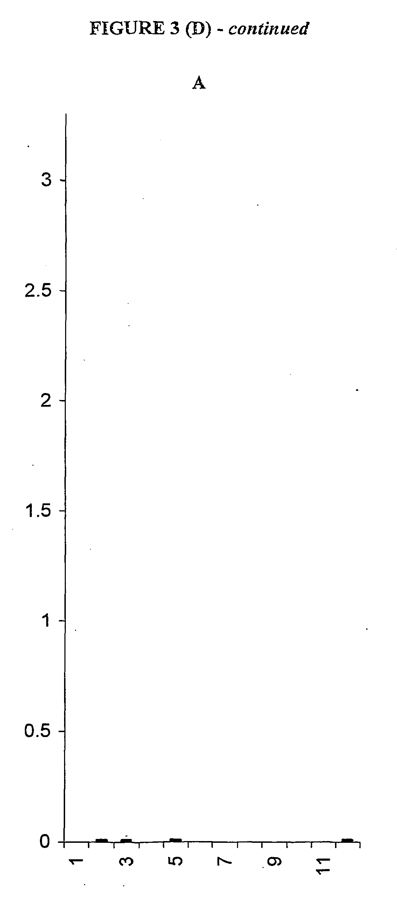

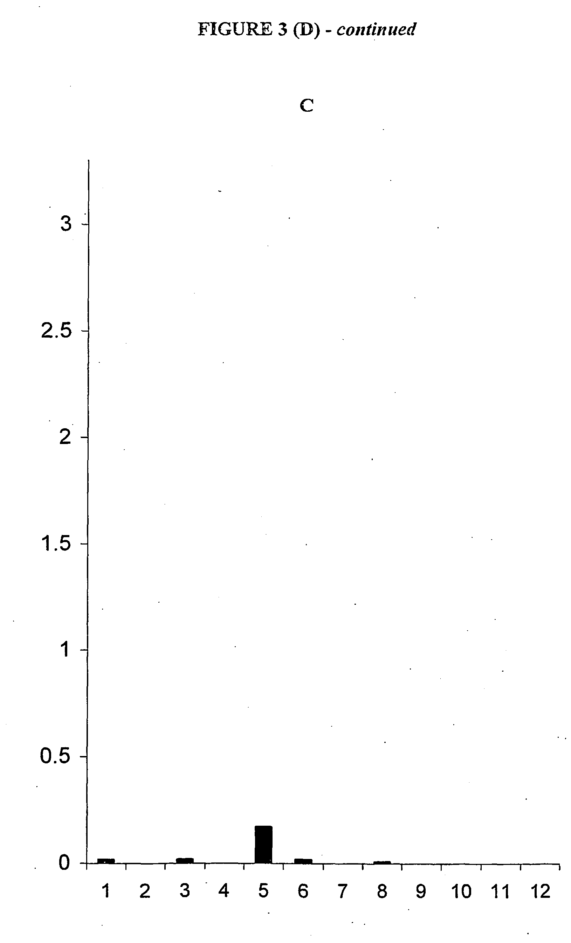

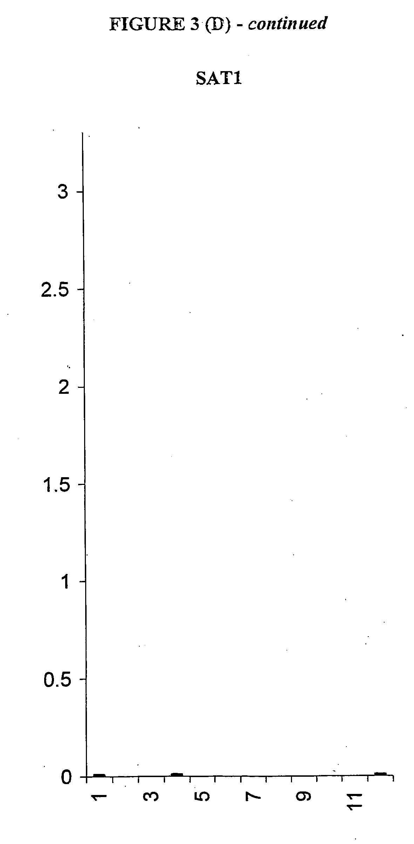

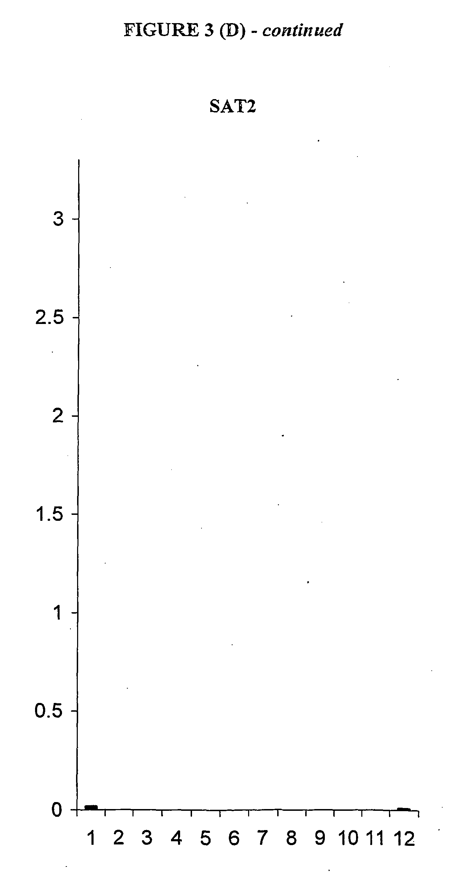

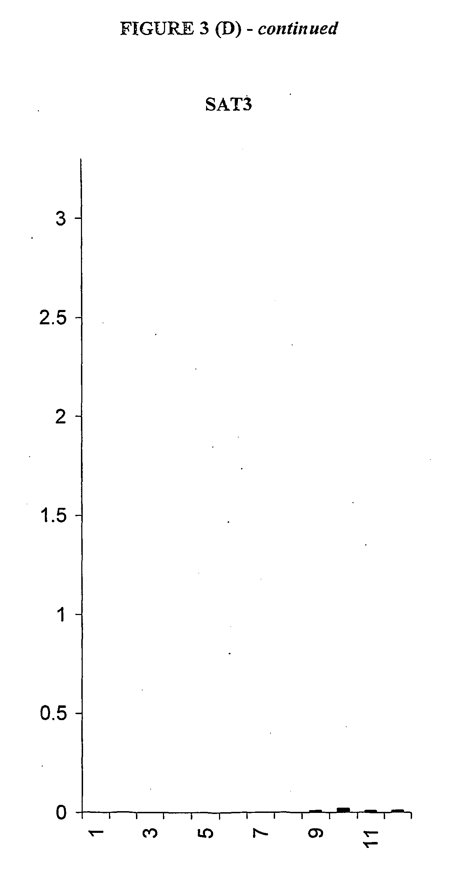

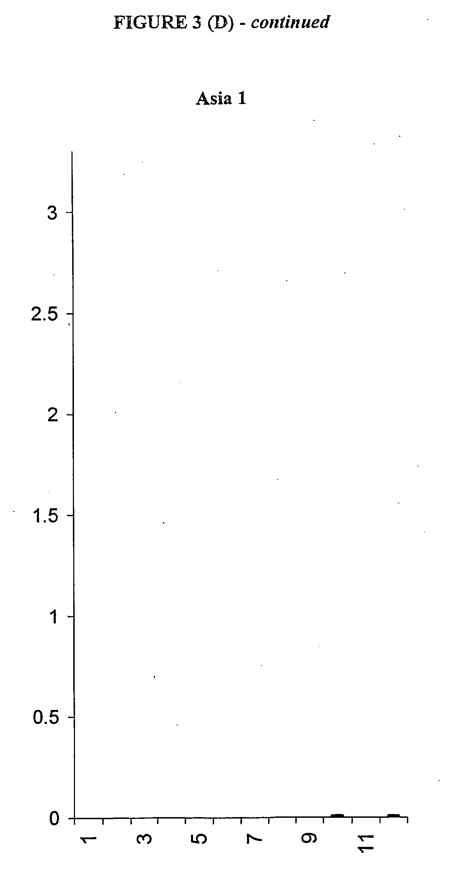

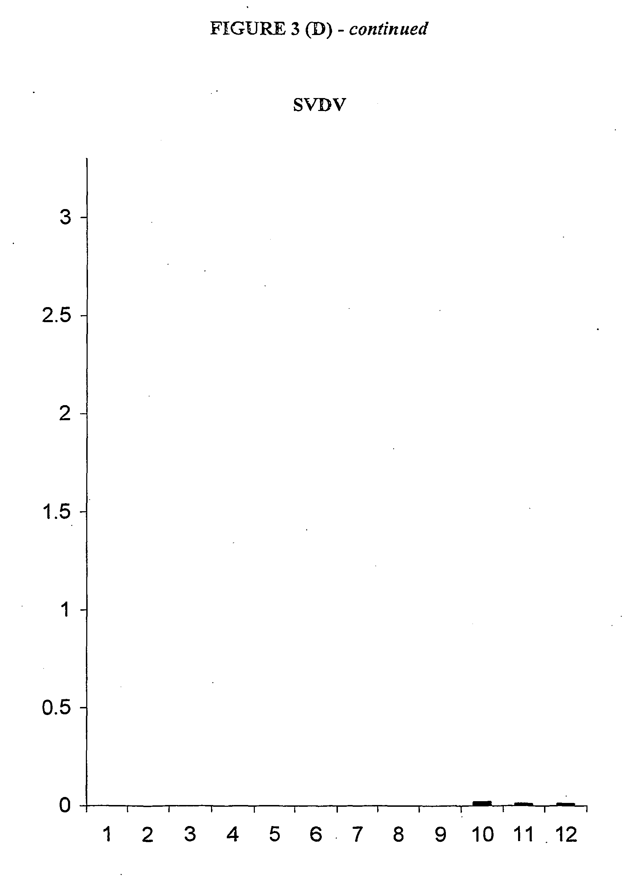

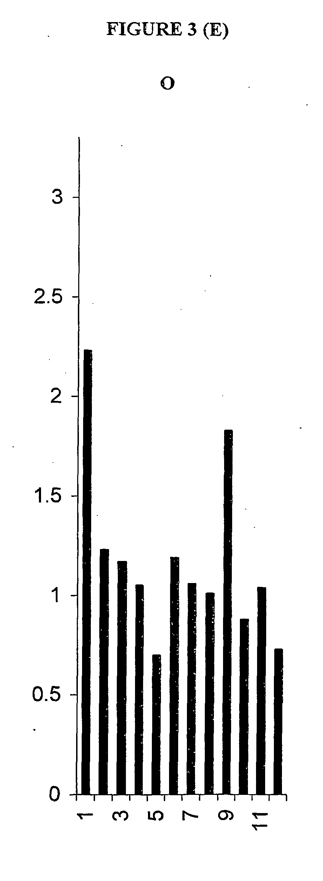

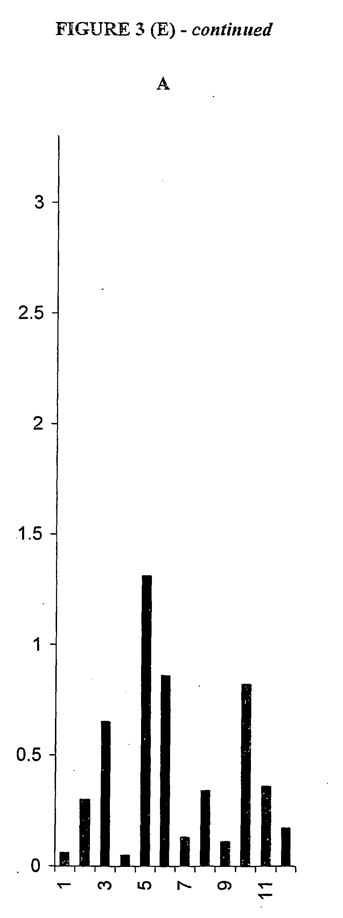

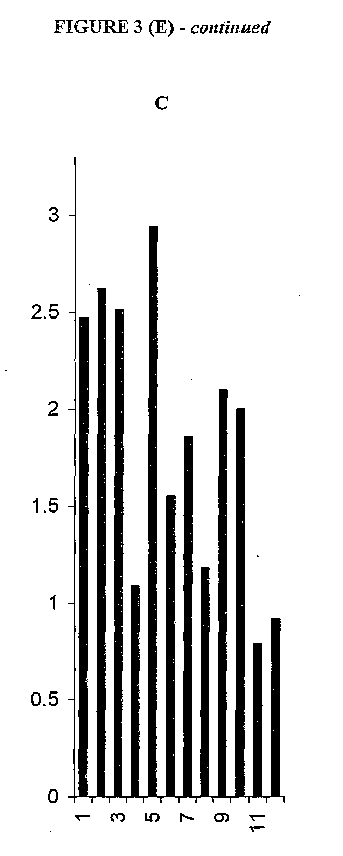

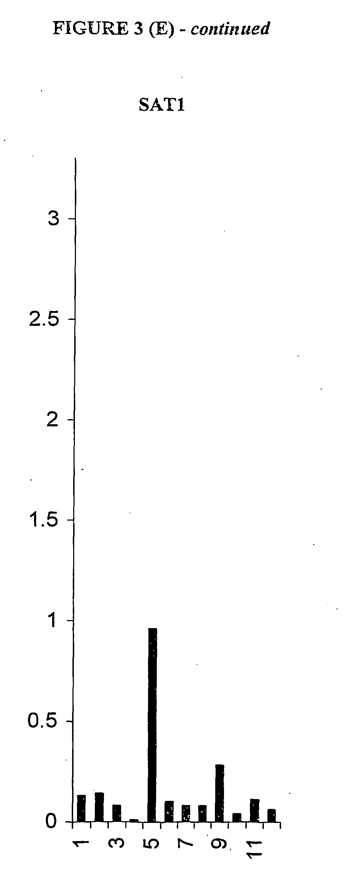

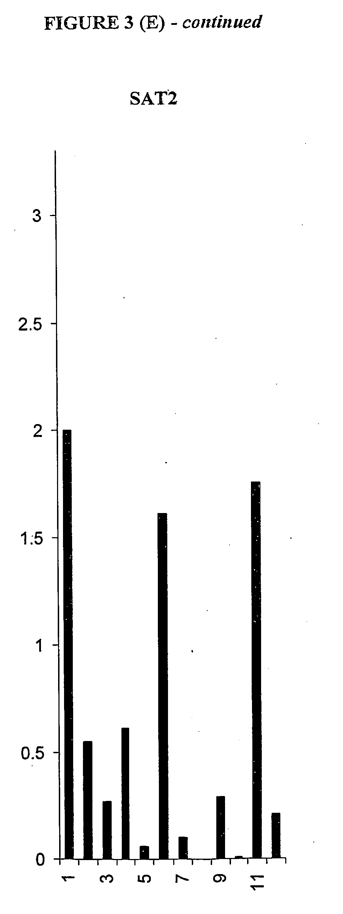

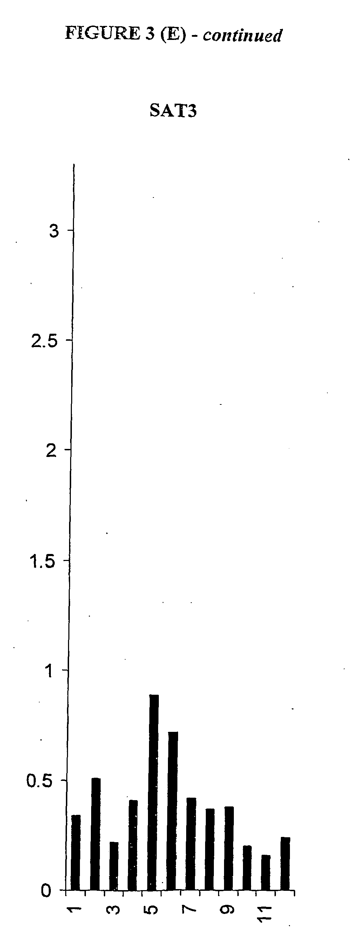

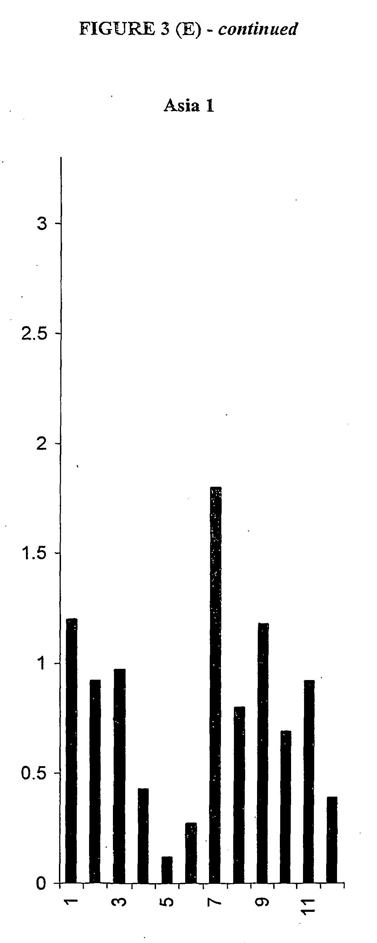

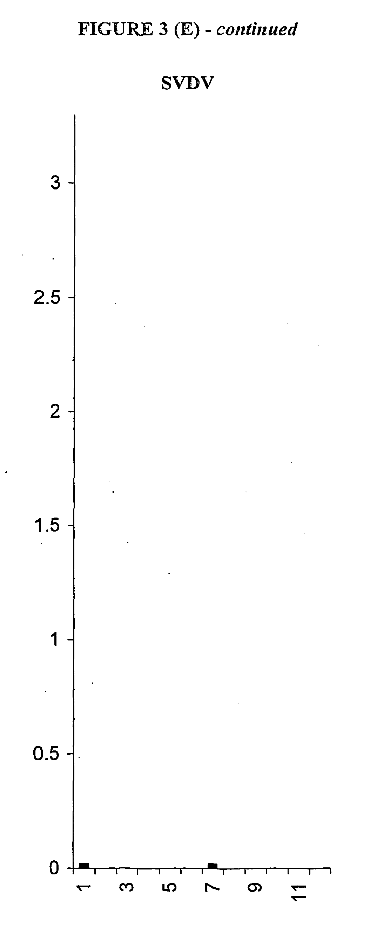

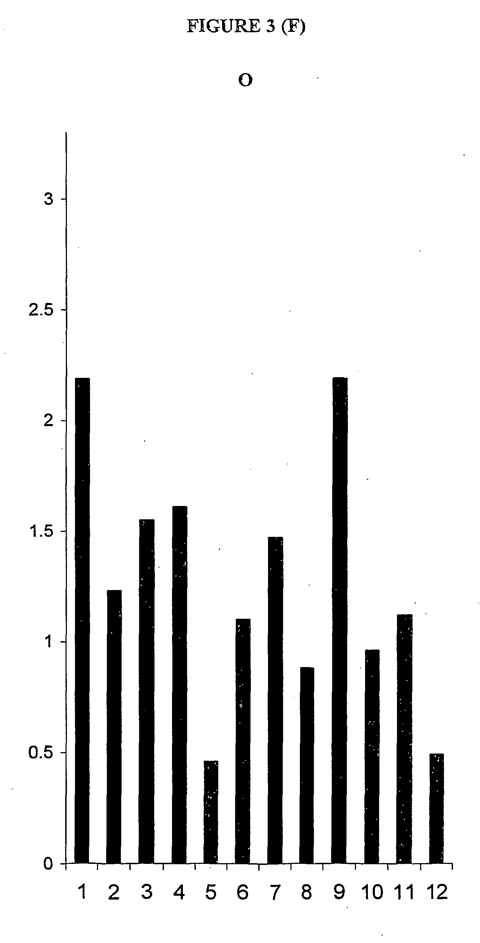

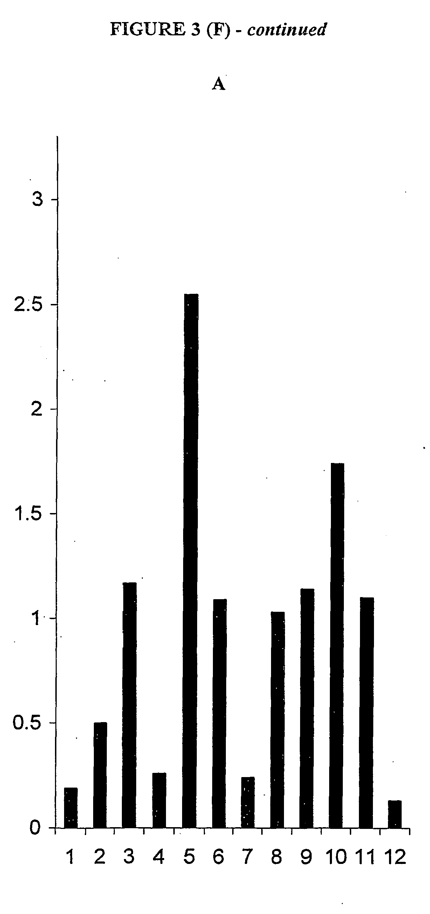

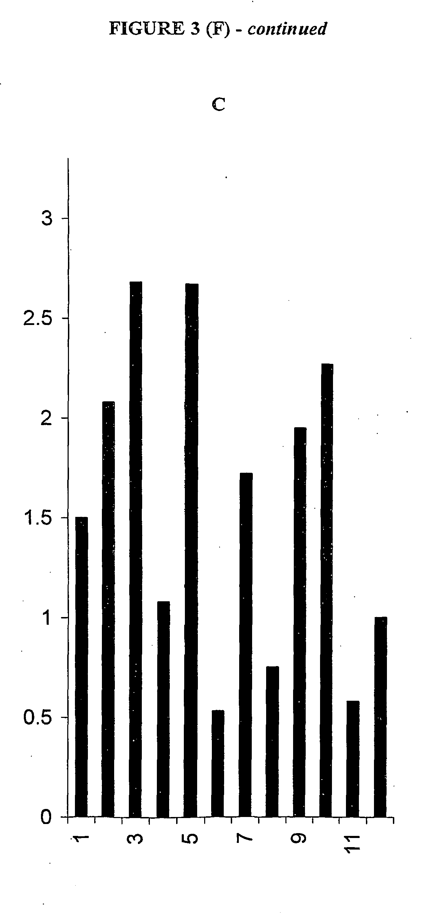

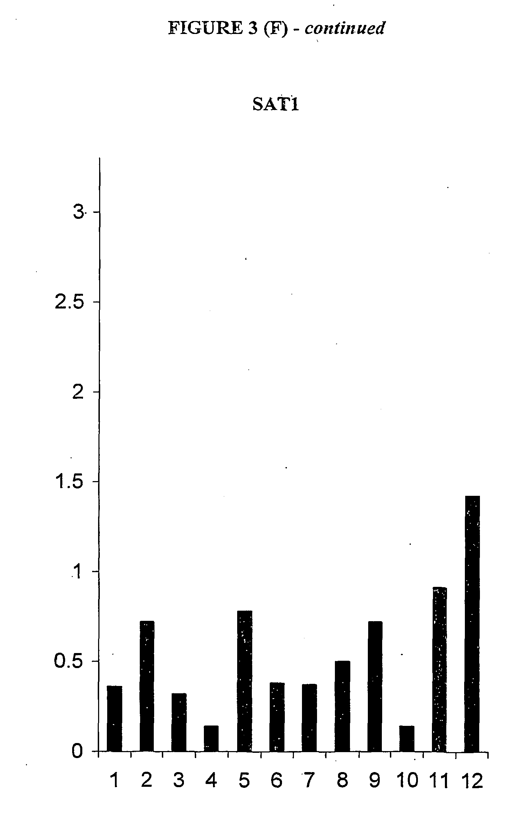

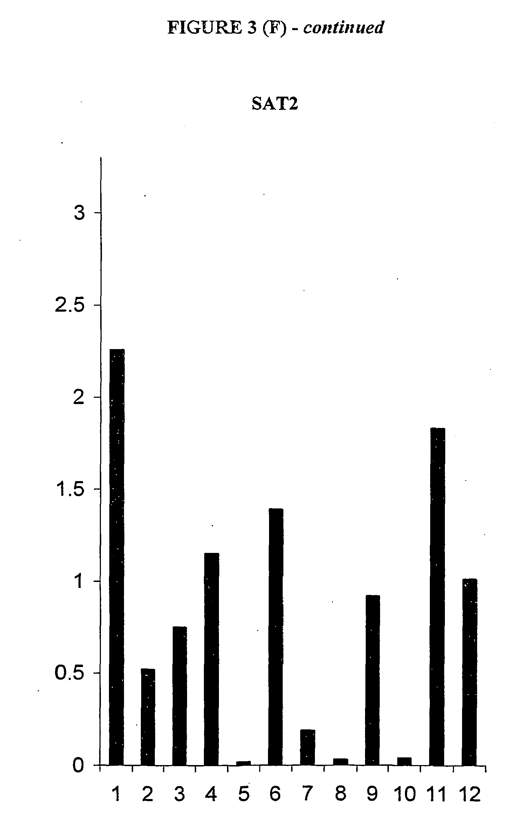

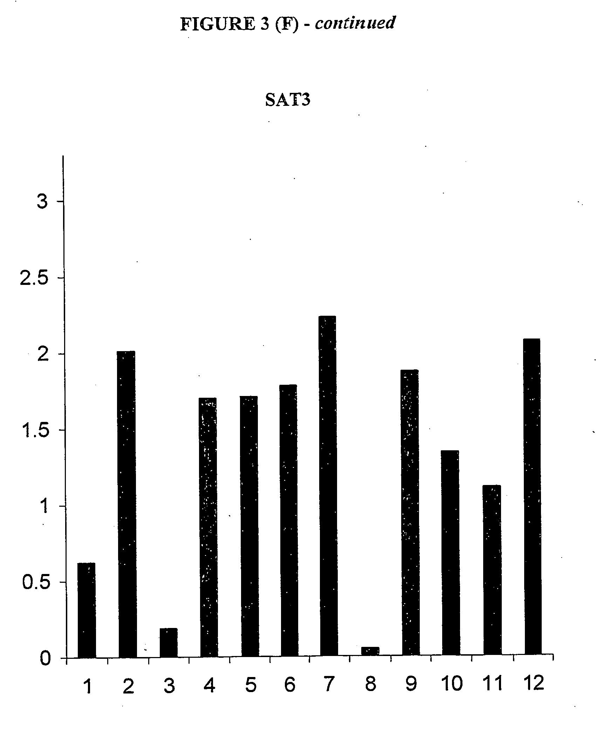

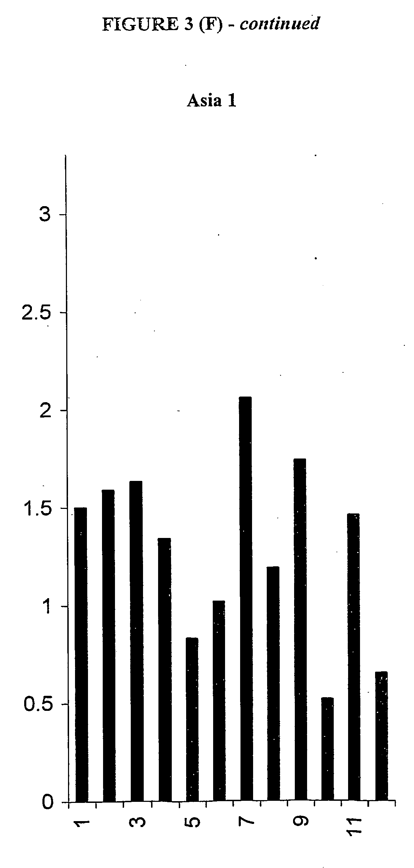

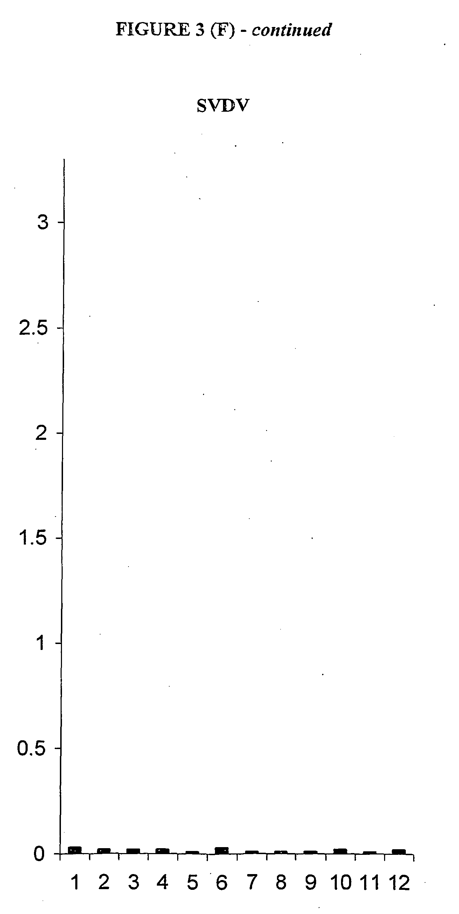

FIG. 3. Homotypic reaction of cell culture grown antigens of each of the seven serotypes of FMD virus plus SVD virus in ELISAs using combinations of reagents for capture/detection of virus as follows. (A) Homotypic rabbit and guinea pig polyclonal antibodies. (B) Homotypic rabbit polyclonal antibodies/pan-reactive FMD virus mab 4A3. (C). Integrin/pan-reactive FMD virus mab 4A3. (D) Non-coated/pan-reactive FMD virus mab 4A3. (E) Homotypic rabbit polyclonal antibodies/pan-reactive FMD virus mab 5F10. (F) Integrin/pan-reactive FMD virus mab 5F10. In each case, the *x*-axis denotes virus number (1 to 12) and the *y-*axis denotes optical density.

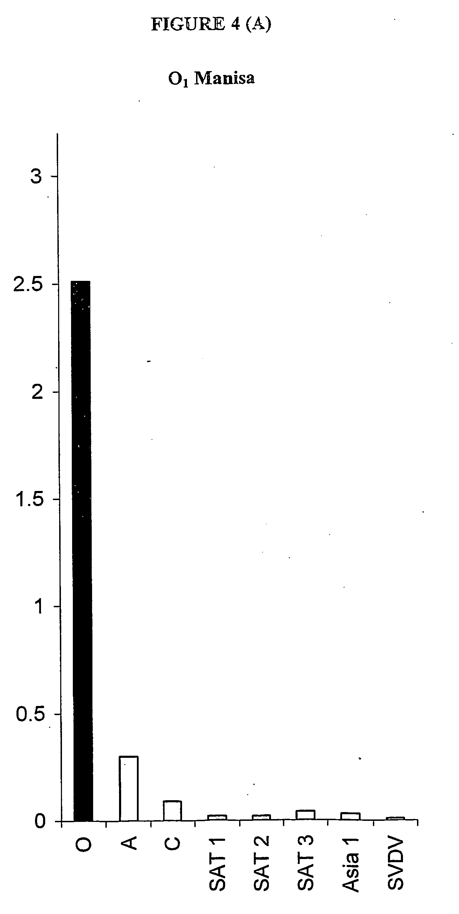

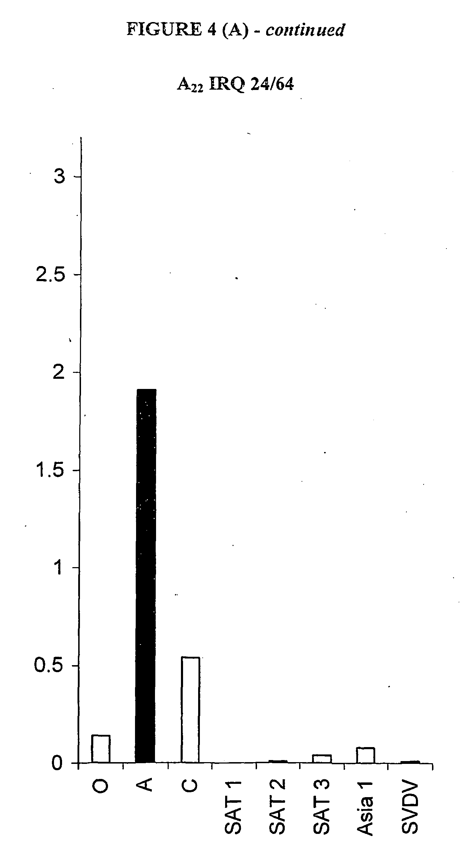

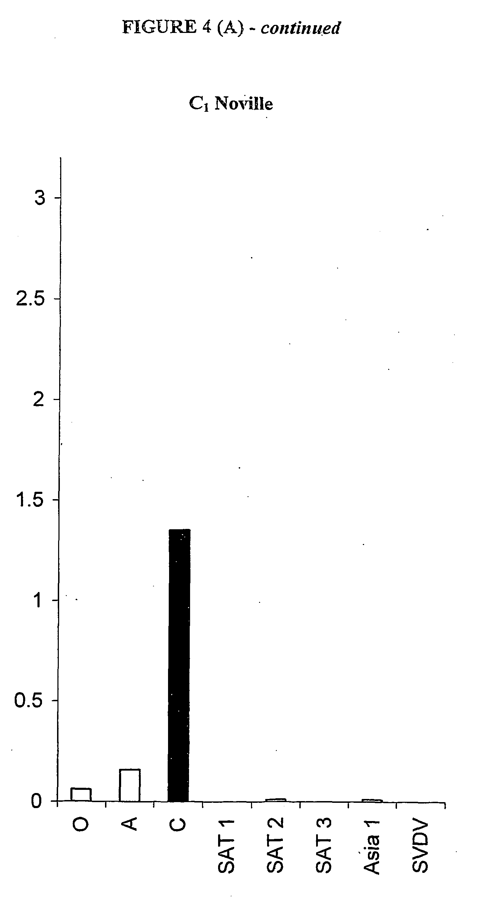

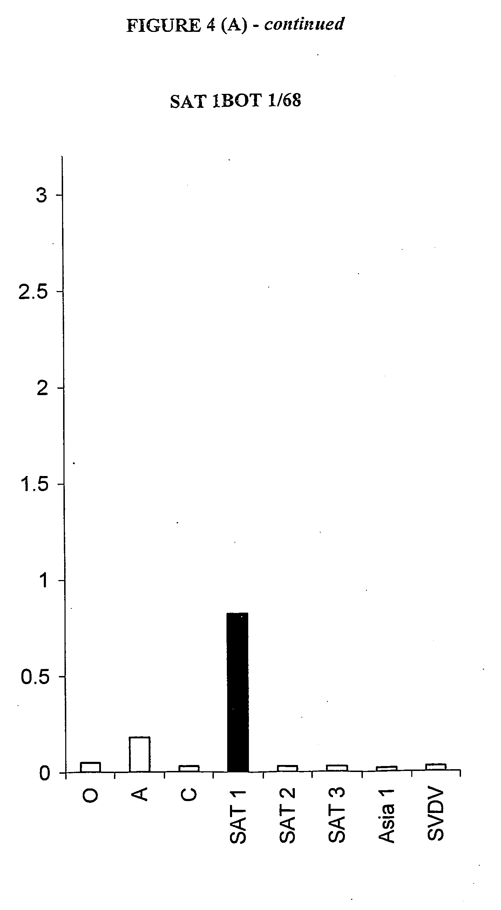

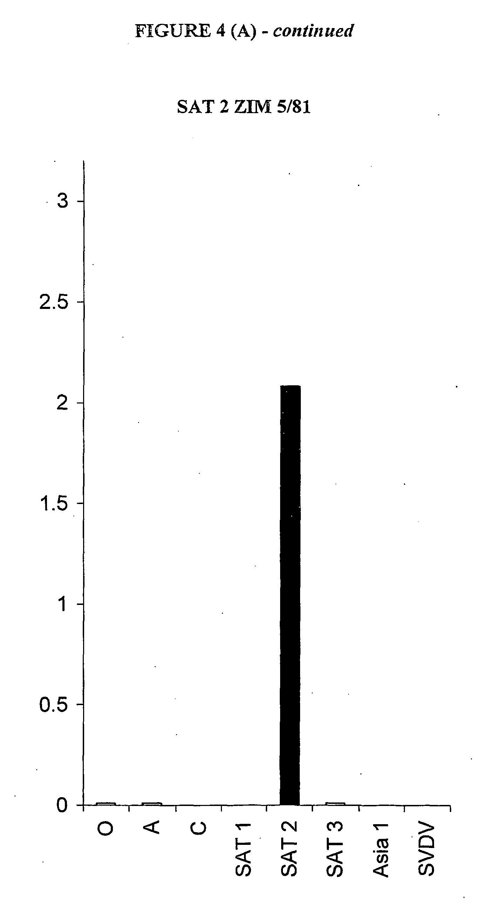

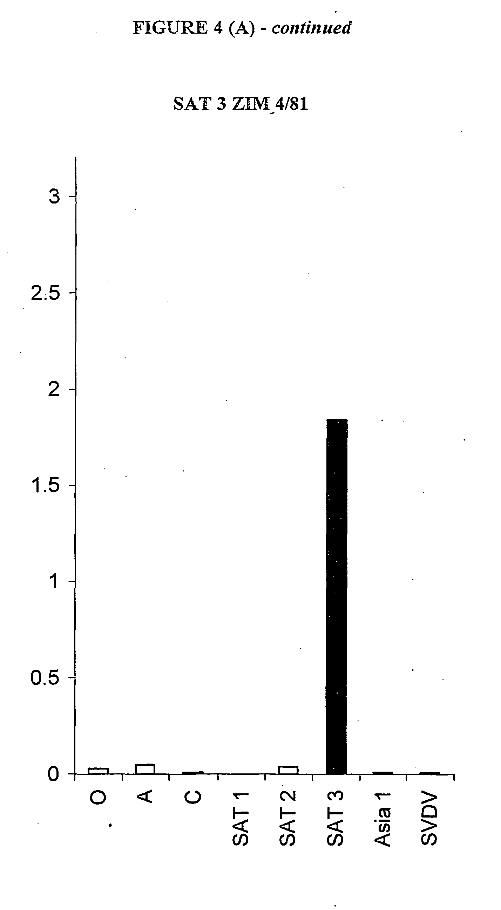

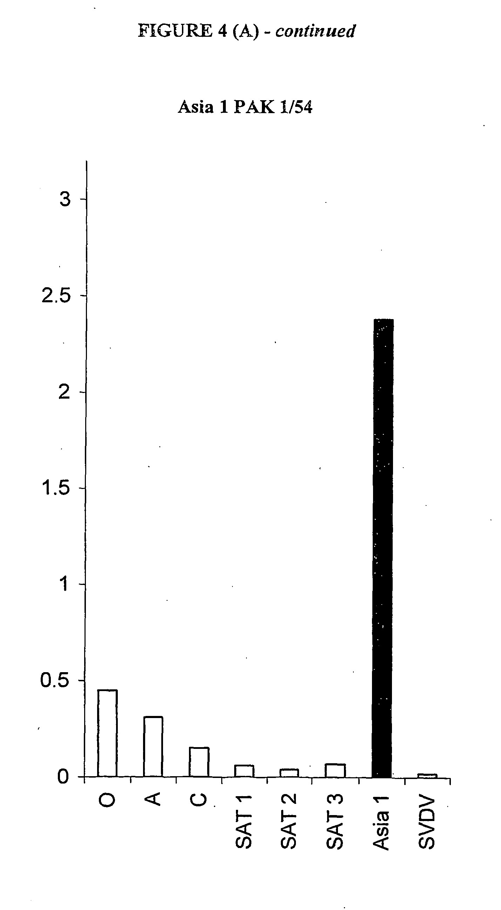

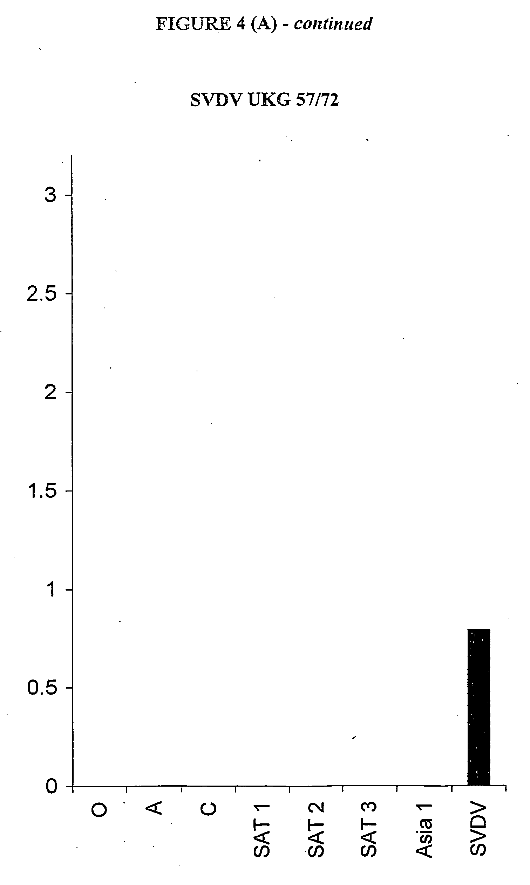

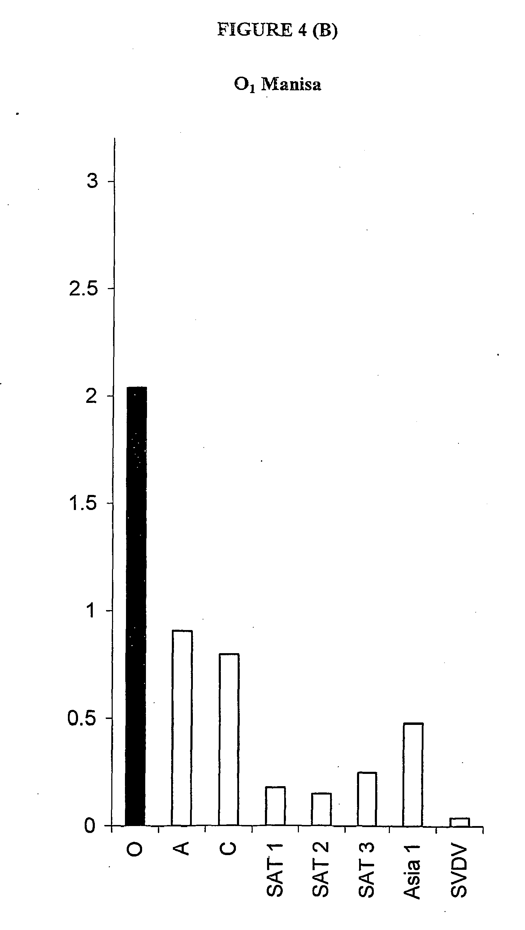

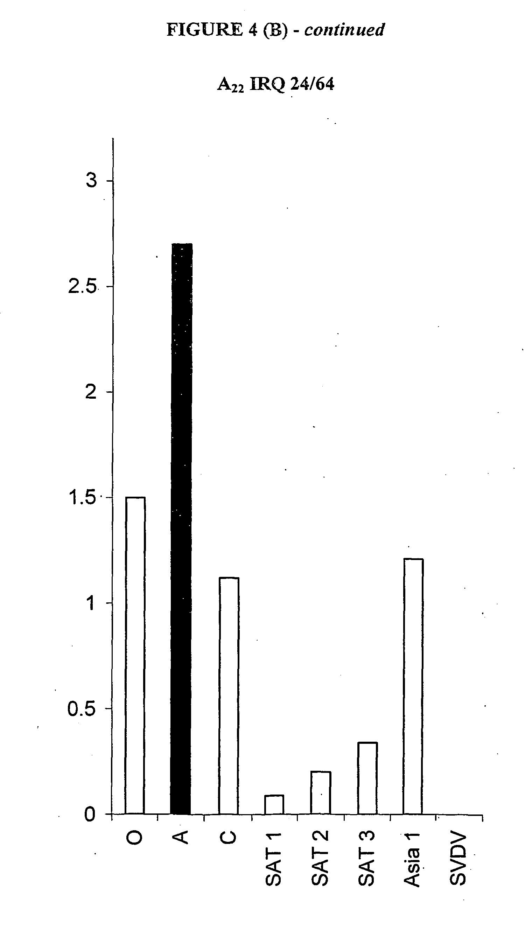

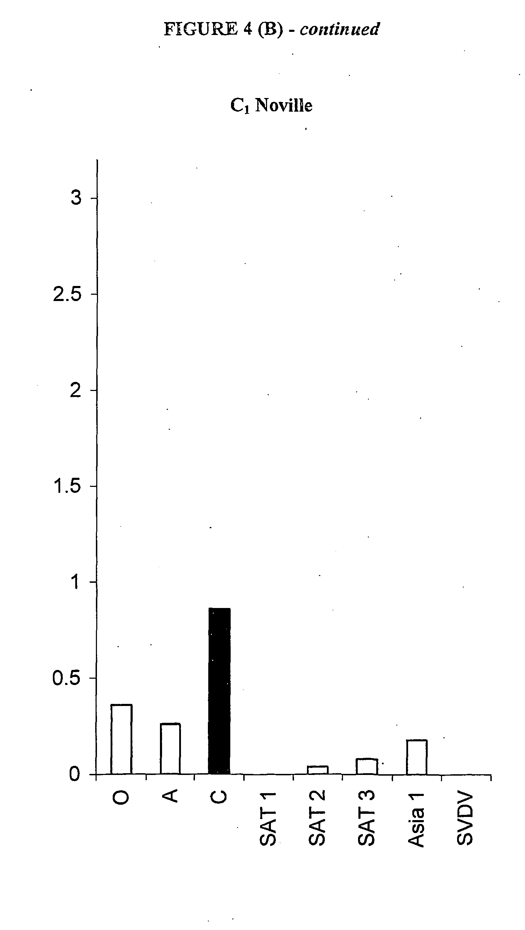

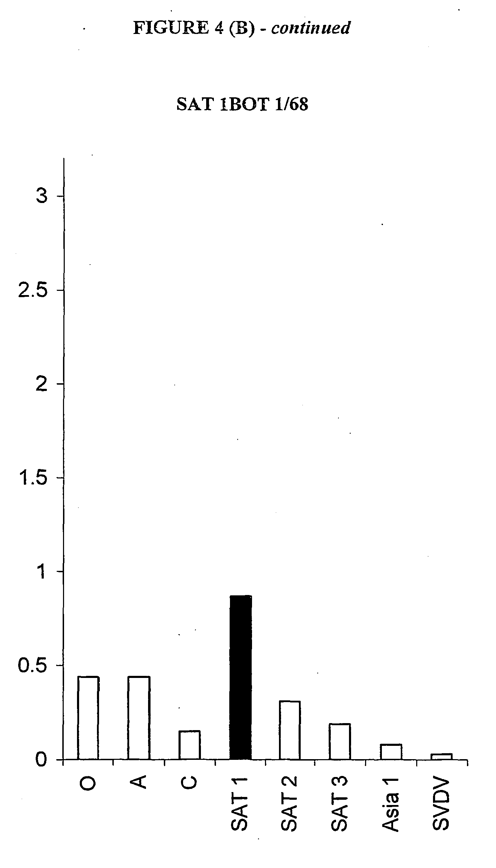

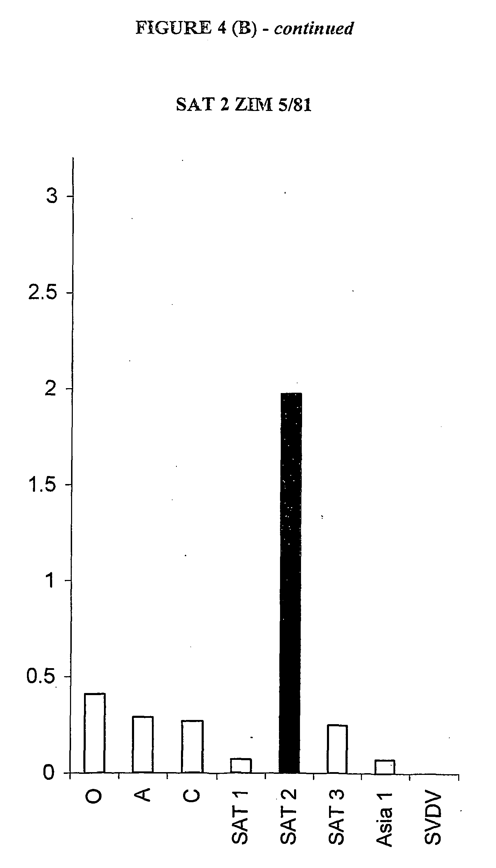

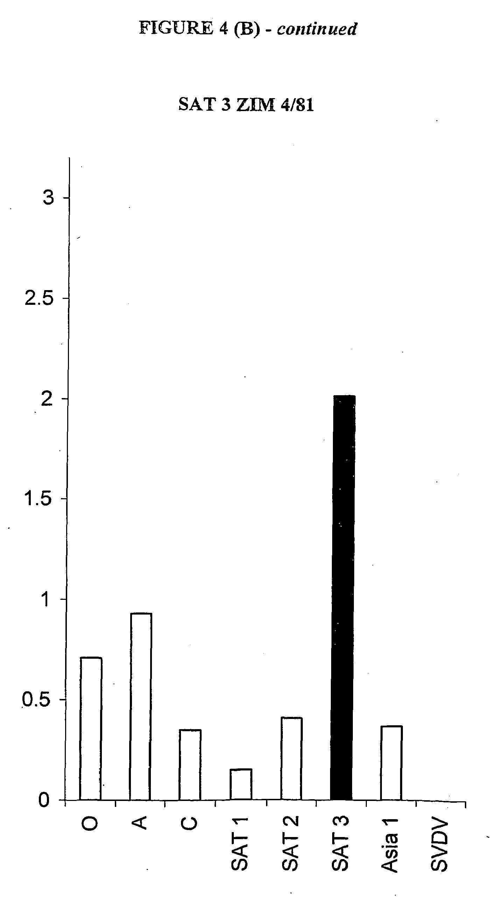

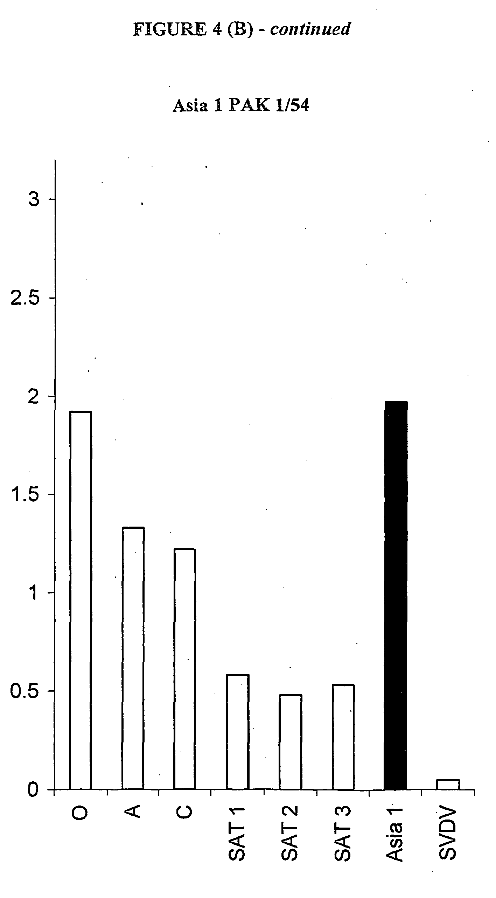

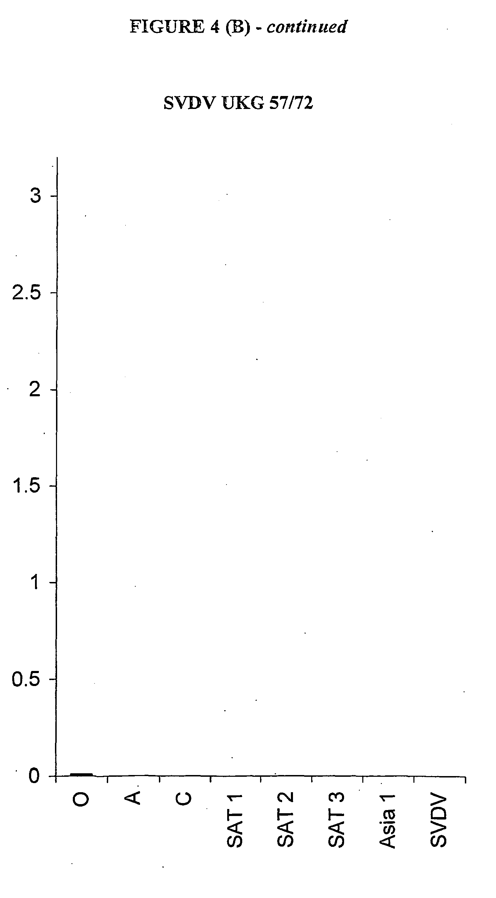

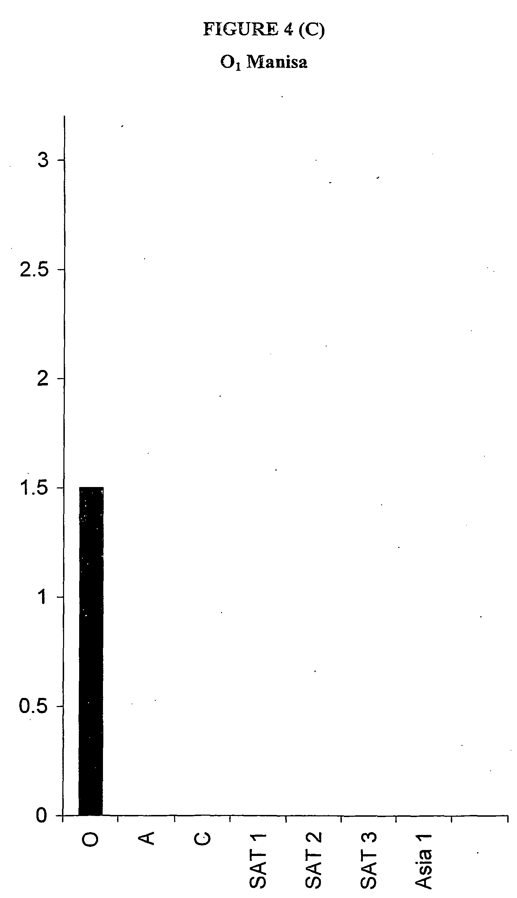

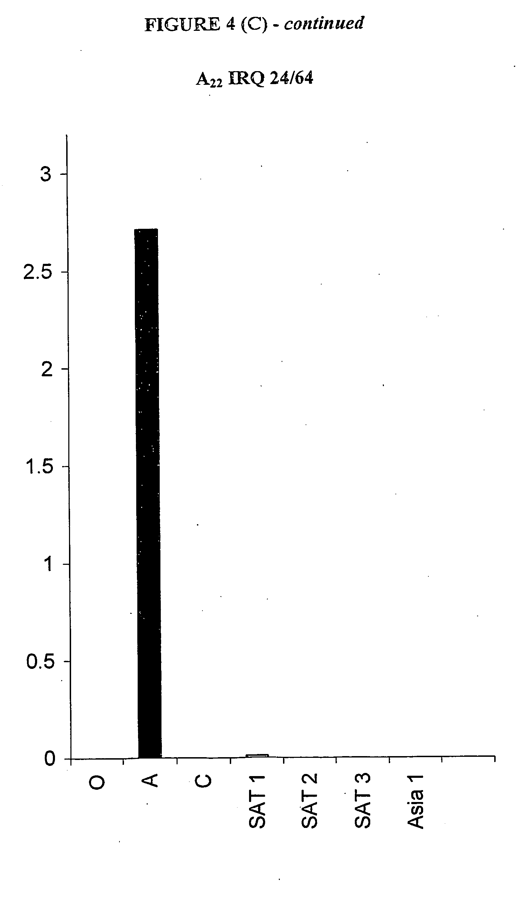

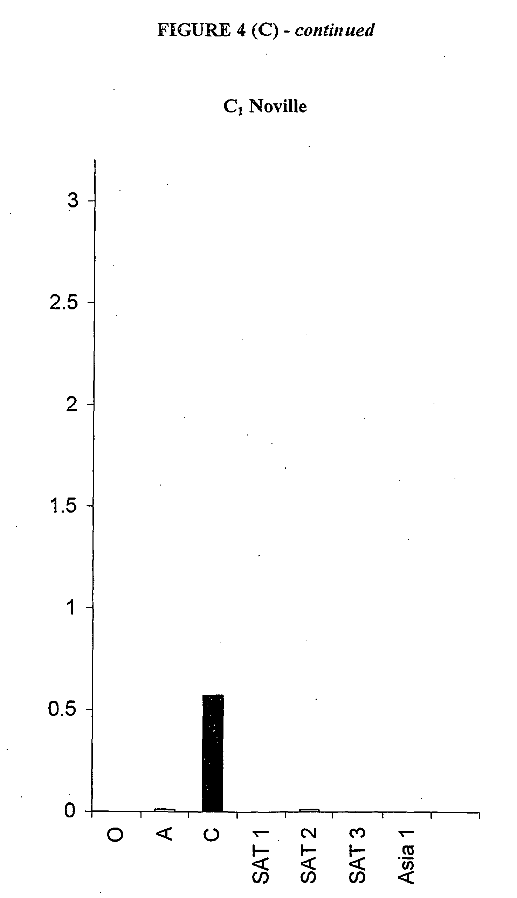

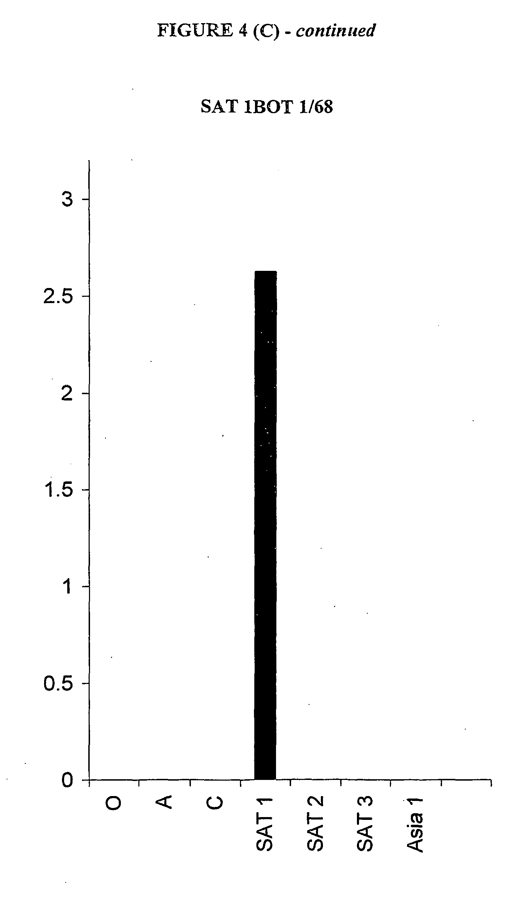

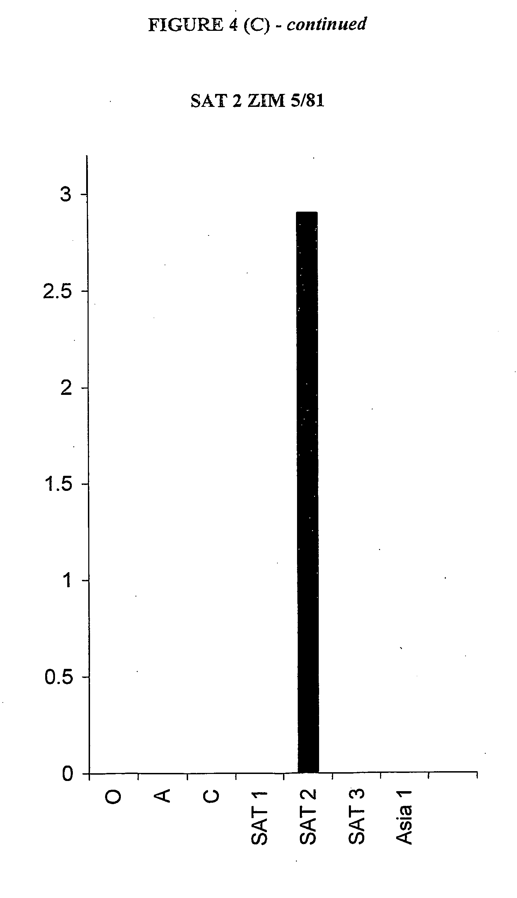

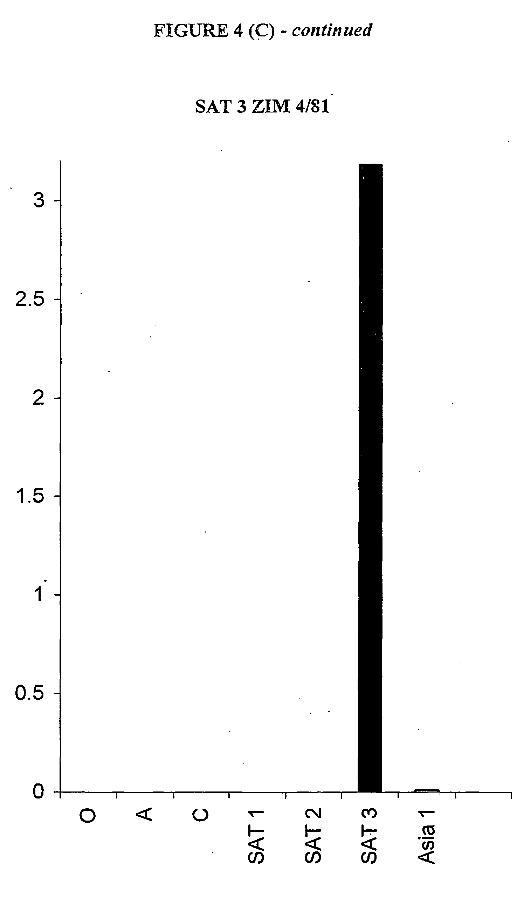

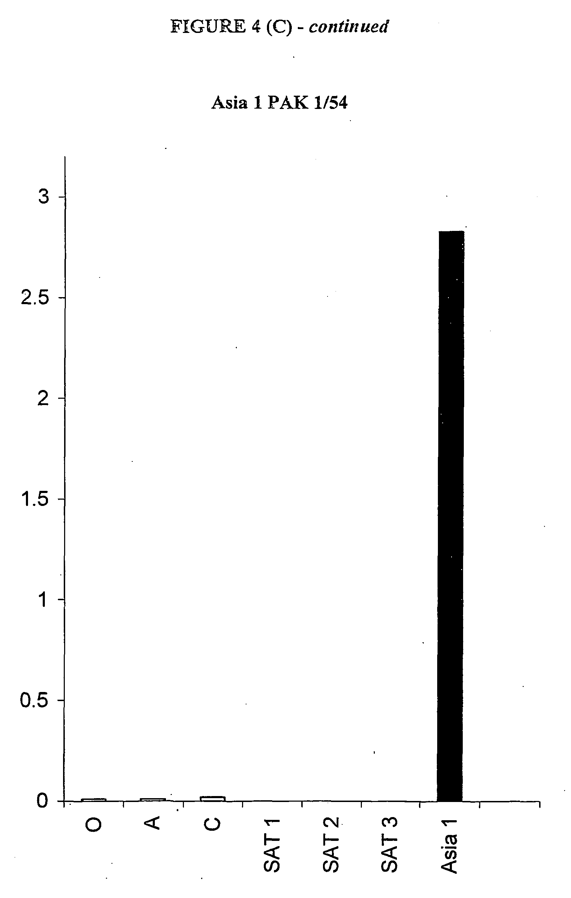

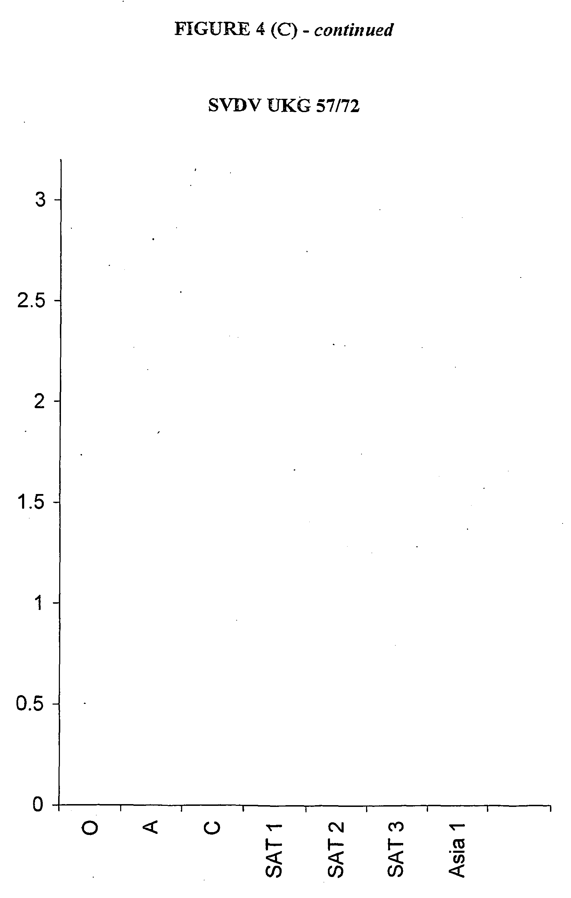

FIG. 4. Homo- and heterotypic reactivity of cell culture grown antigens of each of the seven serotypes of FMD virus plus SVD virus (serotype and strain as indicated by the graph headings) using combinations of reagents for capture/detection of virus as follows. (A) Homotypic rabbit/guinea pig polyclonal antibodies. (B) Integrin/homotypic guinea pig polyclonal antibodies. (C) Integrin/homoypic monoclonal antibodies. Homotypic reactions as bold plots and heterotypic reactions as empty plots. Virus serotype (order on each *x*-axis: FMD virus types O, A, C, SAT1, SAT2, SAT3 and Asia 1 plus SVDV). In each case, the *y*-axis denotes optical density.

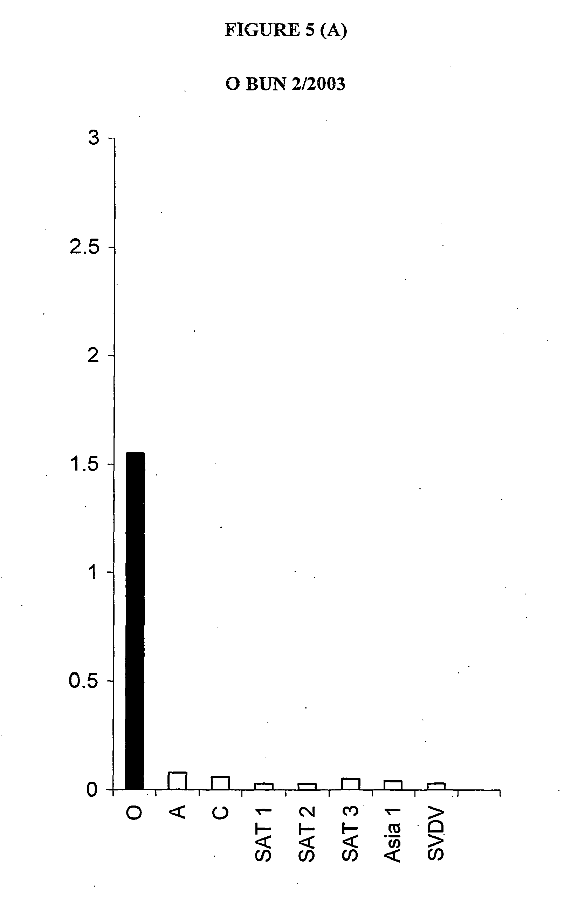

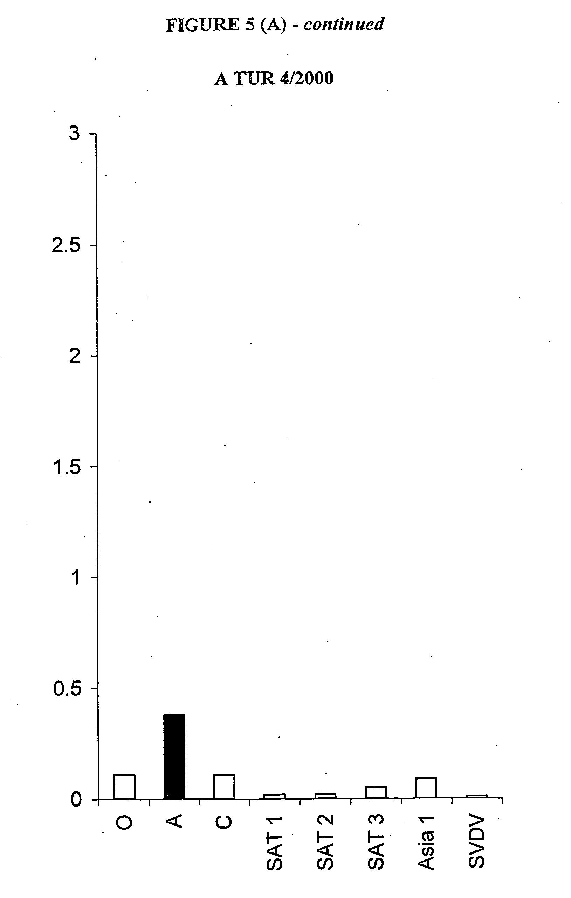

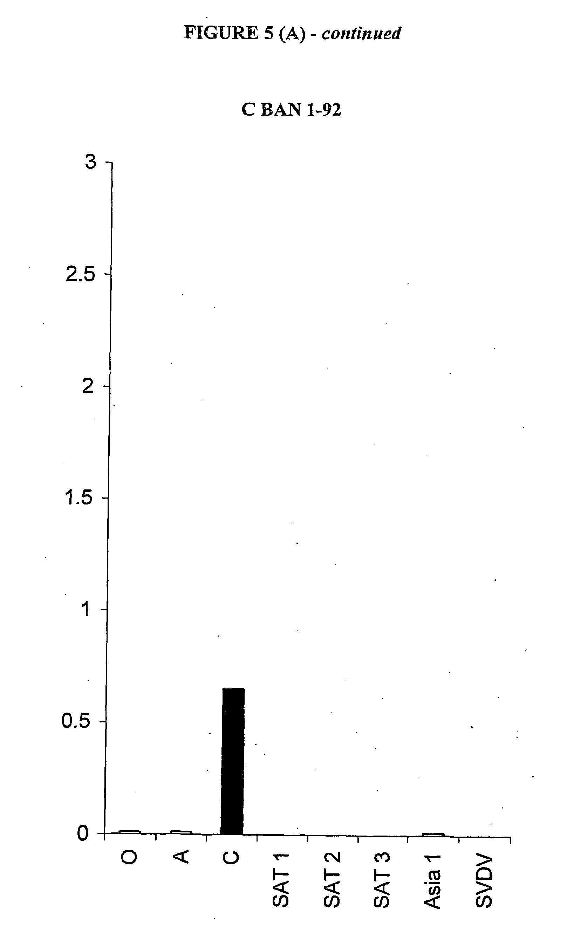

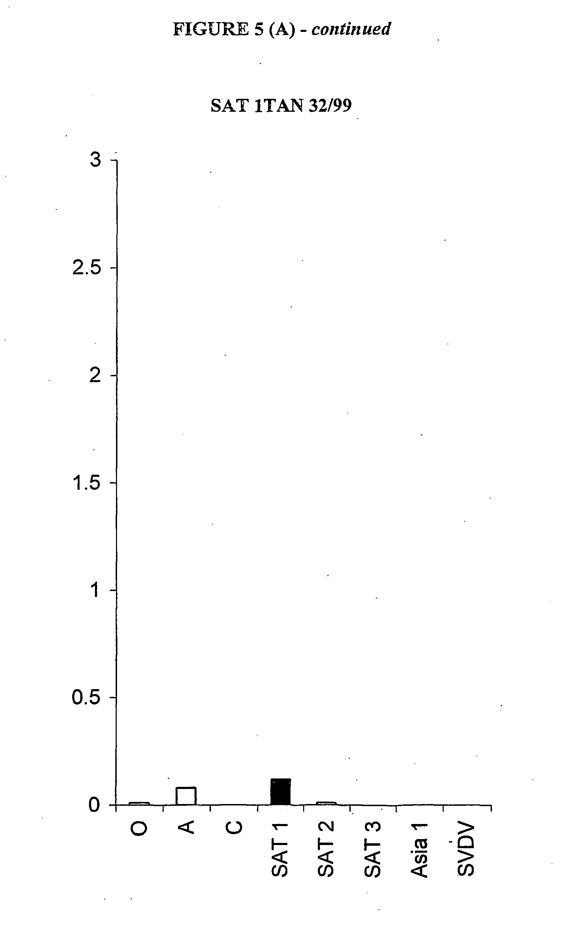

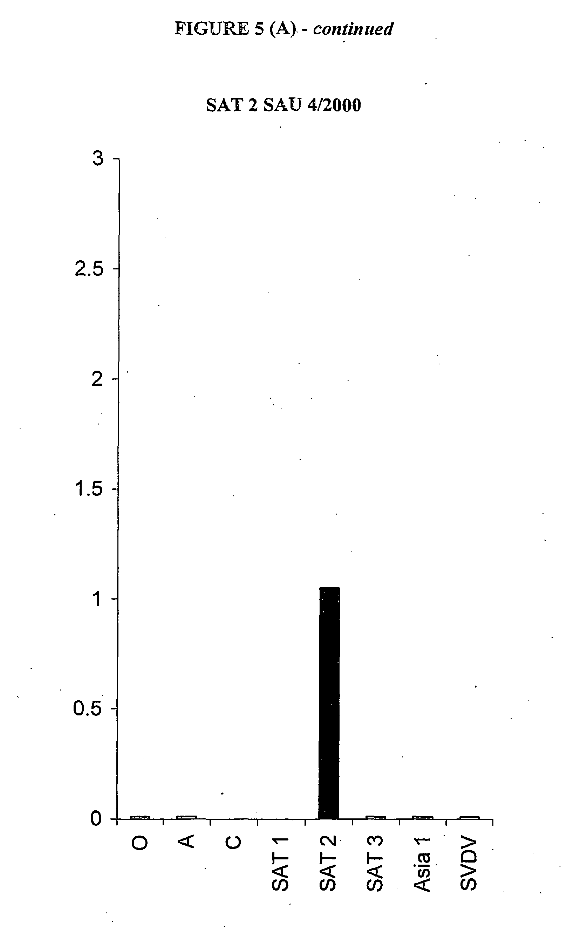

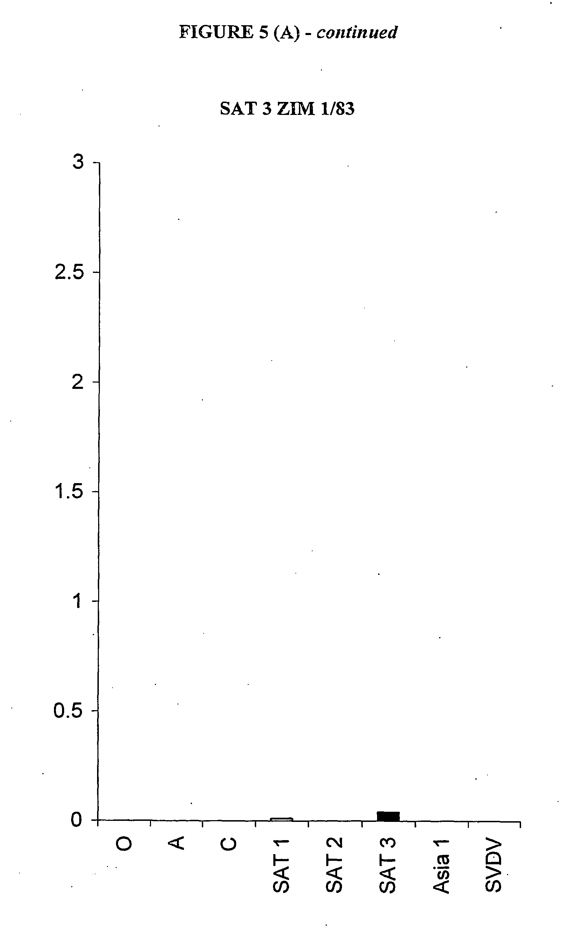

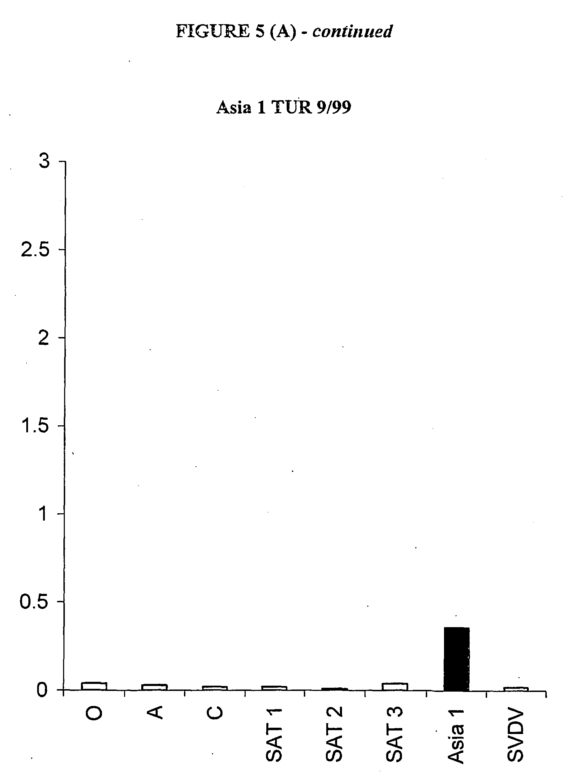

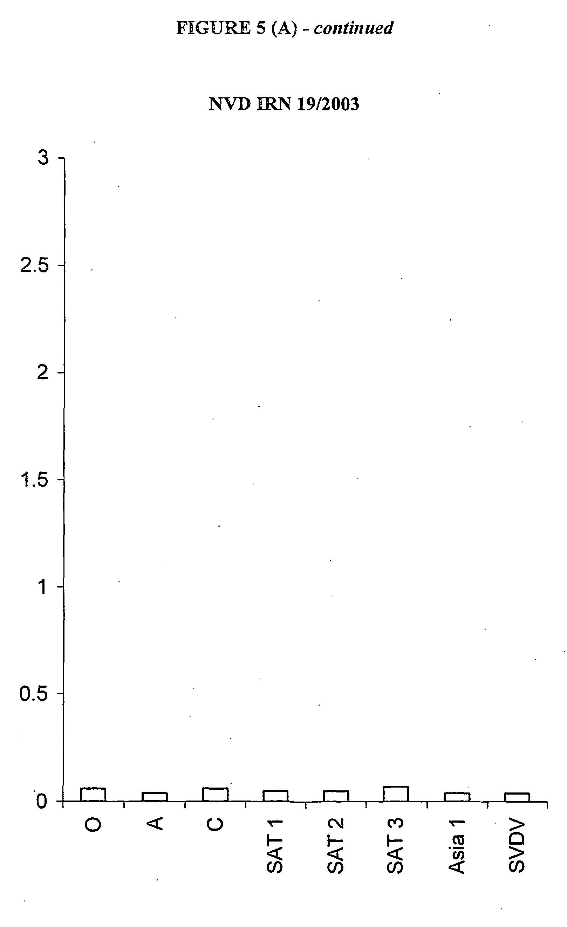

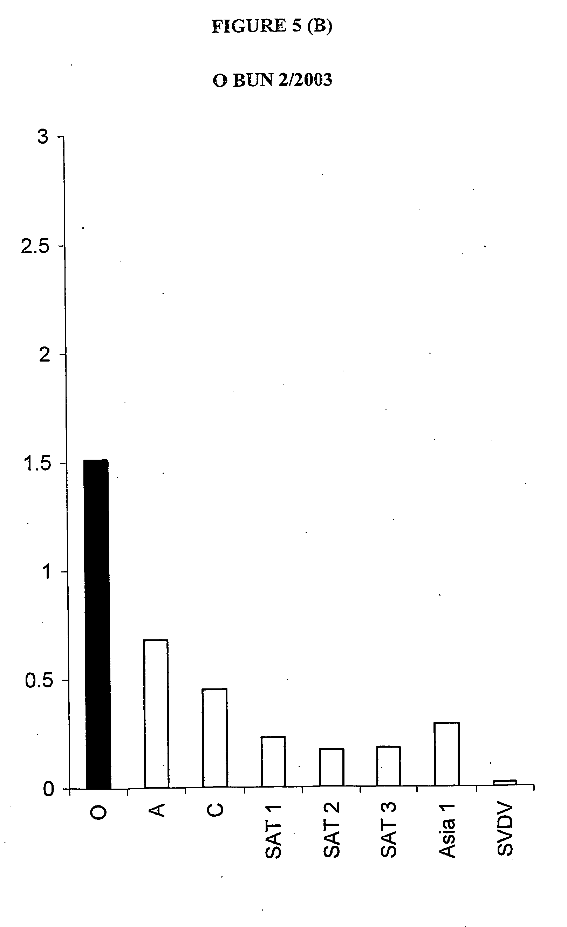

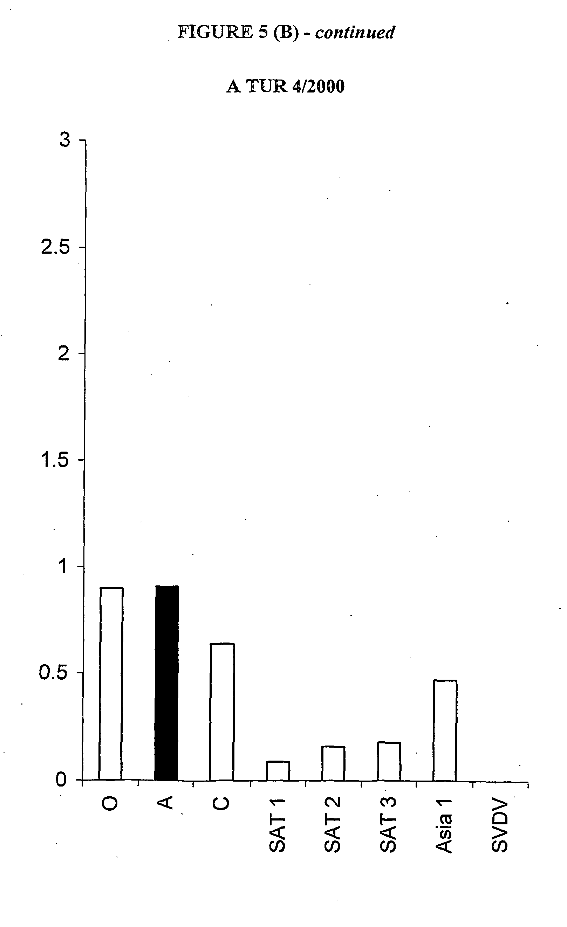

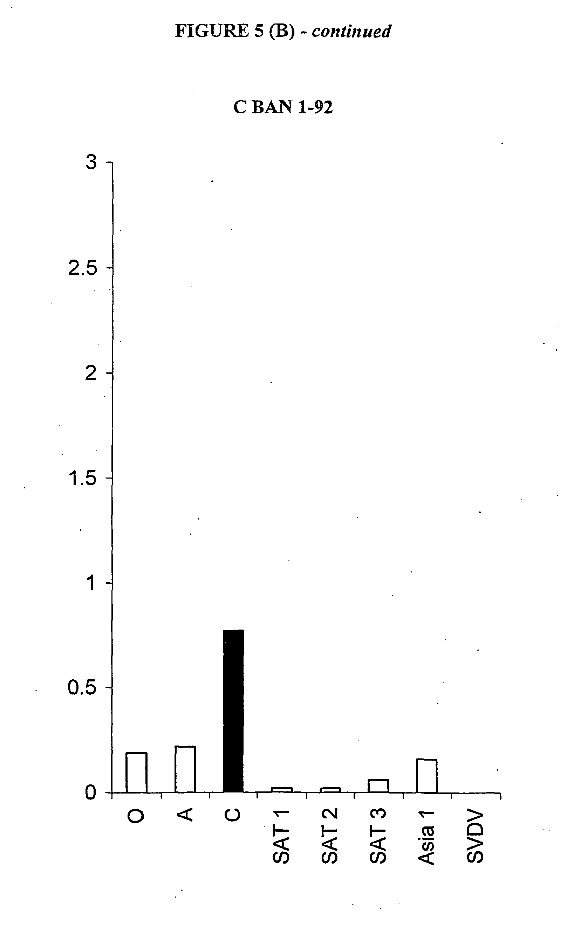

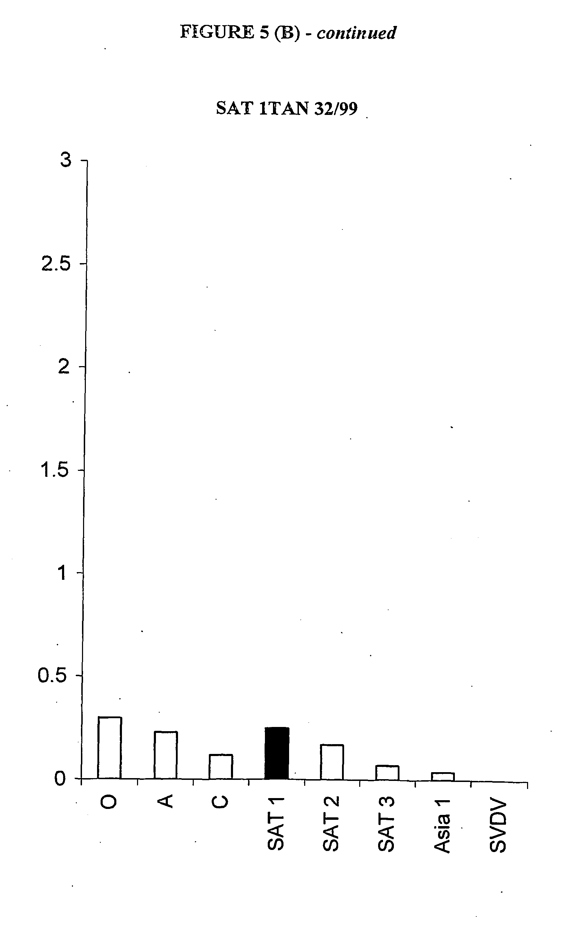

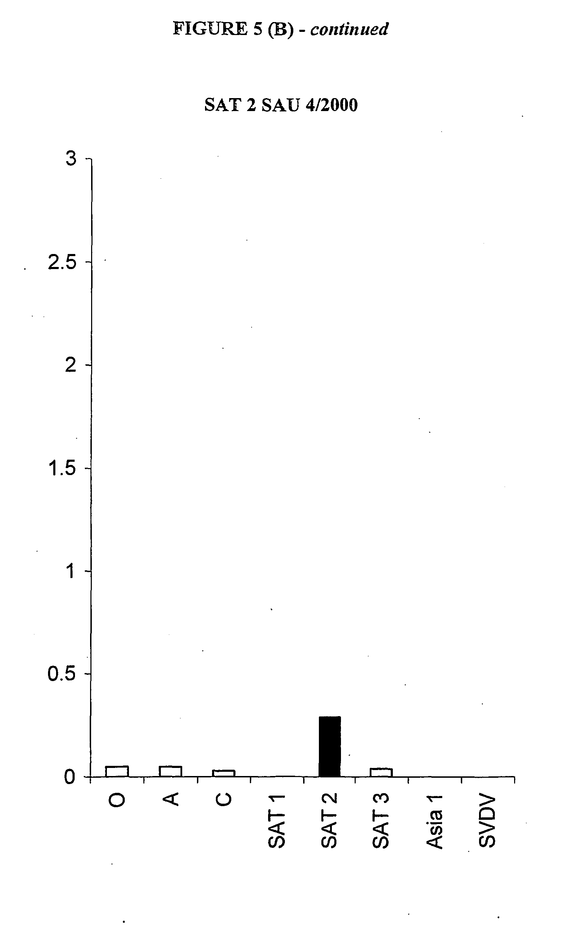

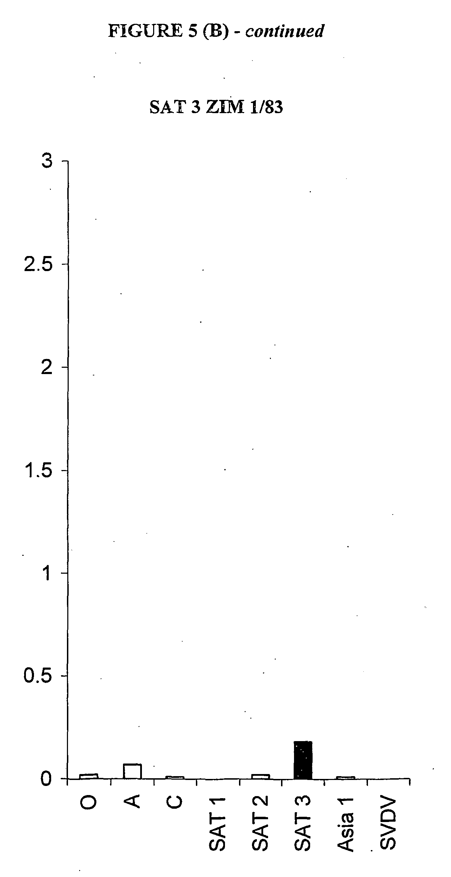

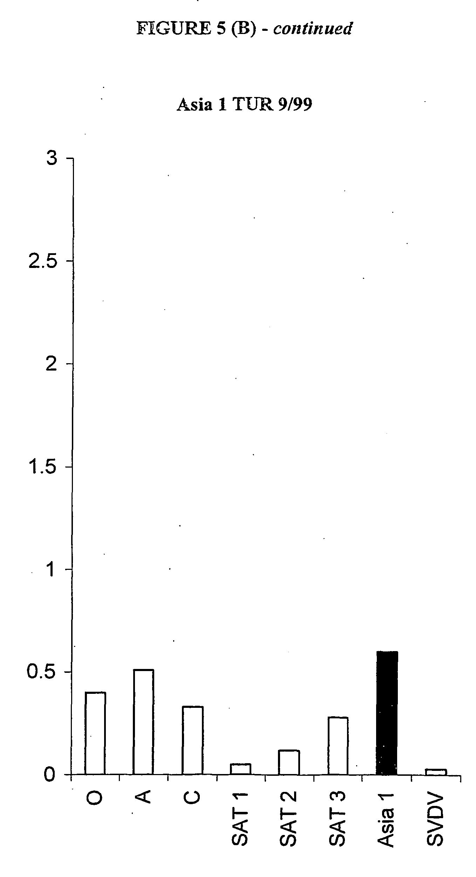

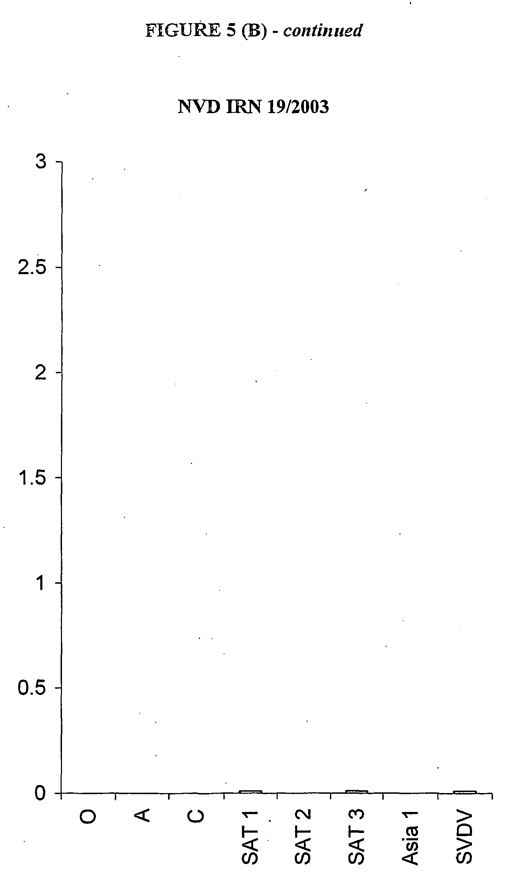

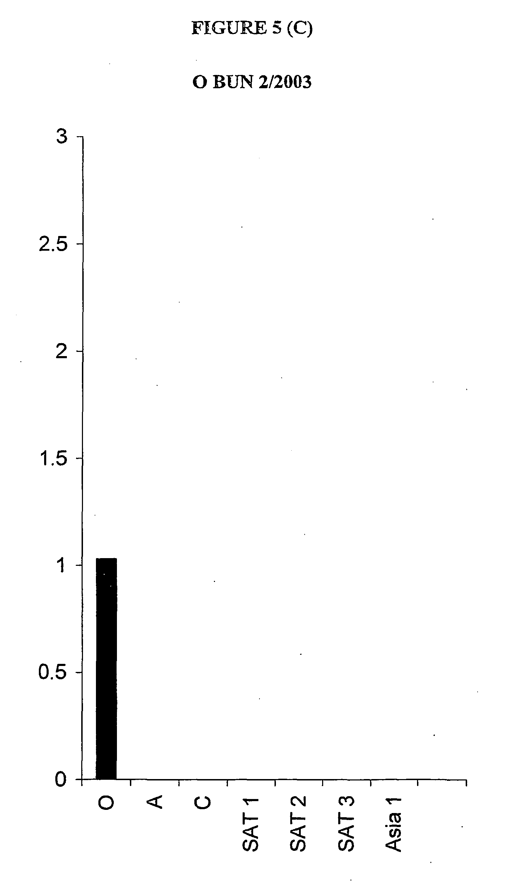

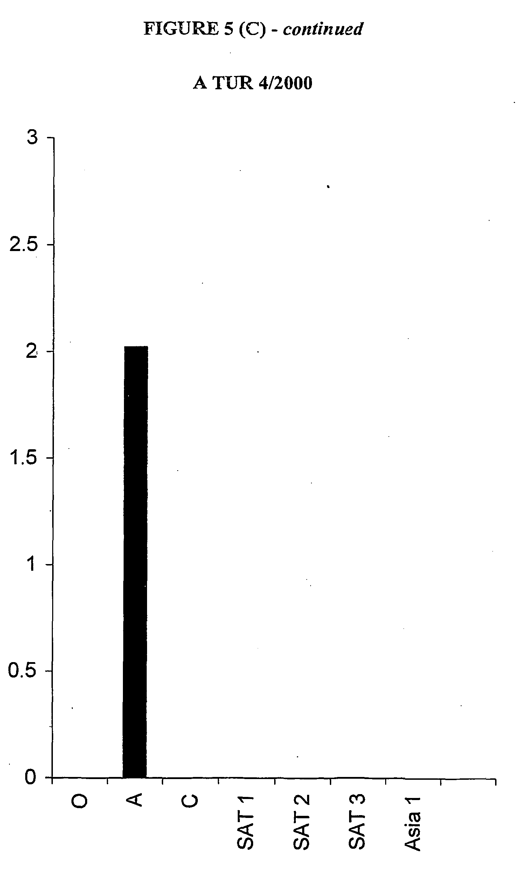

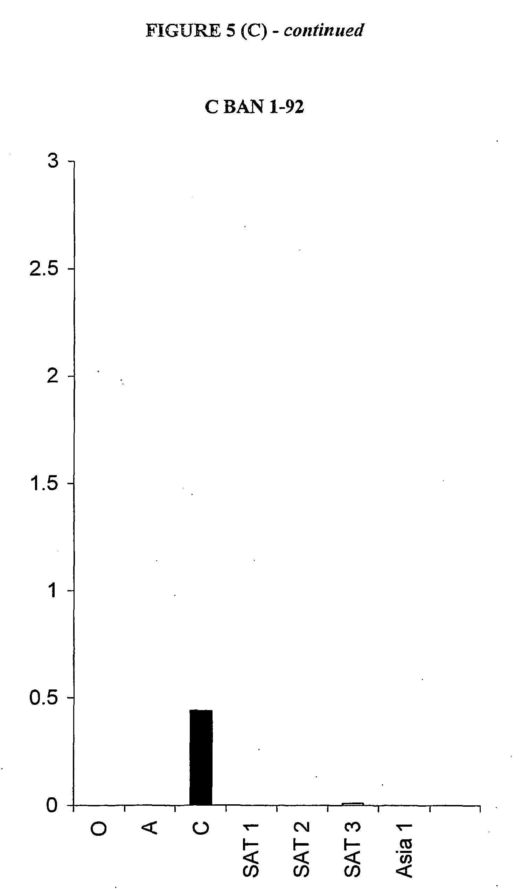

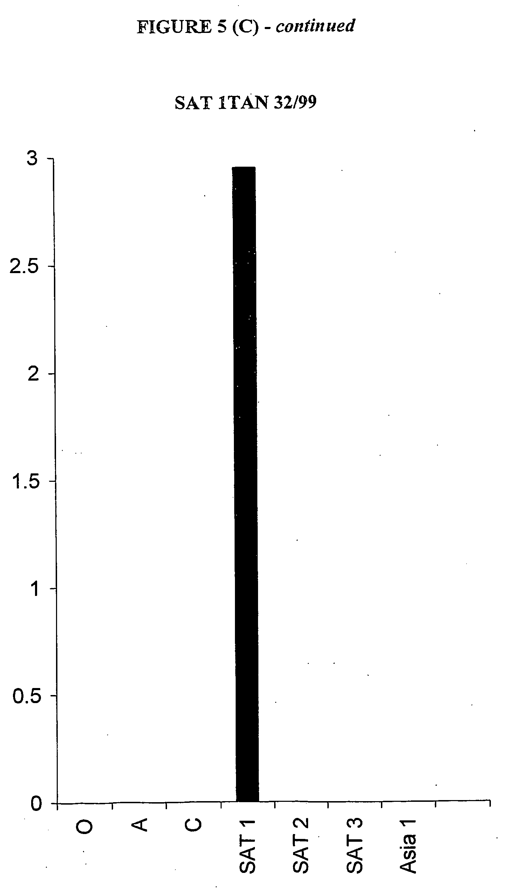

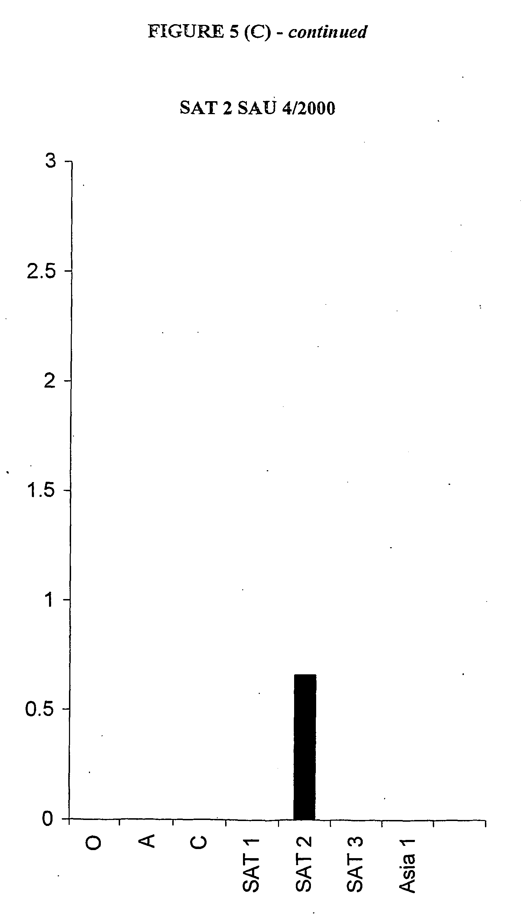

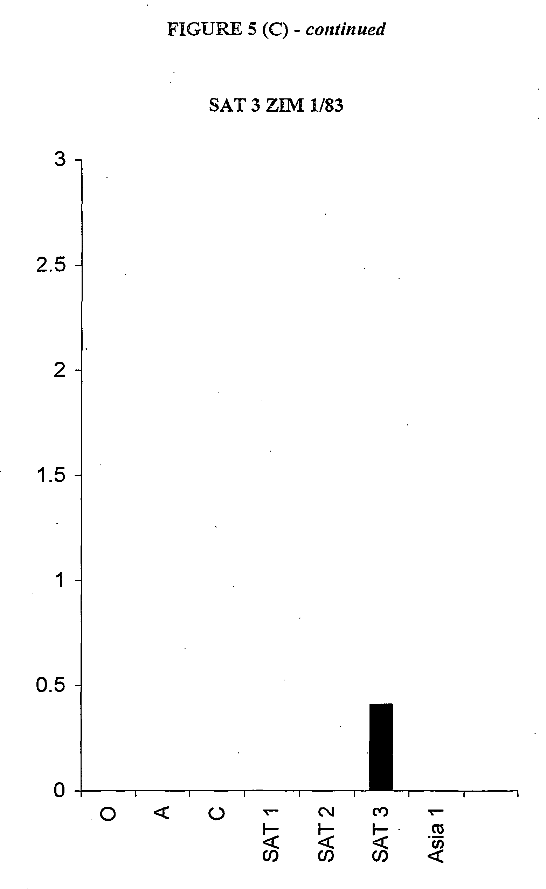

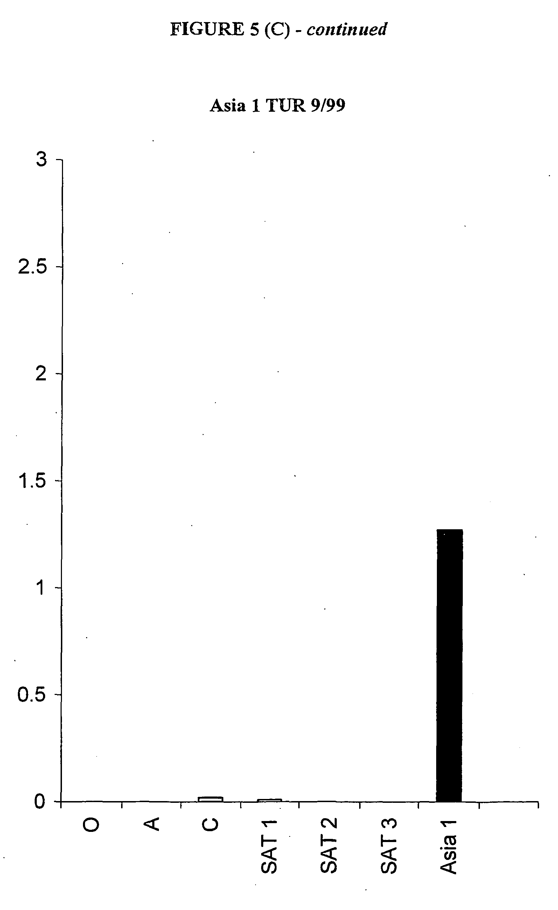

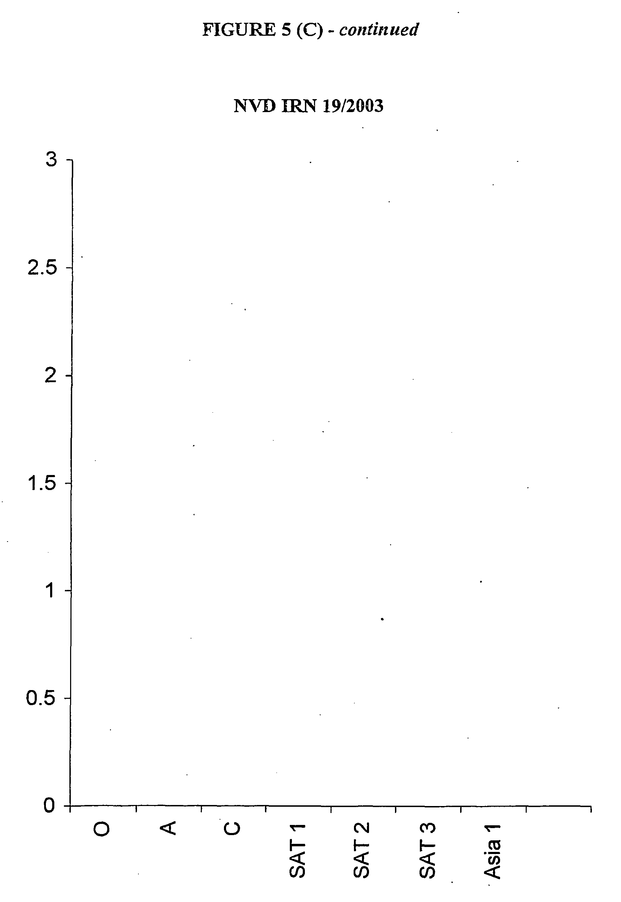

FIG. 5. Homo- and heterotypic reactivity of epithelial suspensions of each of the seven serotypes of FMD virus (serotype and strain as indicated by the graph headings) and a negative sample (NVD, no virus detected) using combinations of reagents for capture/detection of virus as follows. (A) Homotypic rabbit/guinea pig polyclonal antibodies. (B) Integrin/homotypic guinea pig polyclonal antibodies. (C) Integrin/homotypic monoclonal antibodies. Homotypic reactions as bold plots and heterotypic reactions as empty plots. Virus serotype (order on each *x*-axis: FMD virus types O, A, C, SAT1, SAT2, SAT3 and Asia 1 plus SVDV). In each case, the *y*-axis denotes optical density.
FIG. 6. Amino acid sequence of the porcine β6 subunit, and comparison with equivalent ovine, bovine, human, guinea pig and murine sequences.

### EXAMPLE

### Example 1: Cloning of the porcine B6 integrin subunit

The nucleotide sequence of porcine β6 was determined by reverse-transcription polymerase chain reaction. Briefly, template RNA was extracted from porcine tongue tissue using Trizol^{®} reagent (Invitrogen) according to manufacturer's instructions. cDNA was synthesised using annealed random hexamers (Promega), 10 µl of the prepared RNA and M-MLV reverse transcriptase (Invitrogen). The deign of two sets of PCR primers (5' fragment: 5'-ATG GGG ATT GAA CTG CTT TGC C-3' and 5'-CCT CAG ACC GCA GTT CTT CAT AAG-3' [**SEQ ID NO. 3**] and 3' fragment: 5'-CAT TCT CCA GCT GAT CAT CTC AG-3' and 5'-CAT AAA GTA GTT CTA TCC ATC CGT GG-3' [**SEQ ID NO. 4**]) used for the amplification of the entire coding sequence for porcine β6 was based on nucleotide similarities between the available sequences on GenBank. Fifty µL PCR reactions contained 2 mM MgCl₂ (Invitrogen), 50 mM KCl, 10 mM Tris-HCl pH 8.0 (Thermo Buffer, Invitrogen), 0.2 mM of each dNTP, 50 pmol of each primer, and 2.5 U *Taq* DNA polymerase (Invitrogen). Amplification conditions (MJ, GRI, Braintree, UK) were 94 °C for 60 seconds, 61 °C for 60 seconds, 72 °C for 120 seconds for 35 cycles. Chain elongation at 72 °C was extended to 7 minutes for the final cycle. PER products were separated by electrophoresis using 1.2 % agarose gels visualised with ethidium bromide. Amplicons of the correct size were excised from the agarose gels and purified (QIAquick gel extraction kit). These amplicons were cloned (pGEM-T easy, Promega), after which three independent clones containing the PCR inserts were sequenced (Beckman CEQ8000) and were assembled (DNAStar) as overlapping fragments.

### Example 2: Utility of recombinant integrin αvβ6 as a capture reagent in the detection of FMDV' by ELISA

### Materials and methods

### Preparation of recombinant αvβ6

Recombinant αvβ6 was produced from Chinese hamster ovary (CHO) cells stably transfected with αv and β6 genes of human origin (Weinacker et al., 1994) truncated by deletion of the C-terminal transmembrane and intracellular portions of both chains (residues 31-991 and 22-707 for αv (Swiss-Prot: P06756) and β6 (Swiss-Prot: P18564) respectively) to secrete αvβ6 as a soluble protein in serum-free cell culture media. The supernatant was clarified by filtration and stored at temperatures between -30 and 5°C. Initial studies were carried out with a highly purified recombinant αvβ6 to demonstrate that FMD viruses were bound by the integrin in the indirect sandwich ELISA. Soluble αvβ6 was initially precipitated by ammonium sulphate (60% w/v) and re-suspended in 0.05 M Tris HCl (pH 7.5), 0.05 M NaCl, 0.002 M CaCl₂ and 0.001 M MgCl₂ (alternatively. spin columns may be used in this initial concentration of the supernatant). The protein sample was applied to an anion-exchange column (HiTrap-Q, Amersham Pharmacia) and eluted using 0.05-0.5 M NaCl gradient (this step is optional). The collected fractions were checked on SDS-polyacrylamide gel electrophoresis to confirm the presence of the αvβ6 and subsequently concentrated using a 100kDa molecular weight cut-off concentrator (Vivaspin-6ml, Vivascience). The final steps of purification involved two consecutive size-exclusion columns. Sample was injected into a Superose 12HR 10/30 column (Amersham Pharmacia) connected to an AKTA FPLC device at a flow rate of 1ml/min. Dynamic light scattering (DynaPro) confirmed that αvβ6 integrin is monomeric in the solution. All purification processes were carried out at room temperature.

The initial ELISA experiments were replicated and subsequent experiments, on a range of virus preparations, were performed using the cell culture supernatant fluid arising from the propagation of transfected CHO cells without subsequent purification of the recombinant integrin.

### Polyclonal antibodies

Polyclonal antisera to FMD virus types O₁ BFS 1860, A₅/₂₂/₂₄ (Combination), C₃ Resende, SAT 1 BOT 1/68, SAT 2 ZIM 5/81, SAT 3 ZIM 4/81 and Asia 1 CAM 9/80 plus SVD virus UKG 27/72 (and which are routinely employed in the indirect sandwich ELISA for FMD/SVD virus antigen detection within the FAO World Reference Laboratory for Foot and Mouth Disease [WRL for FMD]) were used. These antisera had been raised in rabbits (Have et al., 1984, Acta Vet. Scant. 25:280-296) and guinea pigs (Ferris & Donaldson, 1984, Rev. Sci. Tech. Off. Int. Epiz. 3:563-574) by immunisation with inactivated, purified 146S (FMD) and 156S (SVD) virus particles in Freund's Complete and/or Incomplete Adjuvant.

### Monoclonal antibodies

Mouse monoclonal antibodies (mabs) to strains of each serotype of FMD and SVD virus were selected for test. The mabs to FMD virus serotypes C (strain C₁ Oberbayern, mabs E2B4 and D7G2), SAT 1 (BOT 1/68, Clone 5), SAT 3 (ZIM 4/81, C14) and Asia 1 (PAK 1/54, C1) plus SVD virus (UKG 27/72, C70) were produced in-house. Other mabs against the remaining FMD virus serotypes were gifts from other FMD laboratories : O (O₁ Lausanne, C9) from Dr E Brocchi, Istituto Zooprofilattico Sperimentale Della Lombardia E Dell'Emilia, Via A. Bianchi, 9 - 25124 Brescia. Italy; A (A₂₂ IRQ 24/64, 18H11) from Bayer AG, BG Tiergesundheit, Biologische Produktion, Osteratherstr 1A, D-50739 Koln, Germany and SAT 2 (ZIM 07/83, 810) from Dr D Fargeaud, Botswana Veterinary Institute, Broadhurst Industrial Estate, Private Bag 0031, Gaborone, Botswana). All these mabs have been shown to be type-specific (N.P. Ferris, unpublished results). Two other FMD virus mabs (types C, 4A3 and Asia 1, 5F10) were also obtained from Dr E Brocchi, Italy, both of which react with FMD viruses belonging to each of seven serotypes (E. Brocchi and N.P. Ferris, unpublished results).

### Virus sample preparation

Inactivated, purified antigens to each of the seven serotypes of FMD virus plus SVD virus (Ferris et al., 1984, Rev. Sci. Tech. Off Int. Epi .3:339-350) were used. Epithelial suspensions (ES) of reference samples of all seven serotypes of FMD virus plus SVD virus (as indicated in the figures) had previously been prepared in phosphate buffer (Ferris & Dawson, 1988, Vet. Microbiol. 16:201-209) and had been stored at temperatures between and - 90 and -50°C. Supernatant fluids derived from cell cultures (primary calf thyroid, IB-RS-2 or baby hamster kidney cells) inoculated with ES or cell culture grown antigens of all seven serotypes of FMD virus plus SVD virus (strains as indicated in the figures or as listed in Table 1) were selected from a collection similarly stored at temperatures between and -90 and -50°C.

### Blocking Buffers

Three different blocking buffers were employed depending on the particular ELISA (capture/detecting) reagent combination:

| | |
|---|---|
| Blocking Buffer 1: | PBS (pH 7.6) with 0.05% Tween 20 containing 5% skimmed milk powder ("Marvel") |
| Blocking Buffer 2: | Saline (0.85%) with 0.02 M Tris, 0.002 M CaCl₂ and 0.001 M MgCl₂, and 2% bovine serum albumen (pH 7.6) |
| Blocking Buffer 3: | PBS (pH 7.4) with 0.05% Tween 20 and containing 10% normal bovine serum and 5% normal rabbit serum |

### Indirect sandwich ELISA

The basic format for the indirect sandwich ELISA, unless otherwise stated, was as follows: 50 µl reagent volumes were used throughout; ELISA plates (Nunc Maxisorp immunoplates) were incubated for 1 h at temperatures between 35 to 39°C on a rotary shaker and plates washed with phosphate buffered saline [PBS, pH 7.4] after each incubation step, except the final stage in which sulphuric acid was added to stop the substrate/chromogen reaction.

### ELISA using polyclonal antisera as capture and detecting antibodies

Plates were coated with an optimal dilution of rabbit antiserum to FMD virus in 0.05 M carbonate/bicarbonate buffer, pH 9.6 and incubated in a fridge overnight at +1 to 8°C. Next either purified virus antigen, suspensions of vesicular epithelia or cell culture supernatants were added to each well. Where applicable, antigen was diluted in PBS. After plate incubation, homologous guinea pig antiserum, diluted to the optimal concentration in blocking buffer 1 was added to each well. After plate incubation, an optimal dilution of rabbit anti-guinea pig immunoglobulins conjugated to horse radish peroxidase in blocking buffer 1 was added to each well. The plates were washed after incubation and plates blotted dry before substrate (0.05% H₂O₂)/chromogen (orthophenylene diamine) in citrate/phosphate buffer, pH 5.0 was added. After 15 min incubation at room temperature the reaction was stopped by adding 1.25 M sulphuric acid. The OD of each well was read by using a spectrophotometer with a 492 nm filter.

### ELISA using recombinant αvβ6 as a capture ligand and polyclonal antibodies as detectors

Plates were coated with an optimal dilution of recombinant αvβ6 in a solution of 0.85% saline, 0.02 M Tris, 0.002 M CaCl₂ and 0.001 M MgCl₂, pH 7.6 and incubated in a fridge overnight at +1 to 8°C. The following day, the coating buffer was tipped off the plate without washing with PBS and 100 µl of blocking buffer 2 was added to every well. After plate incubation for 1 h at 37°C, the blocking buffer was simply tipped off the plate, test sample added and the ELISA completed as previously described in the previous section. Where applicable, antigen was diluted in blocking buffer

### ELISA using recombinant αvβ6 as a capture ligand and monoclonal antibodies as detectors

Plates were coated with recombinant αvβ6, wells blocked with blocking buffer 2. and test sample added. Where applicable, antigen was diluted in blocking buffer 2. After plate incubation, an optimum dilution of mab in blocking buffer 3 was added and the ELISA completed as described, except that rabbit anti-mouse, instead of rabbit anti-guinea pig, immunoglobulins conjugated to horse radish peroxidase, and diluted in blocking buffer 3, was used.

### Optimisation of recombinant αvβ6 dilution for use in the ELISA

The optimal dilution of recombinant αvβ6 to use to coat immunoplates was assessed as follows. Two-fold dilution series of the integrin in saline coating buffer were made (from columns 1 to 12 of immunoplates) from either a starting concentration of 10 µg/ml of purified recombinant or undiluted CHO cell culture supernatant fluid. A fixed dilution (1 µg/ml) of purified antigen of each FMD virus serotype plus SVD virus was then added, one virus serotype for each of the 8 rows, and the test run to completion. The optimal dilution of each recombinant preparation was then chosen from the titration plot of the reactivity of recombinant dilution against the virus antigens.

### Evaluation of recombinant αvβ6 as a ligand for FMD virus

The ability of the recombinant αvβ6 to bind FMD virus was assessed by comparing a series of immunoplates which had been coated with an optimal concentration of unpurified recombinant integrin with others which had been left uncoated. The plates were blocked after 'incubation' with blocking buffer 2 prior to the addition of cell culture supernatants to 12 isolates of each FMD virus serotype plus SVD virus (Table 2). The two pan-reactive mabs (4A3 and 3F10) were used as detecting antibodies and the format of the ELISA was carried out as previously described for recombinant αvβ6 as a capture ligand and mabs as detectors. The results were also compared to the reactivity of the antigens in the described ELISA procedures using rabbit antiserum as a trapping reagent in combination with either guinea pig polyclonal or mouse monoclonal antibodies.

### FMD virus diagnosis by ELISA

The three strategies for the ELISA (*i.e*. rabbit polyclonal antibody as trapper and guinea pig polyclonal antibody as detector, recombinant αvβ6 as trapper/guinea pig as detector and recombinant αvβ6 as trapper/type-specific mab as detector) were compared for their ability to serotypically discriminate samples of cell culture grown antigens and epithelial suspensions of each serotype of FMD and SVD virus (plus another suspension negative for FMD or SVD virus).

**TABLE 2. FMD and SVD virus strains used to evaluate the capacity of recombinant αvβ6 to act as a ligand**

| **Virus sample no.** | **FMD virus serotype** | | | | | | | **SVD virus** |
|---|---|---|---|---|---|---|---|---|
| | **O** | **A** | **C** | **SAT 1** | **SAT 2** | **SAT 3** | **Asia 1** | |
| 1 | BUR 1/89 | BHU 2/90 | ITL 2/89 | SA 13/61 | IVY 2/90 | BOT 109/66 | ISR 3/89 | UKG 314/73 |
| 2 | KEN 102/60 | BRA 1/58 | C₃ Indaial | ZAM 3/88 | TAN 5/68 | RHO 1/74 | BUR 1/88 | NET 3/92 |
| 3 | BHU 1/90 | TUR 1/90 | NEP 35/96 | ZAM 2/88 | ZIM 19/89 | RV 7/34 | IND 5/89 | MTA 22/75 |
| 4 | SUD 3/89 | BUN 4/90 | BAN 1/92 | TUR 323/62 | GHA 2/90 | KNP 1/86 | OMN 21/89 | AUR 1/73 |
| 5 | ISR 1/90 | KEN 46/65 | PHI 9/94 | ISR 4/62 | ZAM 6/82 | RHO 4/75 | NEP 2/90 | HKN 1/89 |
| 6 | CAR 3/89 | USSR 1/64 | C 997 | SWA 40/61 | SEN 1/83 | MAL 3/76 | CAM 1/90 | GRE 1/79 |
| 7 | CAM 1/89 | NEP 30/90 | C ARG 69 | YEM 15/84 | ZIM 12/91 | UGA 92/70 | ISR 3/63 | FRA 2/73 |
| 8 | IND 1/89 | Peru 69 | C₁ Noville | ZIM 22/89 | BOT 11/77 | ZIM 2/84 | MAY 2/90 | BEL 2/79 |
| 9 | HKN 14/90 | A Zambia | C Pando | ZAM 2/92 | UGA 3/91 | P26/90 HV5 | TUR 11/2000 | ITL A/89 |
| 10 | TUN 6/89 | A₃₁ COL 69 | IND 51/79 | KNP 1/88 | UGA 5/70 | P27/90 DSA39 | MYA 2/97 | HKN 1/80 |
| 11 | ETH 3/90 | KEN 3/64 | SAU 9/74 | P30/90 Cher30 | CIV 5/90 | P20/90 Cher32 | IRN 15/2001 | HKN 1/82 |
| 12 | JOR 1/90 | NYE 8/89 | C₁ Oberbayern | KNP 6/88 | KEN 8/91 | ZIM 4/83 | LAO 1/96 | HKN 1/76 |

### Results

### Polyacrylamide gel electrophoresis

Fig. 1 shows a photograph of a polyacrylamide gel electrophoresis, which was carried out on two concentrations of CHO cell culture supernatant fluid. The analysis of the materials show two clear bands which correspond to the expected sizes of 116 and 92 kDA (the molecular weights of recombinant αv and β6 being 128.3 and 87 kDA respectively), confirming the presence of αv and β6 in the supernatant.

### Optimal recombinant αvβ6 dilution for use in the ELISA

It was found that the purified αvβ6 protein preparation bound the inactivated, purified viruses of all seven FMD virus serotypes but did not react with SVD virus. The results of performing titrations of the purified integrin indicated that 1 µg/ml was an optimum concentration for coating plates (results not shown). This experiment was next repeated using unpurified CHO cell culture supernatant fluid and the results of titrations of this recombinant preparation against purified virus preparations are shown in Fig. 2A. The plots illustrate that the soluble αvβ6 duplicated the reaction of the purified preparation in binding purified FMD viruses but not SVD virus. Too high an integrin concentration proved to be inhibitory for binding virus and reactivity rapidly tailed off past an integrin dilution of 1:512 but the mid-range of the dilution series (from dilutions 1:16 to 1:256) proved effective for trapping FMD virus. Consequently, a dilution of 1:100 of the CHO cell culture supernatant fluid was chosen to coat immunoplates for subsequent examination of test antigen preparations in the ELISA.

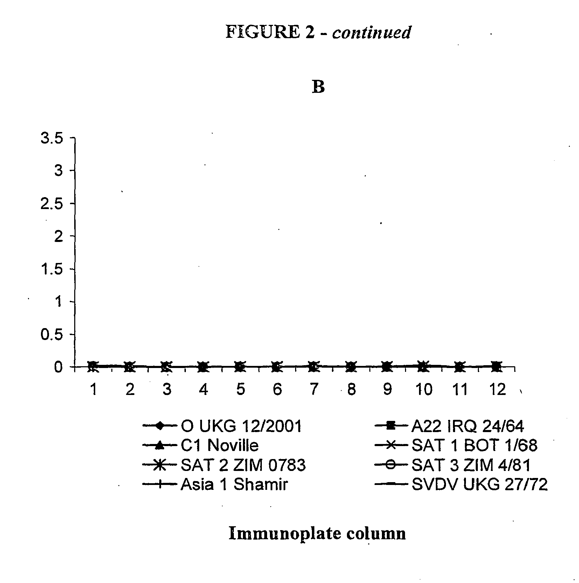

The conclusion that FMD virus was indeed bound by the recombinant was supported by the demonstration that virus preparations were prevented from binding directly to the surface of immunoplate wells treated with blocking buffer 2 prior to the antigen step (Fig. 2B). However, this treatment did not interfere with the positive reaction between integrin and FMD virus on integrin coated plates.

### Evaluation of recombinant αvβ6 as a ligand for FMD virus

The reaction of 12, cell culture grown antigens of each FMD virus serotype and SVD virus (Table 2) was evaluated in each ELISA format and the results are shown in Fig. 3 (each mini graph is entitled with the serotype of each group of 12 viruses used for the ELISA reaction). The results show that the integrin bound all FMD viruses irrespective of serotype (the plot profiles being similar between Fig. 3 sections A, B, C, E and F) but not SVD virus. The specificity of the reactions being confirmed by the inability of the viruses to bind directly to the surface of immunoplates uncoated with either rabbit antiserum or integrin but which had been first 'blocked' with blocking buffer 2 (Fig. 3D). SVD virus was bound and detected by type-specific rabbit and using either guinea pig polyclonal antisera (Fig. 3A) or monoclonal antibody C70 as detectors (results not shown). However, SVD virus was not bound by the integrin (results not shown).

### Serotypic characterisation of test samples

The results of comparisons between the three ELISA formats to classify the serotype of the test samples are shown in Fig. 4 (using cell culture grown antigens) and Fig. 5 (using epithelial suspensions). Each mini graph is entitled with the virus serotype and sample strain used for the ELISA reaction.

Although the signals for the homotypic reactions were strongest, there was evidence of a degree of heterotypic cross-reactivity with some of the test samples using rabbit/guinea pig polyclonal antisera (Fig. 4A and 5A). This heterotypic cross-reactivity was further and considerably exacerbated using the integrin/guinea pig polyclonal antibody combination (Fig. 4B and 5B). Conversely, totally type-specific reactions resulted from the ELISA employing recombinant integrin as capture and monoclonal as detecting reagents (Fig. 4C and 5C). No reactions were evident when utilising integrin as the trapping reagent with the addition of SVD virus or the negative epithelial suspension.

### Discussion

Molecular techniques, such as RT-PCR procedures, are certain to play an essential role in FMD and other vesicular disease diagnosis in the near future. The real-time, fluorogenic RT-PCR is now being shown to be more sensitive than the ELISA for FMD diagnosis (Reid et al., 2003, J. Virol. Methods 107:129-139; Shaw et al., 2004, Rev. Sci. Tech. Off. Int. Epiz. 23 [in press]). It has the potential to examine certain diagnostic samples such as blood, milk and other fluids, which cannot be examined directly in the ELISA, and to reduce the necessity for virus isolation and amplification in cell cultures.. It will also reduce the necessity for culturing test samples in sensitive cells in attempts to isolate and amplify virus both to demonstrate positivity and to confirm negativity of clinical material. However, it is equally certain that there is still a role for the ELISA and that it will remain widely used for diagnosis.

Although the assay has inferior sensitivity to real-time RT-PCR, it can often be high. For example, around 90% of positive epithelium samples received during the course of the 2001 FMD outbreak in the UK were so defined at the initial stage of testing epithelial suspensions (N.P. Ferris, unpublished results). The ELISA is quicker and easier to perform than the PCR and so a positive result can be achieved in a shorter time-scale. Additionally, a negative ELISA result on a vesicular epithelial suspension is neither a final classification of the test sample nor a final, reportable result. RT-PCR equipment (*e.g*. robotic workstations, PCR plate readers etc) can be very expensive and the cost of consumables, kits and reagents is high, features which may either deter or prevent certain FMD laboratories around the world with minimal financial support from using the RT-PCR. The ELISA is an assay, which is readily transferable from a reference laboratory to other FMD laboratories, the majority of which already have the necessary equipment and reagents for its use and the expertise to perform it.

The main disadvantage of the ELISA as used by the WRL for FMD and the majority of other FMD laboratories (besides not having 100% sensitivity) is that it uses FMD virus type-specific polyclonal antisera : rabbit antibodies to trap FMD virus onto the plate and guinea pig antibodies for its detection. The disadvantages of polyclonal antisera include a continual need to ensure that reagents for diagnostic use have suitable affinity for new emerging field virus strains and that each stock is of finite supply, while the replacement stock often exhibits slightly different reaction characteristics. To counter the finite supply of polyclonal antiserum reagents, type-specific mabs can be used (Brocchi *et al.,* 1986, *Report of the Session of the Research Group of the Standing Technical Committee of the European Commission for the Control of Foot-and-Mouth Disease,* Madrid, Spain, 14-17 October 1986, Appendix 5:30-31) but the problem of ensuring that the selection of panels of either individual mabs, or cocktails of several, is suitable for the recognition and serotypic discrimination of new antigenic virus strains remains.

The objective of the work reported on here was to examine the utility of recombinant integrin αvβ6 (arising from CHO cells, which have been transfected to secrete soluble integrin into the maintenance medium) for binding strains of all serotypes of FMD virus in the ELISA. This was demonstrated, initially by testing purified FMD and SVD virus antigens in an ELISA using purified recombinant integrin protein as the capture reagent. These results were next replicated by using the serum-free maintenance medium from the transfected CHO cells as collected directly from the culture flasks (save for a clarification of the medium to remove cell debris by centrifugation). This was followed by the demonstration that panels of cell culture grown antigens of each FMD virus serotype were bound by the recombinant protein while those of SVD virus were not. Although there were examples of variable optical density values for certain viruses between the different ELISA formats, the difference in signals for the majority was probably a reflection of the reactivity of the reagent used to detect the bound virus. For example, although the pan-reactive FMD mabs 4A3 and 5F10 react with FMD viruses of each serotype, mab 4A3 had a higher affinity for SAT 1 FMD viruses than mab 5F10 while the reverse was evident for FMD viruses of the Asia 1 serotype.

A limitation of the conventional ELISA (using rabbit antisera as capture antibodies and polyclonal antibodies as detectors) is that of heterotypic cross-reactions and which presents a problem for defining the serotype of the virus strain with total certainty. Such heterotypic reactions were evident from the described experiments on serotyping both cell culture grown viruses and epithelial suspensions of field strains of FMD viruses (as illustrated in Figs 4 and 5). Homotypic signals were the highest for each of the viruses examined by the ELISA using rabbit/guinea pig antibody combination with a reasonably clear distinction from the heterotypic reaction (Figs 4A and 5A). This proves to be generally the case during the course of examining submitted diagnostic samples received under the auspices of the WRL for FMD. However, problems of test interpretation occur in two situations. Firstly, high concentrations of antigens in sample preparations and which have a poor antigenic match with the typing reagent, often give rise to a lower optical density value in the ELISA against the homotypic reagent than would normally be expected. Consequently, the differences between homotypic and heterotypic reaction signals are reduced, lowering the confidence with which the serotype of the FMD virus is defined. Secondly, it is normal to assume that heterotypic reactions are indeed non-specific cross-reactions. However, this can be a dangerous assumption. Multiple-infected samples are uncommon but they do occasionally occur and their classification by ELISA, with or without virus isolation in cell culture, is problematic (Ferris et al., 1995, Rev. sci. tech. Off. Int. Epiz. 14:557-565). The combination of integrin as capture and guinea pig polyclonal antibody as detector yielded even greater heterotypic cross-reactions (Figs 4B and 5B). This was disappointing and somewhat surprising but illustrates that the integrin is not specific solely for FMD virus and that the guinea pig anti-146S sera are not as FMD virus type-specific as previously thought or hoped for. However, replacing the polyclonal reagents with mabs eliminated heterotypic reactions and yielded totally FMD virus type-specific results of the correct definition. This is not to suggest that the particular mabs used in this study are necessarily the optimum ones for routine diagnostic use (some are almost certainly not). Rather that the results show that the use of the recombinant protein as a capture reagent in the ELISA in combination with appropriate mab(s) as the detector has the potential to improve upon conventional diagnostic assays using rabbit and guinea pig polyclonal antisera.

There is further potential for use of the recombinant integrin αvβ6 protein in other FMD virus test procedures where the use of a single reagent to recognise FMD viruses of all serotypes could be advantageously exploited, *e.g*. in immunocapture RT-PCR, pen-side chromatographic strip-test devices and other biosensors. Additionally, the recombinant could be usefully employed in any immunoassays employed for FMD diagnosis. Those designed for characterising the antigenicity of field virus strains (Kitching et al., 1988, Vaccine 6:403-408; Samuel et al., 1991, Biologicals 19:299-310) and for detecting FMD virus antibody (Mackay et al., 2001, J. Virol. Methods 97:33-48) come to mind. These assays commonly use type-specific rabbit polyclonal antibody to trap virus onto the immunoplate, the efficiency of which is limited by the antiserum selection.

### Example 3: Utility of recombinant integrin αvβ6 as a capture reagent in the detection of FMDV by chromatographic strip test

Chromatographic strip test devices may be made following procedures described by Brüning et al., 1999, J. Virol. Methods 81: 143-154 and Reid et al., 2001, J. Virol. Methods 96: 189-202.

Firstly, the optimal concentration of purified integrin on microparticles is established. Carboxy-modified latex particles coloured with a dye is washed once in 2-morpholinoethanesulphuric acid buffer, pH 5.5, at room temperature prior to inactivation by water-soluble carbodiimide 1-ethyl-3-(-3-dimethylaminopropyl)-carbodiimide and integrin added to the activated microparticles and incubated at room temperature for 2 hours. The microparticles are then washed three times with Tris-BSA buffer, pH 8.0 and stored at 1 to 8°C. A pan-reactive FMDV monoclonal antibody and a monoclonal antibody to either the integrin β6 chain or the αvβ6 heterodimer (commercially available from, for example, Chemicon Europe Ltd, The Science Centre, Eagle Close, Chandlers Ford, Hampshire S053 4NF; Cat nos MAB2075Z [recognises β6 chain], 2074Z or 2077Z [both recognise avb6 heterodimer]) is applied to the nitrocellulose membrane using Bio-Dot air-brush equipment. The pan-reactive FMDV monoclonal antibody is applied to the test line and the anti-integrin β6 or αvβ6 is applied to the control band. The membrane is dried at 37°C for 45 min and stored in sealed foil sachets until use.

The latex conjugate is applied to glass-fibre filters (Whatman) using Bio-Dot air-brush equipment (BioDot). The glass-fibre filters is then dried at room temperature for 45 min and stored in foil sachets until required.

The glass-fibre filter is overlaid onto the base of the nitrocellulose membrane, parallel to the control and test antibody bands, stuck to the membrane with adhesive and cut into 0.8 cm wide strips. The glass-fibre filter/membrane strips are then assembled into a prototype device so that the glass-fibre constitutes the sample pad, situated above the nitrocellulose membrane strip.

Epithelial suspensions of FMDV and other viruses (and supernatant fluids arising from sensitive cell cultures inoculated with these suspensions) from current and historical field samples which have been submitted to the laboratory are evaluated for their reaction in prototype strip-test devices. The test principle of the prototype chromatographic strip device is that upon addition of test sample to the sample pad, the air-dried integrin-labelled microparticles will be re-hydrated and will move by capillary action along the nitrocellulose strip (positioned below the pad) towards the immobilised band of trapping monoclonal antibody to FMDV. If FMD viral antigen is present in the test sample, it will bind to the integrin attached to the microparticles and the whole complex will be captured by the immobilised band of antibody on the nitrocellulose. This results in the accumulation of the dye microparticles, which appear as a coloured line in the sample window (at the test line) to signify a positive result. Excess integrin-labelled microparticles continue to migrate along the nitrocellulose to reach the band of immobilised anti-integrin monoclonal antibody and result in a second coloured line in the control window to act as an internal control for test validation.

An alternative strategy is to add the FMDV pan-reactive monoclonal antibody to the dye-coloured latex microparticles and apply the latex conjugate to the glass-fibre filters, the integrin to the nitrocellulose membrane at the test band and rabbit anti-mouse antibody to the nitrocellulose membrane at the control band.

## Claims

1. A method for determining whether a sample contains a target microbial agent comprising:
(a) contacting the sample with an αvβ6 integrin under conditions that allow the target microbial agent, if present in the sample, to bind to the integrin; and
(b) determining whether the integrin has any target microbial agent bound thereto.

2. A method according to Claim 1 wherein the method comprises a first step of contacting the sample with a capture ligand having affinity for the target microbial agent and a subsequent second step of determining whether the capture ligand has any target microbial agent bound thereto using a detection ligand having affinity for the target microbial agent, wherein either the capture ligand or the detection ligand is an αvβ6 integrin.

3. A method according to Claim 2 wherein the capture ligand or detection ligand comprises an antibody having specificity for the target microbial agent or antigen-binding fragment thereof

4. A method for determining whether a sample contains antibodies to a target microbial agent comprising:
(a) contacting the sample with a specimen of the target microbial agent under conditions that allow anti-target microbial agent antibodies, if present in the sample, to bind to the target microbial agent;
(b) contacting the target microbial agent specimen with an immobilised capture ligand having affinity for the target microbial agent;
(c) contacting the immobilised target microbial agent specimen with a competitor ligand having affinity for the target microbial agent; and
(d) determining whether the immobilised target microbial agent specimen has any competitor ligand bound thereto
wherein step (b) may be performed either before or after step (a) wherein either the capture ligand or the competitor ligand is an αvβ6 integrin
and wherein the amount of the competitor ligand bound to the target microbial agent specimen provides an indication of the presence of antibodies to a target microbial agent in the sample.

5. A method according to Claim 4 wherein the capture ligand or competitor ligand comprises an antibody or antigen-binding fragment thereof.

6. A method according to any one of Claims 1 to 5 wherein the target microbial agent is foot-and-mouth disease virus (FMDV).

7. A method according to any one of the preceding claims wherein the αvβ6 integrin comprises a naturally occurring αv subunit and/or a naturally occurring β6 subunit.

8. A method according to any one of Claims 1 to 7 wherein the αvβ6 integrin immobilised.

9. A method according to Claim 1 or 4 wherein sep (b) is performed by ELISA.

10. A method according to Claim 1 further comprising step (c) of characterising the virus strain detected in step (b).

11. A method according to Claim 10 wherein step (c) comprises characterisation by RT-PCR or ELISA

12. A kit of parts for performing a method according to any one of the preceding claims comprising an αvβ6 integrin or cells capable of expressing αvβ6 integrin when grown in culture or vectors comprising a nucleotide sequence which encodes αvβ6 integrin,
wherein the kit further comprises a substrate upon which the integrin is or can be immobilised.

13. A kit according to any one of Claims 12 further comprising means for detecting a target microbial agent.

14. A kit according to Claim 13 wherein, the means for detecting the target microbial agent is an anti-target microbial agent antibody.

15. A kit according to any one of Claims 12 to 14 further comprising an immunoconjugate comprising the antigen-binding domain of an antibody with specificity for the anti-target microbial agent antibody fused to a directly or indirectly detectable label.

16. A kit according to any one of Claims 12 to 15 further comprising one or more reagents for performing an ELISA.

17. Use of an αvβ6 integrin to detect a target microbial agent in a sample.

18. Use of an αvβ6 integrin in the in vitro diagnosis of infection with a target microbial agent.

19. Use of an αvβ6 integrin to purify or concentrate a target microbial agent from a sample.

20. A use according to Claim 17, 18 or 19 wherein the target microbial agent is foot-and-mouth disease virus (FMDV).

21. A use according to any one of Claims 17 to 20 in an ELISA.

22. A use according to any one of Claims 12 to 21 wherein the αvβ6 integrin comprises a naturally occurring αv subunit and/or a naturally occurring β6 subunit.

23. The use according to 17 wherein the αvβ6 integrin is immobilised.

24. An isolated polypeptide comprising or consisting of the amino acid sequence of SEQ ID NO. 1.

25. An isolated complex comprising a polypeptide according to Claim 24 and an αv integrin subunit.

26. A complex according to Claim 25 wherein the complex is immobilised.

27. An isolated polynucleotide encoding a polypeptide according to Claim 24.

28. A polynucleotide according to Claim 27 wherein the polynucleotide comprises or consists of the nucleotide sequence of SEQ ID NO 2.

## Patentansprüche

1. Verfahren zur Bestimmung, ob eine Probe ein mikrobielles Zielobjekt enthält, umfassend:
(a) Inkontaktbringen der Probe mit einem αvβ6-Integrin unter Bedingungen, die ein Binden des mikrobiellen zielobjekts, falls es in der Probe vorhanden ist, an das Integrin ermöglichen; und
(b) Bestimmen, ob das Integrin ein daran gebundenes mikrobielles Zielobjekt aufweist.

2. Verfahren nach Anspruch 1, wobei das Verfahren eine erste Stufe des Inkontaktbringens der Probe mit einem Fangliganden, der Affinität für das mikrobielle Zielobjekt aufweist, und eine nachfolgende zweite Stufe des Bestimmens, ob der Fangligand ein daran gebundenes mikrobielles zielobjekt aufweist, unter Verwendung eines Detektionsliganden, der Affinität für das mikrobielle Zielobjekt aufweist, umfasst, wobei entweder der Fangligand oder der Detektionsligand ein αvβ6-Integrin ist.

3. Verfahren nach Anspruch 2, wobei der Fangligand oder der Detektionsligand einen Antikörper, der Spezifität für das mikrobielle Zielobjekt aufweist, oder ein antigenbindendes Fragment desselben umfasst.

4. Verfahren zur Bestimmung, ob eine Probe Antikörper zu einem mikrobiellen Zielobjekt enthält, umfassend:
(a) Inkontaktbringen der Probe mit einem Prüfling des mikrobiellen Zielobjekts unter Bedingungen, die ein Binden von Antikörpern gegen das mikrobielle Zielobjekt, falls diese in der Probe vorhanden sind, an das mikrobielle Zielobjekt ermöglichen;
(b) Inkontaktbringen des Prüflings des mikrobiellen Zielobjekts mit einem immobilisierten Fangliganden, der Affinität für das mikrobielle Zielobjekt aufweist;
(c) Inkontaktbringen des immobilisierten Prüflings des mikrobiellen Zielobjekts mit einem konkurrierenden Liganden, der Affinität für das mikrobielle Zielobjekt aufweist; und
(d) Bestimmen, ob der immobilisierte Prüfling des mikrobiellen Zielobjekts einen daran gebundenen konkurrierenden Liganden aufweist,
wobei die Stufe (b) entweder vor oder nach der Stufe (a) durchgeführt werden kann,
wobei entweder der Fangligand oder der konkurrierende Ligand ein αvβ6-Integrin ist,
und wobei die Menge des an den Prüfling des mikrobiellen Zielobjekts gebundenen konkurrierenden Liganden eine Anzeige für das Vorhandensein von Antikörpern zu einem mikrobiellen Zielobjekt in der Probe liefert.

5. Verfahren nach Anspruch 4, wobei der Fangligand oder der konkurrierende Ligand einen Antikörper oder ein antigenbindendes Fragment desselben umfasst.

6. Verfahren nach einem der Ansprüche 1 bis 5, wobei das mikrobielle Zielobjekt das Maul- und Klauenseuche-Virus (MKSV) ist.

7. Verfahren nach einem der vorhergehenden Ansprüche, wobei das αvβ6-Integrin eine natürlich vorkommende αv-Untereinheit und/oder eine natürlich vorkommende β6-Untereinheit umfasst.

8. Verfahren nach einem der Ansprüche 1 bis 7, wobei das αvβ6-Integrin immobilisiert ist.

9. Verfahren nach Anspruch 1 oder 4, wobei die Stufe (b) durch ELISA durchgeführt wird.

10. Verfahren nach Anspruch 1, das ferner die Stufe (c) der Charakterisierung des in Stufe (b) detektierten Virusstamms umfasst.

11. Verfahren nach Anspruch 10, wobei die Stufe (c) eine Charakterisierung durch RT-PCR oder ELISA umfasst.

12. Kit aus Teilen zur Durchführung eines Verfahrens nach einem der vorhergehenden Ansprüche, wobei das Kit ein αvβ6-Integrin oder Zellen, die, wenn sie in einer Kultur gezüchtet werden, αvβ6-Integrin exprimieren können, oder Vektoren, die eine αvβ6-Integrin codierende Nucleotidsequenz umfassen, umfasst, wobei das Kit ferner ein Substrat umfasst, auf dem das Integrin immobilisiert ist oder immobilisiert werden kann.

13. Kit nach Anspruch 12, das ferner Mittel zum Detektieren eines mikrobiellen Zielobjekts umfasst.

14. Kit nach Anspruch 13, wobei das Mittel zum Detektieren des mikrobiellen Zielobjekts ein Antikörper gegen das mikrobielle Zielobjekt ist.

15. Kit nach einem der Ansprüche 12 bis 14, das ferner ein Immunkonjugat umfasst, das die antigenbindende Domäne eines Antikörpers mit Spezifität für den Antikörper gegen das mikrobielle Zielobjekt an eine direkt oder indirekt detektierbare Markierung gebunden umfasst.

16. Kit nach einem der Ansprüche 12 bis 15, das ferner ein oder mehrere Reagenzien zur Durchführung eines ELISA umfasst.

17. Verwendung eines αvβ6-Integrins zum Detektieren eines mikrobiellen Zielobjekts in einer Probe.

18. Verwendung eines αvβ6-Integrins bei der In-vitro-Diagnose einer Infektion mit einem mikrobiellen Zielobjekt.

19. Verwendung eines αvβ6-Integrins zur Reinigung oder Konzentrierung eines mikrobiellen Zielobjekts aus einer Probe.

20. Verwendung nach Anspruch 17, 18 oder 19, wobei das mikrobielle Zielobjekt das Maul- und Klauenseuche-Virus (MKSV) ist.

21. Verwendung nach einem der Ansprüche 17 bis 20 in einem ELISA.

22. Verwendung nach einem der Ansprüche 12 bis 21, wobei das αvβ6-Integrin eine natürlich vorkommende αv-Untereinheit und/oder eine natürlich vorkommende β6-Untereinheit umfasst.

23. Verwendung nach Anspruch 17, wobei das αvβ6-Integrin immobilisiert ist.

24. Isoliertes Polypeptid, das die Aminosäuresequenz SEQ ID NO. 1 umfasst oder aus dieser besteht.

25. Isolierter Komplex, der ein Polypeptid nach Anspruch 24 und eine αv-Integrin-Unterbeinheit umfasst.

26. Komplex nach Anspruch 25, wobei der Komplex immobilisiert ist.

27. Isoliertes Polynucleotid mit Codierung für ein Polypeptid nach Anspruch 24.

28. Polynucleotid nach Anspruch 27, wobei das Polynucleotid die Nucleotidsequenz SEQ ID NO. 2 umfasst oder aus dieser besteht.

## Revendications

1. Procédé pour déterminer si un échantillon contient un agent microbien cible, consistant à :
(a) mettre en contact l'échantillon avec une intégrine αvβ6 dans des conditions qui permettent à l'agent microbien cible, s'il est présent dans l'échantillon, de se lier à l'intégrine; et
(b) déterminer si l'intégrine a un quelconque agent microbien cible lié à celle-ci.

2. Procédé selon la revendication 1, lequel procédé comprend une première étape consistant à mettre en contact l'échantillon avec un ligand de capture ayant de l'affinité pour l'agent microbien cible et une seconde étape subséquente consistant à déterminer si le ligand de capture a un quelconque agent microbien cible lié à celui-ci, en utilisant un ligand de détection ayant de l'affinité pour l'agent microbien cible, dans lequel soit le ligand de capture soit le ligand de détection est une intégrine αvβ6.

3. Procédé selon la revendication 2, dans lequel le ligand de capture ou le ligand de détection comprend un anticorps ayant de la spécificité pour l'agent microbien cible ou un fragment de liaison à un antigène de celui-ci.

4. Procédé pour déterminer si un échantillon contient des anticorps contre un agent microbien cible, consistant à :
(a) mettre en contact l'échantillon avec un spécimen de l'agent microbien cible dans des conditions qui permettent aux anticorps anti-agent microbien cible, s'ils sont présents dans l'échantillon, de se lier à l'agent microbien cible ;
(b) mettre en contact le spécimen d'agent microbien cible avec un ligand de capture immobilisé ayant de l'affinité pour l'agent microbien cible ;
(c) mettre en contact le spécimen d'agent microbien cible immobilisé avec un ligand compétiteur ayant de l'affinité pour l'agent microbien cible ; et
(d) déterminer si le spécimen d'agent microbien cible immobilisé a un quelconque ligand compétiteur lié à celui-ci dans lequel l'étape (b) peut être effectuée soit avant soit après l'étape (a)
dans lequel soit le ligand de capture soit le ligand compétiteur est une intégrine αvβ6
et dans lequel la quantité du ligand compétiteur lié au spécimen d'agent microbien cible procure une indication de la présence d'anticorps contre un agent microbien cible dans l'échantillon.

5. Procédé selon la revendication 4, dans lequel le ligand de capture ou le ligand compétiteur comprend un anticorps ou un fragment de liaison à un antigène de celui-ci.

6. Procédé selon l'une quelconque des revendications 1 à 5, dans lequel l'agent microbien cible est un virus de fièvre aphteuse (FMDV).

7. Procédé selon l'une quelconque des revendications précédentes, dans lequel l'intégrine αvβ6 comprend une sous-unité αv naturelle et/ou une sous-unité β6 naturelle.

8. Procédé selon l'une quelconque des revendications 1 à 7, dans lequel l'intégrine αvβ6 est immobilisée.

9. Procédé selon la revendication 1 ou 4, dans lequel l'étape (b) est effectuée par ELISA.

10. Procédé selon la revendication 1, comprenant en outre l'étape (c) consistant à caractériser la souche virale détectée dans l'étape (b).

11. Procédé selon la revendication 10, dans lequel l'étape (c) comprend la caractérisation par RT-PCR ou ELISA.

12. Kit d'éléments pour mettre en oeuvre un procédé selon l'une quelconque des revendications précédentes, comprenant une intégrine αvβ6 ou des cellules capables d'exprimer l'intégrine αvβ6 lorsqu'elles sont développées en culture ou des vecteurs comprenant une séquence nucléotidique qui code l'intégrine αvβ6, lequel kit comprend en outre un substrat sur lequel l'intégrine est ou peut être immobilisée.

13. kit selon la revendication 12, comprenant en outre un moyen pour détecter un agent microbien cible.

14. kit selon la revendication 13, dans lequel le moyen pour détecter l'agent microbien cible est un anticorps anti-agent microbien cible.

15. kit selon l'une quelconque des revendications 12 à 14, comprenant en outre un immunoconjugué comprenant le domaine de liaison à un antigène d'un anticorps ayant de la spécificité pour l'anticorps anti-agent microbien cible fusionné à un marqueur détectable directement ou indirectement.

16. Kit selon l'une quelconque des revendications 12 à 15, comprenant en outre un ou plusieurs réactifs pour effectuer une ELISA.

17. Utilisation d'une intégrine αvβ6 pour détecter un agent microbien cible dans un échantillon.

18. Utilisation d'une intégrine αvβ6 dans le diagnostic *in vitro* d'une infection par un agent microbien cible.

19. Utilisation d'une intégrine αvβ6 pour purifier ou concentrer un agent microbien cible à partir d'un échantillon.

20. Utilisation selon la revendication 17, 18 ou 19, dans laquelle l'agent microbien cible est le virus de la fièvre aphteuse (FMDV).

21. Utilisation selon l'une quelconque des revendications 17 à 20, dans une ELISA.

22. Utilisation selon l'une quelconque des revendications 12 à 21, dans laquelle l'intégrine αvβ6 comprend une sous-unité αv naturelle et/ou une sous-unité β6 naturelle.

23. Utilisation selon la revendication 17, dans laquelle l'intégrine αvβ6 est immobilisée.

24. Polypeptide isolé comprenant ou consistant en la séquence d'acides aminés de la SEQ ID NO: 1.

25. Complexe isolé comprenant un polypeptide selon la revendication 24 et une sous-unité d'intégrine αv.

26. Complexe selon la revendication 25, lequel complexe est immobilisé.

27. Polynucléotide isolé codant un polypeptide selon la revendication 24.

28. Polynucléotide selon la revendication 27, lequel polynucléotide comprend ou consiste en la séquence nucléotidique de la SEQ ID NO: 2.
